# FASCICULE DE BREVET EUROPEEN

(11) **EP 2 585 616 B1**
(45) Date de publication et mention de la délivrance du brevet: **30.07.2014**
(21) Numéro de dépôt: 11736433.1
(22) Date de dépôt: 24.06.2011
(51) Int. Cl.: C12Q 1/68

(54) **DISPOSITIF POUR DETERMINER OU ETUDIER L'ETAT DE STIMULATION DES DEFENSES NATURELLES DE PLANTES OU PARTIES DE PLANTES**
VORRICHTUNG ZUR BESTIMMUNG ODER UNTERSUCHUNG DES STIMULATIONSZUSTANDES NATÜRLICHER ABWEHRKRÄFTE VON PFLANZEN ODER PFLANZENTEILEN
DEVICE FOR DETERMINING OR STUDYING THE STATE OF STIMULATION OF THE NATURAL DEFENCES OF PLANTS OR PORTIONS OF PLANTS

(30) Priorité: 24.06.2010 FR 1055042
(43) Date de publication de la demande: 01.05.2013
(73) Titulaire: Institut National De La Recherche Agronomique (INRA), 75007 Paris (FR)
(72) Inventeur: BRISSET, Marie-Noëlle, F-49000 Angers (FR); DUGE de BERNONVILLE, Thomas, F-49400 Saumur (FR)
(74) Mandataire: Coralis Harle
(86) Numéro de dépôt international: PCT/FR2011/051470
(87) Numéro de publication internationale: WO 2011/161388

(56) Documents cités:
- EP-A1- 1 038 965
- WO-A1-2009/041805
- WO-A2-2007/062737
- DE VOS MARTIN ET AL: "Signal signature and transcriptome changes of Arabidopsis during pathogen and insect attack", MOLECULAR PLANT-MICROBE INTERACTIONS, vol. 18, no. 9, septembre 2005 (2005-09), pages 923-937, XP002612140, ISSN: 0894-0282
- PRIME-A-PLANT GROUP ET AL: "Priming: getting ready for battle", MOLECULAR PLANT-MICROBE INTERACTIONS : MPMI OCT 2006,, vol. 19, no. 10, 1 octobre 2006 (2006-10-01), pages 1062-1071, XP002487044,
- BONASERA JEAN M ET AL: "PR genes of apple: identification and expression in response to elicitors and inoculation with Erwinia amylovora", BMC PLANT BIOLOGY, BIOMED CENTRAL, LONDON, GB, vol. 6, no. 1, 9 octobre 2006 (2006-10-09) , page 23, XP021022649, ISSN: 1471-2229, DOI: DOI:10.1186/1471-2229-6-23

## Description

### Domaine de l'invention

La présente invention concerne le domaine des dispositifs pour déterminer et/ou étudier l'état de stimulation des défenses naturelles de plantes.

Plus précisément, cette invention concerne un dispositif comprenant des moyens pour déterminer le niveau ou profil d'expression d'une combinaison déterminée de gènes cibles endogènes dans des plantes ou des parties de plantes, de sorte à identifier la présence, le niveau et/ou l'intensité de la stimulation des défenses naturelles desdits plantes ou parties de plantes.

### Art antérieur

Les plantes sont exposées à de multiples agressions, lesdites agressions englobant les événements consistant en des stress dits « abiotiques » (sécheresse, température extrême, rayonnements ultraviolets, etc.) et des stress dits « biotiques » (virus, bactéries, champignons, ravageurs, etc.).

Il est connu que les plantes possèdent une pluralité de mécanismes endogènes qui sont capables d'offrir une défense efficace à l'encontre d'un panel large de tels stress.

Dans le cas de stress biotiques, trois niveaux de mécanismes de défense peuvent se développer de manière spatio-temporelle à partir du point d'infection ou d'infestation, et concourir à freiner la propagation de la maladie.

Sur le site de pénétration du pathogène, les cellules en contact avec l'agent pathogène peuvent s'autodétruire et retarder ainsi sa progression ; ce premier phénomène de défense est connu sous l'appellation de réaction d'hypersensibilité (HR).

A partir du site d'infection, des signaux d'alertes peuvent être transmis aux cellules avoisinantes, ce qui crée une zone locale de résistance acquise, où s'accumulent de nombreux composés de défense ; ce second niveau de défense assure une résistance acquise locale (LAR).

Des signaux peuvent également être transmis à la plante entière, ce qui induit un troisième niveau de défense : la résistance acquise systémique (SAR).

Des phénomènes ou voies physiologiques bien caractérisés des mécanismes de défense inductibles par les agents pathogènes, impliqués dans les niveaux de défense LAR et SAR, sont par exemple :
(i) la production de composés anti-microbiens, telles que les protéines relatives aux pathogènes (couramment dénommées « protéines PR » ou « PRP ») et les phytoalexines,
(ii) le renforcement des parois cellulaires (dépôt de callose, lignification, réticulation de protéines), et
(iii) la production de certaines hormones de plantes, notamment l'acide salicylique (SA), l'acide jasmonique (JA) et l'éthylène (ET), lesdites hormones jouant un rôle important dans la signalisation des mécanismes de défense induits par les stress.

En plus de ces réactions de défense induites par le stress, l'induction de la SAR confère, à la plante, la capacité de résister à des attaques ultérieures, y compris dans des localisations distantes par rapport au premier site d'infection ou d'infestation dans ladite plante.

Il peut arriver que les mécanismes de défenses ne soient pas activés de manière complète, mais que les tissus soient seulement « sensibilisés ». Une expression plus rapide et renforcée des mécanismes de défense n'est déclenchée qu'après exposition de la plante à un stress ultérieur. Ce phénomène est couramment dénommé « priming » ou « potentialisation ».

Cette connaissance approfondie des mécanismes de défense des plantes a permis le développement et l'utilisation de produits phytosanitaires, qui n'agissent plus directement sur la cause du stress, mais qui présentent la propriété d'agir indirectement par l'activation et la stimulation des mécanismes de défense naturelle.

De tels produits à effet stimulateur des défenses naturelles (encore dénommés « stimulateurs des défenses naturelles » ou sous l'acronyme « SDN ») peuvent être classés selon deux principales familles :
(i) les composés dits « stimulateurs directs », qui entraînent, une fois appliqués sur la plante, une activation complète des réactions de défense, qu'il y ait ou non présence de pathogènes, et
(ii) les composés dits « potentialisateurs », qui déclenchent, après application sur la plante, uniquement le phénomène de « potentialisation » précité (les réactions de défense ne s'activant qu'à la suite d'une attaque par un agent pathogène ou un stress).

La plupart de ces produits SDN sont encore connus sous l'appellation de « éliciteurs » (ou « éliciter ») ou encore de « inducteurs de résistance ».

De nombreuses études ont été menées sur ces mécanismes de défense, et les produits à effet stimulateur des défenses naturelles, à partir d'espèces modèles, en particulier à partir de *Arabidopsis thaliana.*

On précise toutefois qu'à l'heure actuelle, il n'existe pas de cadre règlementaire spécifique régissant les conditions de mise sur le marché et d'utilisation des produits SDN.

Ainsi, pour bénéficier d'une autorisation de mise sur le marché (AMM), de tels produits SDN peuvent entrer (i) tels quels, dans la catégorie des intrants phytopharmaceutiques, ou (ii) en mélange avec des molécules fertilisantes, dans la catégorie des intrants fertilisants.

Très peu d'AMM pour intrants phytopharmaceutiques ont été délivrées à ce jour pour des produits SDN.

Concernant plus spécifiquement la connaissance des effets de ces intrants sur les plantes, la stimulation des défenses naturelles est généralement étudiée seulement à une échelle cellulaire, par exemple sur suspensions cellulaires ou sur organes détachés.

A la connaissance de la demanderesse, un effet de stimulation des défenses naturelles au niveau moléculaire sur les plantes entières n'est généralement pas été démontré.

A l'inverse, on connaît une grande diversité de produits homologués comme intrants phytopharmaceutiques ou comme intrants fertilisants, qui seraient susceptibles d'exercer aussi une action de stimulation des défenses naturelles de plantes, bien qu'une telle activité additionnelle n'ait pas été démontrée ou identifiée.

Certains produits pourraient également avoir un effet inhibiteur des défenses naturelles de plantes.

La situation exposée ci-dessus pour les plantes en général, se retrouve notamment pour les plantes de la famille des Rosaceae qui inclut diverses espèces fruitières.

Il existe par conséquent un besoin pour la mise à disposition des professionnels d'un dispositif ou d'outil polyvalent qui permettrait d'identifier, de manière simple et rapide, l'état de stimulation des défenses naturelles de plantes ou de parties de plantes, notamment de la famille des Rosaceae.

Ce dispositif ou outil polyvalent aurait également avantageusement comme objectif de permettre le criblage des substances pour leurs propriétés de stimulation des défenses naturelles des plantes, y compris de plantes appartenant à la famille des *Rosaceae.*

Un tel dispositif ou outil devrait en particulier offrir la possibilité de réaliser une discrimination entre (i) les produits SDN qui activent effectivement les défenses des plantes, et (ii) les produits qui sont dépourvus d'un tel effet.

Ce dispositif ou outil permettrait ainsi le réaliser le criblage de nouveaux produits SDN et d'effectuer des études diverses sur les SDN, par exemple des études visant à déterminer le ou les mécanismes(s) d'action et/ou la ou les voies moléculaires impliqués dans l'effet de stimulation des défenses d'une plante par un produit SDN particulier, par une combinaison particulière de produits SDN, ou encore par une combinaison entre un ou plusieurs produits SDN avec un ou plusieurs autres intrants susceptibles de présenter une action antagoniste sur la stimulation des défenses.

WO 2009/041805 A1 (2 avril 2009) décrit l'identification de gènes de facteurs de transcription dont l'expression est induite ou réprimée dans des racines d'Arabidopsis lors de la stimulation des défenses naturelles (priming) en présence soit d'une bactérie WCS417r soit d'acide beta-aminobutyrique (BABA), ainsi que l'utilisation d'un jeu sélectionné de 37 facteurs de transcription comme marqueurs de la stimulation des défenses naturelles.

### Résumé de l'invention

La présente invention est relative à un dispositif pour déterminer ou étudier l'état de stimulation des défenses naturelles de plantes ou parties de plantes, lequel dispositif comprend des moyens de détermination du niveau d'expression en ARNm exprimé par une combinaison de gènes cibles dans un échantillon de plantes ou parties de plantes, lesdits moyens de détermination comprenant :
(a) un moyen de détermination du niveau d'expression en ARNm d'au moins un gène cible choisi parmi les gènes cibles suivants : PR-1, PR-2, PR-4, PR-5, PR-8, PR-14, PR-15 ;
(b) un moyen de détermination du niveau d'expression en ARNm d'au moins un gène cible choisi parmi les gènes cibles suivants : PAL, CHS, DFR, ANS, PPO ;
(c) un moyen de détermination du niveau d'expression en ARNm d'au moins un gène cible choisi parmi les gènes cibles suivants : HMGR, FPPS, Far ;
(d) un moyen de détermination du niveau d'expression en ARNm du gène cible CSL ;
(e) un moyen de détermination du niveau d'expression en ARNm d'au moins un gène cible choisi parmi les gènes suivants : APOX, GST, POX ;
(f) un moyen de détermination du niveau d'expression en ARNm d'au moins un gène cible choisi parmi les gènes cibles suivants : CalS, Pect, CAD ;
(g) un moyen de détermination du niveau d'expression en ARNm d'au moins un gène cible choisi parmi les gènes cibles suivants : EDS1, WRKY ;
(h) un moyen de détermination du niveau d'expression en ARNm d'au moins un gène cible choisi parmi les gènes cibles suivants : LOX2, JAR ;
(i) un moyen de détermination du niveau d'expression en ARNm d'au moins un gène cible choisi parmi les gènes cibles suivants : ACCO, EIN3.

Dans certains modes de réalisation, ledit dispositif comprend les moyens de détermination du niveau d'expression en ARNm de la combinaison des gènes cibles suivants : PR-1, PR-2, PR-4, PR-5, PR-8, PR-14, PR-15, PAL, CHS, DFR, ANS, PPO, HMGR, FPPS, Far, CSL, APOX, GST, POX, CalS, Pect, CAD, EDS1, WRKY, LOX2, JAR, ACCO, EIN3.

Dans certains modes de réalisation, lesdits moyens de détermination du niveau d'expression en ARNm d'un gène cible sont choisis parmi les fragments d'acide nucléique capables de s'hybrider de manière spécifique aux ARNm exprimés par ledit gène cible ou aux ADNc correspondants, ou à des fragments desdits ARNm ou desdits ADNc.

Dans certains modes de réalisation, les fragments d'acide nucléique capables de s'hybrider de manière spécifique aux ARNm exprimés par ledit gène cible ou aux ADNc correspondants consistent en des amorces, lesdites amorces étant préférentiellement choisies parmi les séquences suivantes SEQ ID n°32 à 87.

La présente invention est également relative à un procédé pour identifier un profil d'expression en ARNm d'une combinaison de gènes cibles permettant de déterminer, ou au moins d'évaluer, un état de stimulation des défenses naturelles de plantes ou de parties de plantes, lequel procédé comprend les étapes suivantes :
(i) déterminer le profil d'expression en ARNm d'une combinaison de gènes cibles au moyen du dispositif précité, sur un ensemble de plantes ou de parties de plantes, dont l'état de stimulation de leurs défenses naturelles est connu, puis
(ii) déterminer un profil d'expression en ARNm de ladite combinaison de gènes cibles correspondant à un état déterminé de stimulation des défenses naturelles desdites plantes ou parties de plantes, partant des données issues de l'étape (i).

L'invention concerne aussi un procédé pour déterminer ou évaluer l'état de stimulation des défenses naturelles d'une plante ou d'une partie de plante, comprenant les étapes suivantes :
(i) prélever un échantillon à partir de ladite plante ou de ladite partie de plante,
(ii) déterminer le profil d'expression en ARNm d'une combinaison de gènes cibles dans ledit échantillon prélevé à l'étape (i), au moyen du dispositif précité,
(iii) comparer le profil d'expression en ARNm obtenu à l'étape (ii) avec un profil d'expression de référence,
(iv) déterminer ou évaluer l'état de stimulation des défenses naturelles de ladite plante ou de ladite partie de plante, à partir dudit profil d'expression en ARNm obtenu lors de l'étape (ii).

La présente invention concerne encore un procédé pour sélectionner une substance ayant la propriété de moduler l'état de stimulation des défenses naturelles d'une plante ou d'une partie de plante, comprenant les étapes suivantes :
(i) mettre en contact ladite plante ou ladite partie de plante avec la substance à tester,
(ii) déterminer le profil d'expression en ARNm d'une combinaison de gènes cibles dans un échantillon prélevé à partir de ladite plante ou de ladite partie de plante suite à l'étape (i), au moyen du dispositif précité,
(iii) comparer le profil d'expression en ARNm obtenu à l'étape (ii) avec un profil d'expression en ARNm de référence, pour déterminer ou évaluer l'état de stimulation des défenses naturelles dans ledit échantillon,
(iv) sélectionner positivement ladite substance si la comparaison à l'étape (iii) montre que ladite substance testée à l'étape (i) module l'état de stimulation des défenses naturelles de ladite plante ou de ladite partie de plante.

La présente invention a aussi trait à un procédé pour sélectionner une plante présentant un état de stimulation des défenses naturelles susceptible de leur conférer une résistance améliorée à au moins un stress biotique et/ou abiotique d'intérêt, comprenant les étapes suivantes :
(i) appliquer ledit ou lesdits stress à une plante ou une partie de plante,
(ii) déterminer le profil d'expression en ARNm d'une combinaison de gènes cibles dans un échantillon prélevé à partir de la plante ou de ladite partie de plante, au moyen du dispositif précité,
(iii) comparer le profil d'expression en ARNm obtenu à l'étape (ii) avec un profil d'expression de référence, pour déterminer ou évaluer l'état de stimulation des défenses naturelles dans ledit échantillon,
(iv) sélectionner positivement ladite plante ou ladite partie de plante si la comparaison de l'étape (iii) montre que ladite plante ou ladite partie de plante possède un état de stimulation des défenses naturelles susceptible de leur conférer une résistance améliorée à au moins un stress biotique et/ou abiotique d'intérêt.

### Description détaillée de l'invention

Les inventeurs ont identifié un ensemble spécifique de gènes cibles, dont le niveau ou profil d'expression constitue un moyen simple et efficace pour déterminer et/ou étudier l'état de stimulation des défenses naturelles de plantes, avantageusement des plantes de la famille des *Rosaceae.*

Les inventeurs ont en effet mis en évidence que l'analyse de l'expression d'une combinaison spécifique de gènes cibles permet de déterminer l'état de stimulation des défenses naturelles de plantes qui sont (i) exposées à un produit SDN, (ii) exposées à une source ou un événement de stress biotique, (iii) exposées à une source ou un événement de stress abiotique, ou bien (iv) à une combinaison de deux ou des trois des expositions précitées.

Ces résultats ont permis aux inventeurs de mettre au point un dispositif, avantageusement un dispositif comprenant des moyens pour la détermination, avantageusement *in vitro,* du niveau ou profil d'expression d'une combinaison déterminée de gènes cibles (avantageusement par analyse transcriptomique ciblée ou par analyse protéomique ciblée), dans un échantillon provenant d'une plante, y compris un échantillon provenant d'une partie de plante, y compris d'une plante de la famille des *Rosaceae.*

Les inventeurs ont montré que la détermination *in vitro* du niveau ou profil d'expression de cette combinaison déterminée de gènes cibles permet l'étude et/ou la détermination de l'état de stimulation des défenses naturelles de la plante dont provient ledit échantillon.

La présente invention a ainsi pour objet un nouveau dispositif ou un nouvel outil de recherche, avantageusement un dispositif de biologie moléculaire, pour la caractérisation d'un échantillon biologique, par détermination du niveau d'expression d'une combinaison spécifique de gènes cibles.

Plus précisément, la présente invention concerne un dispositif pour déterminer et/ou étudier l'état de stimulation des défenses naturelles de plantes, lequel dispositif comprend des moyens pour déterminer le niveau ou profil d'expression d'une combinaison de gènes cibles dans un échantillon de plante ou de partie de plante, lesdits moyens de détermination comprenant :
(a) un moyen de détermination du niveau d'expression d'au moins un gène cible choisi parmi les gènes cibles suivants : PR-1, PR-2, PR-4, PR-5, PR-8, PR-14, PR-15 ;
(b) un moyen de détermination du niveau d'expression d'au moins un gène cible choisi parmi les gènes cibles suivants : PAL, CHS, DFR, ANS, PPO ;
(c) un moyen de détermination du niveau d'expression d'au moins un gène cible choisi parmi les gènes cibles suivants : HMGR, FPPS, Far ;
(d) un moyen de détermination du niveau d'expression du gène cible CSL ;
(e) un moyen de détermination du niveau d'expression d'au moins un gène cible choisi parmi les gènes suivants : APOX, GST, POX ;
(f) un moyen de détermination du niveau d'expression d'au moins un gène cible choisi parmi les gènes cibles suivants : CalS, Pect, CAD ;
(g) un moyen de détermination du niveau d'expression d'au moins un gène cible choisi parmi les gènes cibles suivants : EDS1, WRKY ;
(h) un moyen de détermination du niveau d'expression d'au moins un gène cible choisi parmi les gènes cibles suivants : LOX2, JAR ;
(i) un moyen de détermination du niveau d'expression d'au moins un gène cible choisi parmi les gènes cibles suivants : ACCO, EIN3.

Malgré le nombre limité et déterminé de gènes cibles analysés, les inventeurs ont montré que le dispositif selon l'invention constitue un outil particulièrement polyvalent et puissant, autorisant l'étude et/ou la détermination de l'état de stimulation des défenses naturelles des plantes (i) exposées à un ou plusieurs composés ou à une ou plusieurs compositions stimulateurs de défenses naturelles et/ou (ii) exposées à une grande variété de stress, ce qui englobe aussi bien des stress biotiques que des stress abiotiques, et/ou (iii) exposées à une combinaison des expositions précitées (i) et (ii).

Le dispositif selon l'invention présente l'intérêt d'apporter des résultats simples, rapides et efficacement interprétables, sur l'état de stimulation des défenses naturelles d'une plante.

Le dispositif selon l'invention permet notamment d'étudier et/ou d'identifier des composés candidats, susceptibles de consister en des stimulateurs des défenses naturelles des plantes et capable d'induire un mécanisme de réaction des plantes à l'encontre d'une agression par des organismes nuisibles et/ou un mécanisme de réaction de défense à l'encontre des stress abiotiques naturels.

Selon un mode de réalisation préféré, le dispositif selon l'invention comprend les moyens de détermination du niveau d'expression des gènes suivants :
(i) pour le groupe (a), un moyen de détermination du niveau d'expression de l'un au moins des gènes suivants: PR-1, PR-2, PR-4 ou PR-8, un moyen de détermination du niveau d'expression du gène PR-5, un moyen de détermination du niveau d'expression du gène PR-14 et un moyen de détermination du niveau d'expression du gène PR-15, et/ou
(ii) pour le groupe (b), un moyen de détermination du niveau d'expression du gène PAL, un moyen de détermination du niveau d'expression de l'un au moins des gènes suivants : CHS, DFR ou ANS, et un moyen de détermination du niveau d'expression du gène PPO, et/ou
(iii) pour le groupe (c), un moyen de détermination du niveau d'expression de l'un au moins des gènes suivants : HMGR et Far, et un moyen de détermination du niveau d'expression du gène FPPS, et/ou
(iv) pour le groupe (e), un moyen de détermination du niveau d'expression du gène APOX et un moyen de détermination du niveau d'expression de l'un au moins des gènes suivants : GST et POX.

Selon un mode de réalisation encore préféré, ce dispositif comprend les moyens de détermination du niveau d'expression de la combinaison des gènes cibles suivants : PR-1, PR-2, PR-4, PR-5, PR-8, PR-14, PR-15, PAL, CHS, DFR, ANS, PPO, HMGR, FPPS, Far, CSL, APOX, GST, POX, CalS, Pect, CAD, EDS1, WRKY, LOX2, JAR, ACCO, EIN3.

Les moyen de détermination du niveau d'expression d'un gène cible sont avantageusement choisis parmi les fragments d'acide nucléique capables de s'hybrider de manière spécifique aux ARNm exprimés par ledit gène cible ou aux ADNc correspondants, ou à des fragments desdits ARNm ou desdits ADNc.

Dans les modes de réalisation ci-dessus, les fragments d'acide nucléique précités consistent avantageusement en des amorces nucléotidiques s'hybridant spécifiquement avec les ARNm, les ADNc, ou des fragments de ceux-ci, dérivés de chacun des gènes cibles d'intérêt.

Dans certains modes de réalisation, les amorces nucléotidiques correspondantes sont avantageusement adaptées à la détermination du niveau d'expression des gènes cibles par une méthode de PCR quantitative.

Les amorces nucléotidiques sont avantageusement choisies parmi les séquences suivantes SEQ ID n°32 à 87. L'utilisation de ces amorces, dans le disposition selon l'invention, est illustrée dans les exemples.

Selon un mode particulier de réalisation, les fragments d'acide nucléique ou lesdits anticorps sont immobilisés sur un support.

Dans les modes de réalisation du dispositif dans lesquels les moyens de détermination du niveau d'expression d'un gène cible consistent en des acides nucléiques, ledit dispositif consiste ainsi avantageusement en une puce à ADN, pouvant être désignée encore sous la dénomination de « puce à faible densité quantitative » ou « qPFD » (du fait du nombre limité de gènes ciblés).

La présente invention concerne également sur des procédés mettant en oeuvre le dispositif selon l'invention et qui seront présentés en détails dans la description, à savoir :
- un procédé pour identifier un profil d'expression d'une combinaison de gènes cibles (encore désigné « signature » ou « signature d'expression ») permettant de déterminer, ou au moins d'évaluer, un état de stimulation des défenses naturelles de plantes ;
- un procédé pour déterminer ou évaluer l'état de stimulation des défenses naturelles de plantes ou d'une partie de telles plantes ;
- un procédé pour sélectionner une substance ayant la propriété de moduler l'état de stimulation des défenses naturelles d'un plant ou d'une partie de plant ;
- un procédé pour sélectionner une plante ou partie de plante, présentant un état de stimulation des défenses naturelles susceptible de leur conférer une résistance améliorée à au moins un stress biotique et/ou abiotique d'intérêt.

Chacun de ces dispositifs est tout particulièrement adapté pour une mise en oeuvre sur des plantes ou parties de plantes appartenant à la famille des *Rosaceae.*

Tel que divulgué ci-après, la présente invention fournit donc un nouveau dispositif ou outil pour déterminer et/ou étudier l'état de stimulation des défenses naturelles de plantes, qui utilise pour cela la détermination du profil d'expression et/ou la détection du profil d'expression et/ou la quantification du niveau d'expression d'une combinaison de gènes cibles dans un échantillon de plante ou de partie de plante.

Les inventeurs ont ainsi identifié un ensemble de gènes cibles contenus dans le génome de plante qui constituent des marqueurs biologiques aptes à servir d'indicateurs dans l'étude et/ou la détermination de la présence et/ou du niveau et/ou de l'intensité de l'état de stimulation des défenses naturelles d'une plante ou d'une partie de plante.

Les inventeurs ont montré que le dispositif selon l'invention permet, avec un nombre limité et déterminé de marqueurs, de déterminer et/ou d'étudier l'effet d'un composé ou d'une composition d'intérêt et/ou d'un stress biotique et/ou d'un stress abiotique, sur l'état de stimulation des défenses naturelles de plantes ou parties de plantes traitées.

Il s'avère que ce dispositif selon l'invention est en particulier intéressant pour l'étude des plantes ou des parties de plantes du genre des *Rosaceae* ou Rosacées, comme cela est illustré dans les exemples.

Après avoir défini certains termes, la présente divulgue les gènes cibles spécifiquement sélectionnés pour l'étude et/ou la détermination de l'état de stimulation des défenses naturelles des plantes ou parties de plantes, puis décrit le dispositif selon l'invention avec les moyens pour mesurer et/ou déterminer le niveau ou profil d'expression desdits gènes cibles respectifs.

Un tel dispositif présente de nombreuses applications qui seront également développées ci-après, notamment (i) la sélection et l'étude de composés ou de compositions qui sont aptes à modifier l'état de stimulation des défenses naturelles de plantes ou de parties de plantes et/ou (ii) la sélection de plantes ou de parties de plantes présentant ou étant aptes à présenter un état particulier de stimulation des défenses naturelles, par exemple suite à l'application d'un composé ou d'une composition d'intérêt et/ou d'un stress biotique et/ou d'un stress abiotique.

### Définition

Par « plante », on entend tout organisme pluricellulaire appartenant au sous-règne des *Tracheobionta,* comprenant les ptéridophytes et les spermaphytes. Par « spermaphytes », on entend un organisme appartenant aux gymnospermes ou de préférence encore aux angiospermes. Parmi les angiospermes, on entend les monocotylédones ou encore les dicotylédones, et de préférence encore les eudicotylédones. Par « dicotylédones », on entend les sous-classes suivantes : *Asteridae, Caryophyllidae, Dilleniidae, Hamamelidae, Hamamelididae, Magnoliidae* ou *Rosidae.* Par « *Rosidea* », on entend les ordres suivants : *Apiales, Celastrales, Cornales, Euphorbiales, Fabales, Geraniales, Haloragales, Linales, Myrtales, Podostemales, Po*/*ygalales, Proteales, Rafflesiales, Rhamnales, Rhizophorales, Rosales, Santatales, Sapindales.* Dans l'ordre des *Rosidea,* on choisit encore de préférence une plante de la famille des *Rosaceae,* y compris les pommiers.

Par « échantillon », on entend un échantillon biologique contenant le matériel biologique permettant de détecter l'expression de la combinaison de gènes cibles.

Le matériel biologique peut comprendre notamment des protéines et/ou des acides nucléiques tels que notamment les acides désoxyribonucléiques (ADN) ou les acides ribonucléiques (ARN). Ce matériel biologique comprend du matériel spécifique des gènes cibles, tel que notamment les ARNm transcrits par les gènes cibles ou les protéines issues de ces ARNm, mais peut comprendre également du matériel non spécifique des gènes cibles, tels que notamment les ARNm transcrits par les gènes autres que les gènes cibles ou les protéines issues de ces ARNm. Selon un mode de réalisation de l'invention, le matériel biologique comprend des ARN, et encore plus préférentiellement des ARN totaux ; les ARN totaux comprennent les ARN de transfert, les ARN messagers (ARNm), tels que les ARNm transcrits par les gènes cibles mais également transcrits par tout autre gène, et les ARN ribosomaux.

Les échantillons biologiques englobent des fragments de tissus prélevés sur la plante, ainsi que les produits résultant de l'extraction ou la purification des acides nucléiques (ADN, ARNm) ou des protéines contenus dans lesdits fragments de tissus, ainsi que certains produits de transformation des substances contenues dans lesdits fragments de tissu ou dans lesdits produits de transformation, par exemple les acides nucléiques du type ADNc obtenus par transcription inverse des acides nucléiques du type ARNm.

Par « partie de plante », on entend le fragment d'une plante contenant au moins une cellule de plante, par exemple un organe de plante (tel qu'une feuille, un bourgeon, une fleur, une racine, un fruit, une graine ou partie de ceux-ci) ou un tissu de plante (tel qu'un méristème).

Par « stress environnementaux », on désigne l'ensemble des facteurs extérieurs à une plante susceptible d'affecter le métabolisme normal de cette plante et d'induire chez elle une réaction d'adaptation et/ou de défense. Les stress environnementaux peuvent provenir d'être vivants (il s'agit de stress biotique), ou de facteurs autres (on parle alors de stress abiotique).

Les stress biotiques regroupent notamment l'ensemble des agents pathogènes microbiens, tels que les agents pathogènes fongiques, bactériens, viraux ou ravageurs, et les infections ou infestations dont ils sont responsables.

Les stress abiotiques regroupent l'ensemble des stress de nature physique ou chimique, et notamment les stress oxydatifs ou climatiques ; il s'agit notamment des stress hydriques, comme le manque d'eau, ou les stress thermiques comme le froid ou la chaleur. Les stress oxydatifs regroupent l'ensemble des stress aboutissant à l'augmentation de la concentration d'agents oxydants dans une plante ou une partie de plante.

Par « état de stimulation de défenses naturelles », on entend le développement d'un ensemble de modifications biologiques qui confèrent à cette plante (i) une résistance immédiate, notamment LAR ou SAR, et/ou (ii) une pré-sensibilisation du type potentialisation grâce à laquelle elle devient capable de réagir plus efficacement à un stress ultérieur, biotique ou abiotique.

L'« état de stimulation de défenses naturelles » se réfère également au statut, activé ou non-activé, des différentes voies moléculaires impliqués dans les mécanismes de défenses naturelles.

Par « état de stimulation de défenses naturelles », on entend encore la résistance au stress, c'est-à-dire différents niveaux de tolérance au stress, à savoir une faculté des plantes à faire face aux stress biotiques et/ou de stress abiotiques. La résistance au stress peut être classée selon différents niveaux : - sensibilité, - tolérance moyenne à un ou plusieurs agressions biotiques ou abiotiques, et - tolérance élevée ou résistance totale à une ou plusieurs agressions biotiques ou abiotiques.

Par « état de stimulation de défenses naturelles », on entend en particulier la capacité d'une plante à résister aux maladies, c'est-à-dire différents niveaux de résistance et/ou de tolérance d'une plante aux maladies, y compris la sensibilité, la résistance moyenne et la résistance élevée ou une résistance totale à un ou plusieurs agents pathogènes. Elle peut correspondre à une modification des symptômes induits par des agents pathogènes de la maladie (tels que la fréquence et/ou la taille des lésions, etc.), ainsi que l'étendue de la colonisation des tissus par l'agent pathogène ou le pourcentage d'infection, par rapport à ceux observés dans les plantes témoins sensibles et cultivées avec des maladies identiques. La résistance à la maladie peut également être illustrée par une croissance plus élevée et/ou un rendement des plantes résistantes en comparaison des plantes sensibles lorsqu'elles sont cultivées sous la pression de la maladie.

Les expressions « état de stimulation des défenses naturelles activé », « état de stimulation des défenses naturelles induit », « amélioration de la résistance au stress », « résistance accrue à la maladie » et « réaction de défense renforcée » se réfèrent en particulier à toute augmentation significative de la résistance au stress ou de la résistance aux maladies d'une plante ou de tissus végétaux, par rapport à un contrôle approprié tel qu'une plante non soumise à un produit SDN ou ce même stress ou cette même maladie.

Par « état de stimulation des défenses naturelles activé », « état de stimulation des défenses naturelles induit », on se réfère encore à toute activation/induction des voies moléculaires impliqués dans les mécanismes de défenses naturelles dans une plante ou partie de plante soumise à un produit SDN ou un stress, par rapport à un contrôle approprié tel qu'une plante non soumise à ce même produit SDN ou ce même stress.

Par exemple, par « état de stimulation de défenses naturelles », on entend notamment un état « résistant » ou « sensible » à *E. amylovora* responsable du feu bactérien

Par « détermination et/ou étude de l'état de stimulation des défenses naturelles », on entend aussi bien (i) la détermination et/ou l'étude de l'existence ou de l'absence d'une telle stimulation des défenses naturelles, que (ii) la détermination et/ou l'étude du niveau et/ou de l'intensité de cette stimulation des défenses naturelles.

Par « niveau d'expression d'une combinaison de gènes cibles » ou « profil d'expression d'une combinaison de gènes cibles », on entend tout paramètre ou marqueur biologique détectable, mesurable et/ou quantifiable, dans la plante ou partie de plante étudiée, qui correspond, directement ou indirectement, au niveau d'expression de chacun des gènes sélectionnés.

Les termes « niveau d'expression » incluent l'absence et/ou la présence et/ou une valeur représentative de la quantité d'ARN messagers (« ARNm » ou « mRNA ») transcrits à partir de l'ADN génomique correspondant aux gènes cibles sélectionnés.

Par « surexpression » ou « activation » d'un gène, on entend un niveau quantitatif d'expression du marqueur dudit gène qui est multiplié au moins par 3 par rapport à un niveau quantitatif de la référence, de préférence encore au moins 4x, 5x, 10x, 20x, 30x ou plus.

Par « sous-expression » ou « inactivation » ou « répression » d'un gène, on entend un niveau quantitatif d'expression du marqueur dudit gène qui est divisé au moins par 3 par rapport à un niveau quantitatif de la référence, de préférence au moins 4x, 5x, 10x, 20x, 30x ou plus, voire non détectable.

Un niveau d'expression « constant » d'un gène correspond à un niveau quantitatif d'expression du marqueur dudit gène qui est compris dans un domaine délimité par une division par 3 et une multiplication par 3 dudit niveau quantitatif, par rapport au niveau quantitatif de la référence.

Le niveau d'expression des marqueurs peut être « relatif », c'est-à-dire que la variation de niveau d'expression d'un ou de plusieurs gènes est comparée par rapport au niveau d'expression d'autres échantillons, après « normalisation » des niveaux d'expression en utilisant par exemple des gènes contrôle.

Le multiple de modulation d'expression de chacun des gènes cibles, en surexpression ou en sous expression, peut être mesurée en utilisant par exemple des moyens de détermination du type PCR quantitative, avantageusement en temps réel, comme illustré par les exemples.

Les résultats peuvent être obtenus selon la méthode du ΔΔCt (Delta Delta CT) qui fournit les expressions relatives des gènes de défense dans un échantillon donné par rapport à l'échantillon appelé « calibrateur » (par exemple un échantillon d'une plante ou partie de plante non traitée, ou encore par exemple un échantillon d'une plante ou partie de plante traitée), expressions normalisées par la moyenne géométrique des gènes de référence de ces échantillons (Vandesompele et al., Genome Biol. 3(7) : research0034.1-0034.11, 2002 ; Livak et Schmittgen, Methods 25:402-408, 2001).

Le niveau d'expression des marqueurs peut également être « absolu », c'est-à-dire que les niveaux d'expression des marqueurs se référent à la quantité absolue desdits marqueurs (ARNm) dans un échantillon.

Selon l'invention, un « profil d'expression » ou une « signature d'expression » consiste en une représentation de l'ensemble des valeurs de niveaux d'expression de chacun des gènes cibles testés, pour la combinaison de gènes cibles testée.

### Combinaison de gènes cibles utilisés dans le dispositif selon l'invention

Le dispositif selon l'invention comprend des moyens pour déterminer et/ou étudier le niveau d'expression d'une combinaison établie de gènes cibles, ou autrement dit d' « un ensemble de gènes cibles ».

Cette combinaison selon l'invention se compose des neuf groupes de gènes cibles (a) à (i) suivants :
(a) au moins un gène cible choisi parmi les gènes codant des PR-protéines : PR-1, PR-2, PR-4, PR-5, PR-8, PR-14, PR-15 ;
(b) au moins un gène cible choisi parmi les gènes codant des enzymes de la voie des phénylpropanoïdes : PAL, CHS, DFR, ANS, PPO ;
(c) au moins un gène cible choisi parmi les gènes codant des enzymes de la voie des isoprénoides : HMGR, FPPS, Far ;
(d) un gène cible CSL, codant pour une enzyme du catabolisme de la cystéine ;
(e) au moins un gène cible choisi parmi les gènes codant pour des enzymes antioxydantes : APOX, GST, POX ;
(f) au moins un gène cible choisi parmi les gènes codant des enzymes impliquées dans les modifications de paroi : CalS, Pect, CAD ;
(g) au moins un gène cible choisi parmi les gènes impliqués dans la voie de signalisation de l'acide salicylique : EDS1, WRKY ;
(h) au moins un gène cible choisi parmi les gènes impliqués dans la voie de signalisation de l'acide jasmonique : LOX2, JAR ,
(i) au moins un gène cible choisi parmi les gènes cibles impliqués dans la voie de signalisation de l'éthylène: ACCO, EIN3.

En pratique, la surexpression dans un échantillon testé d'au moins l'un des gènes cibles, et de préférence encore d'une combinaison desdits gènes cibles dans au moins deux desdits groupes (a) à (i), par rapport à un échantillon non traité, permet d'identifier les plantes ou parties de plantes présentant un état de stimulation des défenses naturelles activé ou induit.

Ces gènes cibles sélectionnés sont présentés plus en détails dans le tableau 1 ci-après.

**Tableau 1 : liste des génes cibles selon l'invention**

| **SEQ ID N°** | **Nom du gène** | **Unigène Pommier (NCBI)** | **Fonction du gène** | **N°Accession Pommier ADNc (NCBI)** |
|---|---|---|---|---|
| 1 | **PR-1** | Mdo.3966 | Pathogenesis-related protein 1 | AF507974 |
| | | | PR-protéine | |
| 2 | **PR-2** | Mdo 2984 | Pathogenesis-related protein 2 (glucanases) | AF494404 |
| | | | PR-protéine | |
| 3 | **PR-4** | Mdo.2382 | Pathogenesis-related protein 4 (hevein-like) | CN877594 |
| | | | PR-protéine | |
| 4 | **PR-5** | Mdo.999 | Pathogenesis-related protein 5 (thaumatin-like, osmotin) | DR998561 |
| | | | PR-protéine | |
| 5 | **PR-8** | Mdo.3935 | Pathogenesis-related protein 8 (class III chitinase) | DQ318214 |
| | | | PR-protéine | |
| 6 | **PR-14** | Mdo.12217 | Pathogenesis-related protein 14 (lipid transfer protein) | CV656658 |
| | | | PR-protéine | |
| 7 | **PR-15** | - | Pathogenesis-related protein 15 (oxalate oxidase) | GO500607 |
| | | | PR-protéine | |
| 8 | **PAL** | Mdo.2983 | Phenylalanine ammonia-lyase | AF494403 |
| | | | Voie des phénylpropanoïdes | |
| 9 | **CHS** | Mdo.6113 | Chalcone synthase | AF494401 |
| | | | Voie des phénylpropanoïdes | |
| 10 | **DFR** | Mdo.13736 | Dihydroflavonol reductase | AF494390 |
| | | | Voie des phénylpropanoïdes | |
| 11 | **ANS** | Mdo.2932 | Anthocyanidin synthase | DQ156905 |
| | | | Voie des phénylpropanoïdes | |
| 12 | **PPO** | Mdo.2905 | Polyphenol oxidase | L29450 |
| | | | Voie des Phénylpropanoïdes | |
| 13 | **HMGR** | Mdo.2960 | Hydroxymethyl glutarate-CoA reductase | AY043490 |
| | | | Voie des isoprénoïdes | |
| 14 | **FPPS** | Mdo.2964 | Farnesyl pyrophosphate synthase | AY083165 |
| | | | Voie des Isoprénoïdes | |
| 15 | **Far** | Mdo.3011 | (E,E)-alpha-farnesene synthase | EB111255 |
| | | | Voie des isoprénoïdes | |
| 16 | **CSL** | Mdo.12560 | C-S-lyase | AY347795 |
| | | | Catabolisme de la cystéine | |
| 17 | **APOX** | Mdo.1891 | Ascorbate peroxidase | CN928974 |
| | | | Système antioxydant | |
| 18 | **GST** | Mdo.12372 | Glutathion S-transférase | FE969955 |
| | | | Système antioxydant | |
| 19 | **POX** | Mdo.11566 | Peroxidase | CN913385 |
| | | | Système antioxydant | |
| 20 | **CalS** | Mdo.12945 | Callose synthase | CN496203 |
| | | | Modification pariétale | |
| 21 | **Pect** | Mdo.15511 | Pectin methyl esterase | CV628630 |
| | | | Modification pariétale | |
| 22 | **CAD** | Mdo.2625 | Cinnamyl alcool dehydrogenase | AF053084 |
| | | | Modification pariétale | |
| 23 | **EDS1** | Mdo.1759 | Disease résistance protein EDS1 | CN949066 |
| | | | Signalisation acide salicylique | |
| 24 | **WRKY** | - | WRKY transcription factor 30 | AY347836 |
| | | | Signalisation acide salicylique | |
| 25 | **LOX2** | Mdo.10456 | Lipoxygenase AtLOX2 | CN941066 |
| | | | Signalisation acide jasmonique | |
| 26 | **JAR** | Mdo.2326 | Jasmonate resistant 1 | CN879199 |
| | | | Signalisation acide jasmonique | |
| 27 | **ACCO** | Mdo.3241 | 1-aminocyclopropene-1-carboxylate oxidase | AB086888 |
| | | | Signalisation éthylène | |
| 28 | **EIN3** | Mdo.12601 | EIN3-BINDING F BOX PROTEIN 1 | CV082047 |
| | | | Signalisation éthylène | |
| 29 | **TuA** | Mdo.3499 | Tubulin alpha-1 chain | CO065788 |
| | | | Gène de référence | |
| 30 | **Actin** | Mdo.701 | Actin 7 | CV151413 |
| | | | Gène de référence | |
| 31 | **GAPDH** | Mdo.1683 | Glyceraldehyde-3-phosphate dehydrogenase | CN494000 |
| | | | Gène de référence | |

Parmi ces gènes, on peut distinguer des gènes dont la fonction est connue mais qui n'ont jamais été mis en relation avec les mécanismes de défenses naturelles chez les Rosacées, par exemple le gène CSL.

Les gènes TuA, Actin et GAPDH constituent des gènes marqueurs dont le niveau d'expression est indépendant de l'état de stimulation des défenses naturelles. Ces gènes sont ici cités uniquement à titre d'exemple. De tels gènes « rapporteurs » permettent de corriger le niveau d'expression déterminé pour chacun des gènes cibles.

Dans la présente description, les appellations employées pour désigner chacun des gènes cibles correspondent à une appellation internationale reconnue, qui se retrouvent notamment dans les bases de données de séquences de gènes et de séquences de protéines, par exemple les bases UniGene proposées par le National Center for Biotechnology Information (NCBI, Bethesda, MD, USA) Pour cela, on se réfère de préférence à la base de données UniGene *Malus domestica*

Les gènes cibles en question sont encore définis respectivement par les séquences éditables à partir des numéros d'accession Unigene spécifiés dans le tableau 1.

Les gènes cibles selon l'invention désignent également les « variants » ou « unigènes » ou allèles qui correspondent à ces séquences.

Les variants incluent les séquences, ou gènes, homologues ou orthologues rencontrés de préférence dans les genres ou les variétés de Rosacées autres que *Malus domestica.* Plus généralement, les variants incluent avantageusement également les séquences, ou gènes, homologues ou orthologues qui se rencontrent dans toute autre plante, de préférence dans tout organisme appartenant au sous-règne des *Tracheobionta,* de préférence encore appartenant aux spermaphytes, de préférence encore aux angiospermes, de préférence encore aux dicotylédones, et de préférence encore au *Rosidea.*

Le terme « orthologue » ou « homologue » pour une séquence, un gène ou une protéine, se réfère ici à la séquence, au gène ou à la protéine, homologue qui se trouve dans une autre espèce, présentant la même fonction que la séquence, le gène ou la protéine d'intérêt, mais (généralement) qui a divergé(e) en séquence à partir du moment où les espèces comportant lesdits gènes ont divergées (les gènes ont évolué à partir d'un ancêtre commun par spéciation). De tels séquences ou gènes orthologues peuvent ainsi être identifiés dans d'autres espèces végétales par technique de comparaison de séquences (par exemple basés sur des pourcentages d'identité de séquences sur la séquence entière ou sur des domaines spécifiques) et / ou d'analyse fonctionnelle.

De telles séquences orthologues ont par exemple été identifiées chez d'autres genres de la famille des Rosacées.

Le tableau 2A ci-après précise le numéro d'accession dans la base de données GenBank proposée par le National Center for Biotechnology Information (NCBI, Bethesda, MD, USA), de chacune des séquences orthologues identifiées.

**Tableau 2A : Séquences orthologues pour les gènes cibles selon l'invention**

| **N°** | **Nom du gène** | **Orthologue chez les Fragaria** | **Orthologue chez les Prunus** | **Orthologue chez les Pyrus** | **Orthologue chez les Rosa** |
|---|---|---|---|---|---|
| 1 | **PR-1** | | DN556381 | | |
| | | DV440399 | GE653251 | AF195235 | EC586767 |
| 2 | **PR-2** | EX682264 | DN554095 | AJ504892 | EC587362 |
| 3 | **PR-4** | EX657003 | CB823353 | - | - |
| 4 | **PR-5** | DY668082 | | | |
| | | EX674853 | GE653177 | FK939207 | BI977710 |
| 5 | **PR-8** | | CV051978 | | |
| | | DY672350 | CV052812 | AJ504863 | BI977412 |
| 6 | **PR-14** | DY673738 | AM290861 | AF195216 | EC589716 |
| 7 | **PR-15** | DY673400 | GR410635 | - | EC586210 |
| 8 | **PAL** | CO817421 | | | |
| | | EX684393 | CV046544 | - | - |
| 9 | **CHS** | AI795154 | CB820023 | | |
| | | DY667960 | FE969272 | FK939234 | CF349759 |
| 10 | **DFR** | DY672493 | CV046853 | | |
| | | EX661062 | CB819555 | DB999982 | - |
| 11 | **ANS** | CX661854 | BU039495 | FK939239 | BI977949 |
| 12 | **PPO** | DY667415 | | | |
| | | EX674603 | CV045870 | AJ504916 | - |
| 13 | **HMGR** | DY669718 | AM290185 | | |
| | | EX659154 | FC861452 | - | BQ106200 |
| 14 | **FPPS** | DY669331 | | | |
| | | EX660570 | DY644308 | - | BQ104581 |
| 15 | **Far** | DY675841 | DY646265 | - | CF349900 |
| 16 | **CSL** | | AJ533335 | | |
| | | CO817796 | BU047350 | - | - |
| 17 | **APOX** | CX661243 | CV045878 | | EC586214 |
| | | CX661894 | GE653243 | GR957944 | BI978785 |
| 18 | **GST** | DV438230 | | | |
| | | DV438954 | CB820780 | DB999954 | EC587514 |
| 19 | **POX** | DY676157 | DW341091 | | |
| | | DY670759 | GR410510 | DC993457 | EC586635 |
| 20 | **CalS** | EX687641 | DY633833 | DC993380 | BQ104579 |
| 21 | **Pect** | DY666714 | BU044880 | - | BQ104257 |
| 22 | **CAD** | CX662236 | EE488909 | DV440820 | CF349542 |
| 23 | **EDS1** | | DY652698 | | |
| | | DY673255 | FC866180 | - | CF349463 |
| 24 | **WRKY** | EX687984 | AJ873733 | - | - |
| 25 | **LOX2** | DY674691 | BU046906 | - | CF349741 |
| 26 | **JAR** | DY667416 | FC864077 | - | - |
| 27 | **ACCO** | CX662198 | | | |
| | | EX670124 | AJ833064 | DB999960 | EC588379 |
| 28 | **EIN3** | DY666956 | EE488205 | - | - |
| 29 | **TuA** | DY671340 | | | BQ106089 |
| | | DY674254 | BU042671 | - | BQ105408 |
| 30 | **Actin** | DY668010 | DY650839 | | BQ104395 |
| | | EX688327 | CV044868 | - | BI977396 |
| 31 | **GAPDH** | DY667270 | DW357797 | | BI978153 |
| | | DY667095 | DW350728 | - | BQ104075 |

De la même manière, le dispositif selon l'invention pourrait être utilisé pour l'étude de plantes de la famille des *Vitaceae* (vigne), notamment des espèces du genre *Vitis,* et en particulier *Vitis vinifera.*

A titre indicatif, des séquences orthologues, correspondant aux gènes cibles, ont par exemple été identifiées chez *Vitis vinifera.*

Le tableau 2B ci-après précise le numéro d'accession dans la base de données GenBank proposée par le National Center for Biotechnology Information (NCBI, Bethesda, MD, USA) ou dans la base de données proposée par le Génoscope (Evry, France), de chacune des séquences orthologues identifiées.

**Tableau 2B**

| **Nom du gène** | **Orthologue chez Vitis vinifera (Vigne)** | |
|---|---|---|
| | **n°accession EST (NCBI)** | **n°accession gène (genoscope)** |
| **PR-1** | EE253686.1 | GSVIVT01037005001 |
| **PR-2** | GO652966.1 | GSVIVT01033538001 |
| **PR-4** | FG984977.1 | GSVIVT01036279001 |
| **PR-5** | EC925003.1 | GSVIVT01019840001 |
| **PR-8** | EC965083.1 | GIDVvT00013094001 |
| **PR-14** | DT021081.1 | GIDVvT00010281001 |
| **PR-15** | EC968706.1 | GSVIVT01031082001 |
| **PAL** | CN006882.1 | GSVIVT01025703001 |
| **ANS** | CF209704.1 | GSVIVT01019892001 |
| **CHS** | EV241635.1 | GSVIVT01032968001 |
| **DFR** | EE082741.1 | GSVIVT01009743001 |
| **PPO** | CF215866.1 | GIDVVT00029382001 |
| **HMGR** | EC937417.1 | GSVIVT01013435001 |
| **FPPS** | EC962139.1 | GSVIVT01014738001 |
| **Far** | DT009302.1 | GSVIVT01000402001 |
| **Alli** | FC061753.1 | GSVIVT01001413001 |
| **APOX** | CF211522.1 | GSVIVT01015626001 |
| **GST** | EE099033.1 | GSVIVT01027961001 |
| **POX** | DT007525.1 | GSVIVT01009107001 |
| **CAD** | CF511159.1 | GSVIVT01025239001 |
| **CalS** | EE095949.1 | GSVIVT01025362001 |
| **Pect** | EE076956.1 | GSVIVT01011699001 |
| **EDS1** | | GSVIVT01007860001 |
| **WRKY** | | GSVIVT01028718001 |
| **LOX2** | EE107237.1 | GSVIVT01025339001 |
| **JAR** | EC983478.1 | GSVIVT01027057001 |
| **ACCO** | CF511696.1 | GSVIVT01006065001 |
| **EIN3** | CF214803.1 | GSVIVT01015548001 |

Les gènes cibles selon l'invention sont également définis par les séquences SEQ ID n°1 à 28 qui correspondent à leurs ADNc respectifs, ou par les variants desdits séquences selon la définition ci-dessus.

Les séquences correspondantes sont également consultables sur la base de données GenBank (NCBI), au moyen des numéros d'accession précisés dans le tableau 1.

Pour la notion de « variant », on peut se référer aux définitions développées ci-dessus.

Les gènes cibles selon l'invention sont encore désignés par les protéines qu'ils codent respectivement.

Les séquences peptidiques correspondants aux gènes cibles sont précisées par exemple sur la base de données UniGene *Malus domestica,* et peuvent être obtenues au moyen des numéros d'accession précisés dans le tableau 1

Selon un mode de réalisation préféré, le dispositif selon l'invention comporte des moyens pour déterminer et/ou étudier le niveau d'expression d'au moins deux gènes dans chacun des groupes (a), (b), (c), (e), (f), (g), (h) et (i) précités.

Ce mode de réalisation préféré offre notamment les avantages suivants :
- si les gènes cibles d'un même groupe sont co-régulés (c'est surtout le cas des gènes des voies de signalisation qui sont souvent activés de manière transitoire), ils peuvent avoir une expression séquentielle, et le fait d'en choisir un seul augmente le risque de conclure à une absence de modulation de cette voie (si les prélèvements ne sont pas effectués au bon moment pour un gène donné) ;
- si les gènes cibles d'un même groupe sont non co-régulés (c'est particulièrement le cas des protéines PR, ainsi que des enzymes de la voie des phénylpropanoïdes, pour le gène PPO d'une part, PAL d'autre part, et enfin le groupe CHS, DFR et ANS), il devient alors arbitraire de n'en choisir qu'un ;
- il permet de suivre les expressions de plusieurs gènes cibles dans chaque groupe, la redondance d'information sur le niveau d'expression de plus d'un gène cible appartenant à un groupe donné accroissant encore la fiabilité des résultats.

Plus précisément, parmi les gènes cibles ci-dessus, le dispositif selon l'invention permet avantageusement l'étude et/ou l'analyse de l'expression de l'une au moins des combinaisons suivantes de gènes :
- pour le groupe (a), l'un au moins des gènes suivants : PR-1, PR-2, PR-4 ou PR-8, combiné avec l'un au moins des gènes suivants PR-5, PR-14 ou PR-15 ;
- pour le groupe (b), le gène PAL, et l'un au moins des gènes suivants : CHS, DFR ou ANS, et le gène PPO,
- pour le groupe (c), l'un au moins des gènes suivants : HMGR et/ou Far, et le gène FPPS,
- pour le groupe (e), le gène APOX et l'un au moins des gènes suivants : GST et/ou POX.

Selon un mode de réalisation préféré, la combinaison de gènes cibles étudiée dans le dispositif selon l'invention est la suivante : PR-1, PR-2, PR-4, PR-5, PR-8, PR-14, PR-15, PAL, CHS, DFR, ANS, PPO, HMGR, FPPS, Far, CSL, APOX, GST, POX, CalS, Pect, CAD, EDS1, WRKY, LOX2, JAR, ACCO et EIN3.

### Dispositif selon l'invention, et moyens pour déterminer et/ou étudier le niveau d'expression d'une combinaison de gènes cibles selon l'invention

### Caractéristiques générales du dispositif selon l'invention

La présente invention englobe le dispositif ou l'outil, avantageusement sous la forme d'une trousse ou d'un « kit », pour déterminer et/ou étudier l'état de stimulation des défenses naturelles dans un échantillon de plantes ou de parties de plantes, en particulier de plants de *Rosaceae* et en particulier encore de plants de pommier.

De tels dispositifs de détection et de quantification s'appuient sur la préparation d'un mélange réactionnel destiné à contenir les marqueurs biologiques d'intérêt et les réactifs spécifiques appropriées, cela dans des conditions adaptées et pendant un temps suffisant pour permettre au marqueur et son réactif spécifique d'interagir et de se lier, et ainsi former un complexe qui peut être retiré et/ou détecté dans ledit mélange réactionnel.

Le dispositif selon l'invention contient pour cela des moyens adaptés pour détecter tout marqueur biologique détectable, mesurable et/ou quantifiable, dans la plante ou partie de plante étudiée, qui correspond, directement ou indirectement, au niveau d'expression de chacun des gènes sélectionnés.

Selon un mode de réalisation préféré, les acides nucléiques (par exemple ARNm ou ADNc) constituent les marqueurs d'expression de chacun des gènes cibles d'intérêt.

La méthode de détection du dispositif conforme à l'invention est alors basée avantageusement sur la détection d'ARNm, d'ADNc, dans l'échantillon.

Les moyens de détection comprennent ainsi avantageusement des réactifs ou ligands ou agents ou composés ou compositions comprenant, ou consistant en, un ensemble de réactifs spécifiques, chacun d'eux étant capable de se lier spécifiquement avec un marqueur biologique d'intérêt, avantageusement du type acide nucléique, qui est représentatif du niveau d'expression de l'un des gènes cibles.

Les réactifs adaptés pour se lier avec des marqueurs du type acide nucléique, en particulier un ARNm ou un ADNc, incluent les acides nucléiques de séquences complémentaires.

Par exemple, des réactifs adaptés pour détecter/quantifier des acides nucléiques marqueurs peuvent inclure (i) des oligonucléotides (marqués ou non marqués) fixés à un support, (ii) des oligonucléotides marqués non fixés avec un support, (iii) une paire d'amorces pour une technique PCR ou similaire.

La trousse ou le kit selon l'invention contient encore tout composé additionnel approprié, utile pour la mise en oeuvre de l'invention.

Par exemple, le dispositif peut contenir des fluides ou milieux pour l'hybridation d'acides nucléiques complémentaires.

De manière générale, les dispositifs en question comprennent par exemple plusieurs réactifs spécifiques qui sont capables de détecter l'expression des gènes cibles selon l'invention, qui peuvent être associés avec des échantillons contrôle, des plaques de microtitration, des tubes Eppendorf, un mode d'emploi, etc.

Les éléments constitutifs du dispositif selon l'invention sont présentés plus en détails ci-après.

### Moyens pour déterminer le niveau d'expression des génes cibles dans le dispositif selon l'invention

Le dispositif selon l'invention comporte donc des moyens pour quantifier et/ou déterminer l'expression des gènes cibles précités.

Ainsi, tout moyen pour détecter et/ou quantifier un acide nucléique dans un échantillon biologique peut être ici employé.

En l'occurrence, les moyen de détermination du niveau ou du profil d'expression de chaque gène cible sont choisis avantageusement parmi les fragments d'acide nucléique, sélectionnés parmi ceux capables de s'hybrider de manière spécifique aux ARNm exprimés par chacun des gènes cibles ou aux ADNc correspondants, ou à des fragments desdits ARNm ou desdits ADNc.

En pratique, la quantification du niveau d'expression de chaque gène cible comprend les étapes suivantes :
(a) la préparation d'un échantillon d'acides nucléiques (ARNm et/ou ADNc), à partir d'une plante ou d'une partie de plante, et
(b) l'hybridation des ARNm/ADNc de l'échantillon préparé, avec des moyens de détermination comprenant un ou plusieurs réactifs de référence (polynucléotides du type sonde(s) ou amorce(s) notamment) contenus dans le dispositif selon l'invention.

L'échantillon d'acides nucléiques peut être obtenu à partir d'une partie de la plante (par exemple une feuille, racines, etc.), ou de la plante entière (par exemple semences ou jeunes plants), ou d'une pluralité de plantes ou de parties de plantes, tel qu'un lot de plantes ou de feuilles.

Ainsi, dans un premier temps, des parties de plantes sont prélevées, et éventuellement mises en commun, avant les étapes d'isolation de l'échantillon d'acides nucléiques, ou les étapes de détection.

L'échantillon d'acides nucléiques est de préférence extrait des cellules, par exemple en utilisant des méthodes standard d'extraction des acides nucléiques.

Des extraits bruts ou des échantillons de tissus bruts, comme par exemple les tissus végétaux homogénéisés, peuvent aussi être utilisés comme échantillon d'acides nucléiques, dans lequel les marqueurs transcrits sont détectés et/ou quantifiés.

A partir de l'échantillon préparé, le dispositif selon l'invention permet de quantifier le niveau d'expression en ARNm (ou des ADNc correspondants), correspondant aux gènes cibles précités.

Selon un mode de réalisation préféré, le dispositif selon l'invention est adapté pour étudier l'expression de gènes cibles sur la base de marqueurs du type nucléiques.

Dans ce cas, le dispositif peut être réalisé sous la forme d'une puce à ADN, désignée encore par les appellations de puce à gènes ou biopuce, ou par les termes « DNA chip », « DNA-microarray » ou « biochip ».

L'échantillon d'acides nucléiques est de préférence un échantillon d'ARNm ou d'ARN total ou d'ADNc.

Les ADNc peuvent être, de manière optionnelle, amplifiés par l'utilisation de toute méthode basée sur les réactions de polymérisation en chaîne, avant hybridation avec le ou les polynucléotides de référence ; de manière alternative, ces ADNc ne sont pas amplifiés.

Pour l'évaluation du niveau de transcription des gènes cibles en question, les moyens de détermination peuvent par exemple être basés sur (i) les méthodes d'amplification du type PCR quantitative, de préférence la méthode de RT-PCR quantitative, ou (ii) les méthodes d'hybridation d'acides nucléiques, par exemple sous forme de puces à ADN ou « hybridation microarray ».

La PCR quantitative (qPCR ou QPCR) peut être réalisées par des techniques et équipements conventionnels, bien connu de l'homme du métier, décrit par exemple dans S.A. Bustin (Ed.), *et al*., A-Z of Quantitative PCR, IUL Biotechnology series, n°5, 2005.

Une méthode possible est la PCR quantitative avec transcription inverse ou RT-qPCR (voir Czechowski et al., 2004, Plant J. 38, 366-379 ; Czechowski et al. 2005, Plant Physiol. 139, 5-17 ; Vandesompeleet al., 2002, Genome Biol. 3(7):research0034.1-0034.11).

Cette technique de mesure permet la détection d'un niveau relatif ou absolu d'expression de l'ARNm des gènes cibles dans l'échantillon.

Un tel dispositif employant une technique de qPCR est décrit plus en détail par la suite, et une application pratique est présentée dans les exemples.

De manière alternative, la détermination est classiquement obtenue par la mise en contact de l'échantillon d'acides nucléiques avec un support sur lequel sont fixés une pluralité de polynucléotides comportant des séquences complémentaires (par exemple au moins 7, 10, 15, 20, 25, 30, 40, 50, 100, 500 ou plus résidus ou bases nucléotidiques) à la séquence de chacun des acides nucléiques ou polynucléotides marqueurs.

Ces polynucléotides fixés comportent une séquence possédant une spécificité d'hybridation dans des conditions déterminées pour former un complexe d'hybridation avec le marqueur nucléique d'un gène cible.

Selon la présente invention, le marqueur spécifique du gène cible peut être (i) une séquence nucléotidique comprise dans un ARN messager issu du gène cible (on parle alors d'ARNm spécifique du gène cible), (ii) une séquence nucléotidique comprise dans un ADN complémentaire obtenu par transcription inverse dudit ARN messager (on parle alors d'ADNc spécifique du gène cible), ou encore (iii) une séquence nucléotidique comprise dans un ARN complémentaire obtenu par transcription dudit ADNc tel que décrit précédemment (on parlera alors d'ARNc spécifique du gène cible).

Si les différents polynucléotides marqueurs fixés sont détectables indépendamment sur un même support (par exemple par l'utilisation de différents chromophores ou fluorophores, ou fixés à différentes positions sélectionnées dudit support), les niveaux d'expression d'une pluralité de marqueurs peuvent être étudiés simultanément sur ce support unique.

Des méthodes appropriées pour la détection et la quantification par biopuces à polynucléotides fixés sont décrites dans l'état de la technique et peuvent par exemple être trouvées dans : Applications of DNA Microarrays in Biology. R.B. Stoughton (2005), Annu. Rev. Biochem. 74:53-82, ou dans David Bowtell and Joseph Sambrook, DNA Microarrays : A Molecular Cloning Manual, Cold Spring Harbor Laboratory Press, 2003 ISBN 0-870969-625-7.

Pour construire une telle puce à ADN fixé, des molécules d'acide nucléique sont attachées à un support solide à des endroits connus ou « addresses ».

Les molécules d'acide nucléique fixées sont complémentaires aux séquences nucléotidiques selon l'invention, et en particulier aux séquences SEQ ID N°1 à 28, et l'emplacement de chaque acide nucléique sur la puce est connu.

Ces puces avec polynucléotides fixés peuvent, par exemple, être réalisées en utilisant des méthodes (i) de dépôt de sondes pré-synthétisées, (ii) de synthèse in situ ou (iii) de photolithographie.

Les méthodes pour générer des polynucléotidiques marqués et pour l'hybridation à ces puces à ADN sont bien connues dans l'art antérieur (voir par exemple US 2002 / 0144307, et Ausubel et al., Eds. (1994) Current Protocols in Molecular Biology, Current Protocols (Greene Publishing Associates, Inc, et John Wiley & Sons, Inc, New York, 1994 Supplement)).

Dans le dispositif selon l'invention, l'hybridation entre deux acides nucléiques est avantageusement mise en oeuvre dans des conditions d'hybridation stringentes.

Le niveau d'expression, obtenu par la mise en oeuvre du dispositif selon l'invention, peut être exprimé avec une unité arbitraire qui reflète la quantité d'ARN messager transcrit pour chaque gène d'intérêt, tel que l'intensité d'un signal radioactif ou fluorescent émis par le matériel ADNc généré par une analyse PCR du contenu en ARN messager de l'échantillon étudié, incluant des techniques d'analyse PCR en temps réel de l'ARN messager contenu dans l'échantillon.

Toujours de manière alternative, cette valeur de l'expression des gènes peut être exprimée comme une unité arbitraire relative, corrigée par le niveau d'expression d'un ou plusieurs gènes de référence dits « rapporteurs ») qui ne sont pas modifiés dans l'échantillon par l'état de stimulation des défenses naturelles.

Dans ce dernier cas, la valeur de l'expression de chaque gène cible peut être exprimée comme la différence (deltaCT) entre (i) la quantité d'un ARNm formant marqueur biologique et (ii) la quantité d'un ARNm non marqueur, présent dans l'échantillon choisi par exemple parmi les gènes correspondant à la tubuline, l'actine, la GAPDH ou l'ubiquitine.

Pour cela, on peut encore se référer aux documents suivants : Livak et al. (2001, Methods 25:402-408) ou Vandesompele et al. (2002, Genome Biol. 3(7): research0034.1-0034.11).

Deux dispositifs préférés sont détaillés ci-après, sans toutefois constituer une limitation dans les techniques susceptibles d'être employées pour le dispositif selon l'invention.

### Quantification biologique de transcrits marqueurs, par amplification d'acides nucléiques

Le dispositif selon l'invention comprend avantageusement des moyens pour déterminer le niveau d'expression de la combinaison précitée de gènes cibles, par amplification d'acides nucléiques transcrits (ARNm ou ADNc) dans le cadre d'une technique dite de PCR quantitative.

La réaction de polymérisation en chaîne (PCR ou « Polymerase Chain Reaction ») est une méthode hautement efficace pour une telle quantification d'acides nucléiques constituant marqueurs biologiques.

Pour mettre en oeuvre de telles amplifications d'acides nucléiques dans un objectif de quantification des marqueurs biologiques d'intérêt, les moyens de détermination selon l'invention comprennent un ensemble de paires d'amorces, chaque paire d'amorces étant aptes à se fixer et à amplifier spécifiquement avec une cible ARNm ou avec une cible ADNc.

Selon cette technique, les ARN totaux sont extraits et purifiés et les ARN messager contenus dans l'extrait sont avantageusement convertis dans un premier temps en ADN complémentaires (ADNc), à l'aide d'une transcriptase inverse.

Une paire d'amorces apte à s'hybrider spécifiquement avec chacun des acides nucléiques marqueurs peut être dessinée par toute méthode appropriée, connue de la technique.

Les deux amorces sont respectivement choisies sur les brins sens et antisens, de manière à permettre l'amplification d'un fragment d'ADN.

Des paires d'amorces dégénérées ou spécifiques pour l'amplification des acides nucléiques SEQ ID n°1 à 28 (ou d'une partie ou d'un variant de ces séquences) peuvent être synthétisées à partir desdites séquences, ou des variants desdites séquences.

Avantageusement les amorces sens et anti-sens comprennent au moins 15 nucléotides et présentent une identité d'au moins 80 %, préférentiellement 90 %, et encore préférentiellement 95 %, et encore de préférence 100%, avec la séquence des gènes cibles, ou avec sa séquence complémentaire, ou avec les ADNc précités.

Des exemples de telles amorces spécifiques à chacune des séquences SEQ ID n°1 à 28 sont désignés par les séquences SEQ ID n°32 à 87 (tableau 3 dans la partie Exemple).

De plus, des exemples d'amorces spécifiques des gènes de référence SEQ ID n°29 à 31 sont désignés par les séquences SEQ ID n°88 à 93 (tableau 3).

Les moyens de détermination comportent encore des polymérases, préférentiellement la Taq polymérase, mais cela peut être toute autre enzyme présentant une activité polymérase et utilisable dans les conditions opératoires de la PCR.

Au cours de la réaction d'amplification, le produit de la réaction, désigné encore sous l'appellation amplimère ou amplicon, est détecté.

La quantité d'ARNm transcrits pour chaque gène cible est mesurée par des moyens autorisant la mise en oeuvre de la méthode dite de PCR quantitative ou QPCR.

La PCR quantitative permet la quantification d'une quantité initiale d'ADN, d'ADNc ou d'ARN dans l'échantillon préparé.

Elle est pour cela basée sur la détection d'un composé rapporteur fluorescent dont l'intensité de signal augmente lorsque le produit de la PCR s'accumule avec chaque cycle d'amplification.

Les moyens de détermination comportent ainsi des composés rapporteurs fluorescents, c'est-à-dire des composés qui se lient à l'ADN double brin (par exemple dans le cas d'une technique SYBR Green I) ou des sondes spécifiques (par exemple dans le cas d'une technique TaqMan®, d'une technique HybProbes (FRET) ou hybridation de 2 sondes, d'une technique Molecular Beacons ou balises moléculaires, et d'une technique Scorpion ou amorces scorpion).

Pour mettre en oeuvre cette technique PCR dans le cadre de la présente invention, on peut se référer aux revues générales sur les techniques PCR et aux instructions des fabricants et distributeurs de réactifs et de thermocycleurs, et en particulier à la notice explicative intitulée « Quantitation of DNA/RNA Using Real-Time PCR Détection » publiée par Perkin Elmer Applied Biosystems (1999) et au PCR Protocols (Académie Press New-York 1989).

On peut encore employer les informations développées notamment dans les documents suivants : Livak et al. (2001, Methods 25:402-408) et Vandesompele et al. (2002, Genome Biol. 3(7):research0034.1-0034.11).

L'un des avantages de la méthode de détection par QPCR est que l'analyse des produits de PCR se fait directement au cours des cycles de PCR, par la lecture de la fluorescence obtenue au cours des cycles. Il n'est donc pas nécessaire de travailler avec les produits de PCR, qui constitue un risque de contamination pour les analyses ultérieures. Cette détection a lieu avantageusement en pleine phase exponentielle de PCR et non en point final ; ce principe de détection est donc plus sensible et plus spécifique.

En outre la quantification du nombre de cibles initiales dans la réaction est très fiable et reproductible. La détection du produit de PCR se fait au cours des cycles PCR.

Un mode de réalisation particulier pour la quantification de tels marqueurs biologiques, sur la base d'un dispositif mettant en oeuvre une technique de quantification par amplification, est divulgué dans les exemples ci-après.

### Plante ou partie de plante étudiée au moyen du dispositif selon l'invention

Le dispositif selon l'invention s'avère en particulier intéressant pour l'étude de plantes, ou de parties de plantes, appartenant à la famille des *Rosaceae* ou Rosacées.

Ce dispositif sera en particulier intéressant pour étudier des plantes, ou parties de plantes, choisies parmi l'une des sous-familles suivantes : les *Amygdaloideae* (famille du pêcher), les *Maloideae* (famille du pommier), les *Rosoideae* (famille du rosier) et les *Spiraeoideae* (famille de la spirée).

Plus précisément encore, le dispositif selon l'invention sera intéressant pour étudier des plantes, ou parties de plantes, choisies parmi l'un des genres suivants : *Acaena, Adenostoma, Agrimonia, Alchemilla, Amelanchier, Aphanes, Aremonia, Aria, Aruncus, Bencomia,* *Brachycaulos, Cercocarpus, Chaenomeles, Chamaebatia, Chamaebatiaria, Chamaemeles, Chamaemespilus, Chamaerhodos, Cliffortia, Coleogyne, Coluria, Cormus, Cotoneaster, Cowania, Crataegus, Cydonia, Dalibarda, Dichotomanthes, Docynia, Docyniopsis, Dryas, Duchesnea, Eriobotrya, Eriolobus, Exochorda, Fallugia, Filipendula, Fragaria, Geum, Gillenia, Guamatela, Hagenia, Hesperomeles, Heterome*/*es, Holodiscus, Horkelia, Horkeliella, Ivesia, Kageneckia, Kelseya, Kerria, Leucosidea, Lindleya, Luetkea, Lyonothamnus, Maddenia, Malacomeles, Malus, Margyricarpus, Mespilus, Neillia, Neviusia, Nuttalia, Oemleria, Orthurus, Osteomeles, Pentactina, Peraphyllum, Petrophytum, Photinia, Physocarpus, Polylepis, Potanina, Potentilla, Poterium, Prinsepia, Prunus, Pseudocydonia, Purshia, Pyracantha, Pyrus, Quillaja, Rhaphiolepis, Rhodotypos, Rosa, Rubus, Sanguisorba, Sarcopoterium, Sibbaldia, Sibiraea, Sorbaria, Sorbus, Spenceria, Spiraea, Spiraeanthus, Stephanandra, Taihangia, Tetraglochin, Torminalis, Vauquelinia, Waldsteinia, Xerospiraea.*

De préférence, le dispositif selon l'invention est adapté pour étudier des plantes, ou parties de plantes, appartenant à l'une des espèces suivantes : *Prunus armeniaca, Prunus dulcis, Prunus avium, Cydonia oblonga, Rosa canina, Fragaria, Rubus idaeus, Rubus fruticosus agg, Mespilus germanica, Prunus persica, Rubus chamaemorus, Pyrus communis, Malus domestica, Prunus domestica.*

Encore de préférence, le dispositif est destiné à l'étude du pommier, ou *Malus domestica.*

Dans ce cas, le présent dispositif peut être employé pour toutes les variétés de pommier, et en particulier les variétés suivantes : Golden Delicious, MM106 et Evereste.

### Méthode utilisant le dispositif selon l'invention

De manière générale, le dispositif selon l'invention peut être employé pour l'étude de l'état de stimulation des défenses naturelles sur une plante ou une partie de la plante (ou une pluralité de plantes ou de parties de plantes).

En pratique, le profil d'expression d'une pluralité d'échantillons d'acides nucléiques, provenant de plusieurs plantes et/ou parties de plantes, est déterminé au moyen d'un ou d'une série de dispositifs selon l'invention.

Cette démarche permet de tenir compte des éventuelles variations dans l'expression des gènes entre les plantes et/ou parties de plantes, et ainsi obtenir des résultats fiables.

Parallèlement, des échantillons contrôles sont avantageusement étudiés et analysés avec les dispositifs selon l'invention.

Par exemple, les échantillons de contrôle (ou les échantillons de référence) peuvent être obtenus à partir de plantes ou de parties de plantes dont l'état de stimulation des défenses naturelles est connu.

Ainsi, les échantillons de contrôle peuvent être issus de plantes ou de parties de plantes qui sont non-traitées et non-stressées, ou qui sont soumises préalablement à un composé stimulateur des défenses naturelles et/ou un stress.

Ensuite, afin de déterminer l'état de stimulation des défenses naturelles de végétaux d'intérêt (ayant un état de stimulation des défenses naturelles inconnu), les échantillons obtenus à partir de ces plantes ou parties de plantes seront comparés avec des échantillons provenant de plantes ou parties de plantes dont l'état de stimulation des défenses naturelles est connu.

Ainsi, plusieurs échantillons peuvent être analysés au moyen du dispositif selon l'invention, tels que des échantillons d'acides nucléiques provenant (i) de plantes ou parties de plantes dont l'état de stimulation des défenses naturelles est connu, (ii) de plantes ou parties de plantes dont l'état de stimulation des défenses naturelles est inconnu, (iii) de plantes ou parties de plantes préalablement soumises à au moins un stress biotique et/ou abiotique ou (iv) de plantes ou parties de plantes préalablement soumises à au moins un produit pour vérifier ou pour rechercher un effet stimulateur des défenses naturelles.

Le dispositif selon l'invention peut ainsi être mis en oeuvre dans un ensemble de procédés, dont certains sont présentés en détails ci-après.

### Procédé pour identifier un profil d'expression d'une combinaison de gènes cibles

Une première application du dispositif selon l'invention est l'identification de profils d'expression de la combinaison précitée de gènes cibles, encore désignés « signatures », qui permettrait de déterminer, ou au moins d'évaluer, l'état de stimulation des défenses naturelles de plantes.

Le procédé pour identifier un profil d'expression d'une combinaison de gènes cibles comprend les étapes suivantes :
(i) déterminer le profil d'expression d'une combinaison de gènes cibles au moyen du dispositif selon l'invention, sur un ensemble de plantes, ou parties de plantes, appartenant avantageusement à la famille des *Rosaceae*, dont l'état de stimulation des défenses naturelles est connu, puis
(ii) déterminer un profil d'expression d'intérêt de ladite combinaison de gènes cibles correspondant à un état déterminé de stimulation des défenses naturelles desdites plantes ou parties de plantes, partant des données issues de l'étape (i).

Au moyen du dispositif selon l'invention et au cours de l'étape (i), on obtient au moins une valeur quantitative d'expression pour chacun des marqueurs biologiques utilisés.

L'obtention de plusieurs valeurs quantitatives, pour chaque marqueur biologique utilisé, permet un pronostic plus précis de l'état de stimulation des défenses naturelles.

Ces quantifications sont mises en oeuvre sur des échantillons de plantes ou de parties de plantes dont l'état de stimulation des défenses naturelles est connu, choisis avantageusement parmi :
- les échantillons de plantes ou de parties de plantes non soumises à un stress et/ou à un produit stimulateur des défenses naturelles ; ou
- les échantillons de plantes ou de parties de plantes soumises à un stress ; ou
- les échantillons de plantes ou de parties de plantes soumises à un produit stimulateur des défenses naturelles.

Par exemple, pour obtenir des échantillons représentatifs d'un stress biotique, on peut pulvériser du peroxyde d'hydrogène sur les plantes ou de parties de plantes.

Pour obtenir des témoins négatifs, on peut pulvériser de l'eau osmosée sur les plantes ou de parties de plantes.

Pour obtenir des échantillons représentatifs d'une protection vis-à-vis du feu bactérien, on peut appliquer du Bion® sur les plantes ou de parties de plantes.

L'étape (ii) consiste à comparer les valeurs quantitatives obtenues à l'étape (i) pour chaque marqueur biologique.

Les valeurs d'expression de référence pour chacun des gènes cibles peuvent ainsi être déterminées.

Elles constituent ensemble « un profil de référence » qui est pertinent pour distinguer et/ou identifier et/ou déterminer une modification de l'état de stimulation des défenses naturelles.

Chaque profil de référence déterminé pour ladite combinaison de marqueurs biologiques est ainsi corrélé à un état de stimulation des défenses naturelles.

Selon un mode de réalisation avantageux, les profils de référence, pour la combinaison de marqueurs biologiques selon l'invention, peuvent être prédéterminé par la réalisation d'un procédé comprenant les étapes suivantes :
a) fournir au moins une collection d'échantillons de plantes ou de parties de plantes, dont l'état de stimulation des défenses naturelles est déterminé ;
b) quantifier au moyen du dispositif selon l'invention, pour chaque échantillon fourni à l'étape a), le niveau d'expression desdits marqueurs biologiques, par lequel une série de valeurs quantitatives pour les marqueurs biologiques de ladite collection d'échantillons est obtenue ;
c) déterminer, à partir de ladite série de valeurs de quantification obtenue à l'issue de l'étape b), une corrélation entre un profil déterminé d'expression desdits marqueurs biologiques et un état spécifique de stimulation des défenses naturelles.

Certains profils d'expression intéressants, obtenus au moyen du dispositif selon l'invention, sont présentés dans les exemples.

### Procédé pour déterminer ou évaluer l'état de stimulation des défenses naturelles

Le dispositif selon l'invention a également l'intérêt de pouvoir être mis en oeuvre dans le cadre d'un procédé pour l'analyse de l'état de stimulation des défenses naturelles d'une plante ou partie de plante.

Le procédé pour déterminer ou évaluer l'état de stimulation des défenses naturelles d'une plante comprend les étapes suivantes :
(i) prélever un échantillon à partir de la plante ou à partir de ladite partie de plante, appartenant avantageusement à la famille des Rosaceae, ayant éventuellement subie tout traitement d'intérêt (par exemple stress ou stimulateur des défenses naturelles),
(ii) déterminer le profil d'expression d'une combinaison de gènes cibles dans ledit échantillon prélevé à l'étape (i), au moyen du dispositif selon l'invention,
(iii) comparer le profil d'expression obtenu à l'étape (ii) avec un profil d'expression de référence,
(iii) déterminer ou évaluer l'état de stimulation des défenses naturelles de ladite plante ou de ladite partie de plante, à partir dudit profil d'expression obtenu lors de l'étape (ii).

Le profil d'expression obtenu à l'étape (ii), au moyen du dispositif selon l'invention, est comparé avec un profil d'expression de référence qui correspondent aux valeurs observées pour des échantillons de plantes ou de parties de plantes choisis avantageusement parmi :
- les échantillons de plantes ou de parties de plantes non soumises à un stress ou un stimulateur des défenses naturelles ; ou
- les échantillons de plantes ou de parties de plantes soumises à un stress (par exemple E. *amylovora*) ; ou
- les échantillons de plantes ou de parties de plantes soumises à un stimulateur des défenses naturelles (par exemple Bion®).

En particulier, la surexpression d'au moins un gène cible, et de préférence encore d'une combinaison de gènes cibles, dans un échantillon étudié, en comparaison d'échantillons de plantes ou de parties de plantes non soumises à un stress ou un stimulateur des défenses naturelles, permet d'identifier les plantes ou parties de plantes présentant un état de stimulation des défenses naturelles qui est activé ou induit.

De même, l'expression constante d'au moins un gène cible, et de préférence encore d'une combinaison de gènes cibles, dans un échantillon étudié, en comparaison d'échantillons de plantes ou de parties de plantes soumises à un stress ou un stimulateur des défenses naturelles, permet d'identifier les plantes ou parties de plantes présentant un état de stimulation des défenses naturelles qui est activé ou induit.

### Procédé pour sélectionner une substance ayant la propriété de moduler l'état de stimulation des défenses naturelles

Il existe un besoin pour de nouveaux agents stimulateurs de défenses naturelles, et de méthodes fiables et faciles pour le dépistage d'un grand nombre de composés biologiques ou chimiques, ou de compositions, pour leur utilisation comme agents stimulateurs de défenses naturelles.

Il existe également un besoin de vérifier l'activité biologique de composés ou de compositions présentés comme stimulateurs de défenses naturelles.

Le dispositif selon l'invention constitue un outil particulièrement intéressant pour identifier de tels composés biologiques ou chimiques, ou de telles compositions contenant de ces composés biologiques ou chimiques, qui soient capables de stimuler les défenses naturelles d'une plante ou partie de plante, appartenant avantageusement à la famille des *Rosaceae.*

L'outil selon l'invention permet en particulier de cribler toute composé actif ou composition active, biologique ou chimique, employé dans le domaine de l'agriculture, de préférence de l'arboriculture et de préférence encore dans le domaine des *Maloideae.*

L'identification d'un tel effet supplémentaire permettrait d'éviter les traitements redondants (notamment par l'application de deux composés activant les mêmes mécanismes de défenses) et de limiter les interactions négatives (certains produits SDN ont des effets antagonistes au regard des voies moléculaires qu'ils régulent).

Le procédé de mise en oeuvre du dispositif selon l'invention comprend avantageusement les étapes suivantes :
(i) la mise en contact, avec une plante ou partie de la plante (appartenant avantageusement à la famille des *Rosaceae*), d'un ou plusieurs composés biologiques ou chimiques, ou des compositions comprenant un ou plusieurs composés biologiques ou chimiques,
(ii) la détermination du profil d'expression de la combinaison de gènes cibles dans un échantillon prélevé à partir de ladite plante traitée ou de ladite partie de plante traitée suite à l'étape (i), au moyen du dispositif selon l'invention,
(iii) la comparaison du profil d'expression obtenu à l'étape (ii) avec un profil d'expression de référence, pour déterminer ou évaluer l'état de stimulation des défenses naturelles dans ledit échantillon,
(iv) la sélection positive de ladite substance si la comparaison à l'étape (iii) montre que ladite substance testée à l'étape (i) module l'état de stimulation des défenses naturelles dudit plant ou de ladite partie de plant.

Dans l'étape (i), tout composé biologique ou chimique, ou toute composition comprenant un ou plusieurs composés biologiques ou chimiques, peuvent être mis en contact avec les plantes ou parties de plantes.

Au moins deux composés et/ou compositions différents peuvent être mise en contact, simultanément ou successivement, avec les plantes ou parties de plantes.

De préférence, chaque composé ou composition est mis en contact physique avec une ou plusieurs plantes individuelles.

Ce contact peut également être réalisé par différents moyens, tels que la pulvérisation, le brossage, l'application de solutions ou de solides dans ou sur la terre, dans la phase gazeuse entourant les plantes ou parties de plantes, par immersion, etc.

Les composés ou compositions testées peuvent être solides, liquides, semi-solides ou gazeux.

Les composés ou compositions testées peuvent être synthétisés artificiellement ou naturels, comme les protéines, fragments de protéines, composés organiques volatils, plantes ou animaux ou extraits de micro-organisme, les métabolites, les sucres, les graisses et huiles, les microorganismes comme les virus, bactéries, champignons, etc.

Le composé ou la composition biologique comprend encore, ou est constituée, d'un ou de plusieurs micro-organismes, ou un ou plusieurs extraits de plantes.

Le profil d'expression obtenu à l'étape (ii), au moyen du dispositif selon l'invention, est comparé selon une méthode décrite précédemment, c'est-à-dire par exemple avec un profil d'expression de référence qui correspondent aux valeurs observées pour des échantillons de plantes ou de parties de plantes choisis avantageusement parmi :
- les échantillons de plantes ou de parties de plantes non soumises à un stress ou un stimulateur des défenses naturelles ; ou
- les échantillons de plantes ou de parties de plantes soumises à un stress (par exemple *E*. *amylovora*) ; ou
- les échantillons de plantes ou de parties de plantes soumises à un stimulateur des défenses naturelles (par exemple Bion®).

En particulier, la surexpression d'au moins un gène cible, et de préférence encore d'une combinaison de gènes cibles, dans un échantillon étudié, en comparaison d'échantillons de plantes ou de parties de plantes non soumises à un stress ou un stimulateur des défenses naturelles, permet d'identifier les plantes ou parties de plantes présentant un état de stimulation des défenses naturelles du fait de l'action du composé testé ou de la composition testée.

Par exemple, la surexpression de la combinaison de gènes cibles PR-1, PR-2, PR-4, PR-5, PR-8, PR-14, HMGR, Far, CSL, POX, Pect, EDS1 et WRKY permet de sélectionner une substance ayant la propriété de générer un état de stimulation des défenses naturelles d'une plante ou d'une partie de plante appartenant à la famille des Rosaceae, qui assure une protection vis à vis de stress biotiques.

De préférence, ce profil d'expression permet la sélection d'une substance ayant la propriété de générer un état de stimulation des défenses naturelles d'une plante ou d'une partie de plante appartenant à la famille des *Rosaceae,* qui assure une protection vis à vis de *Erwinia amylovora.*

En particulier, cette surexpression pour chacun des gènes cibles précités consiste en un niveau de surexpression relative (avantageusement par rapport à un traitement à l'eau) supérieur à 3 (ou log₂(expression relative)>1,58).

De même, l'expression constante d'au moins un gène cible, et de préférence encore d'une combinaison de gènes cibles, dans un échantillon étudié, en comparaison d'échantillons de plantes ou de parties de plantes soumises à un stimulateur des défenses naturelles, permet d'identifier les plantes ou parties de plantes présentant un état de stimulation des défenses naturelles du fait de l'action du composé testé ou de la composition testée.

La plante ou partie de plante traitée peut éventuellement être soumise simultanément à un stress abiotique (environnemental), afin d'étudier l'effet de conditions de culture stressantes sur l'efficacité des stimulateurs de défenses naturelles.

L'outil selon l'invention peut permettre de sélectionner, et distinguer, des composés stimulateurs directs et des composés potentialisateurs.

Pour cela, on peut par exemple étudier le changement du profil d'expression des gènes cibles suite à l'application d'un stress biotique, avec ou sans application préalable d'un composé ou d'une composition d'intérêt.

La surexpression d'une combinaison de gènes cibles dans un échantillon après l'application d'un stress biotique, en comparaison d'échantillons de plantes ou de parties de plantes non soumise à un tel stress biotique, permet d'identifier les composés potentialisateurs (ou au moins susceptible d'avoir un tel effet potentialisateur).

### Procédé pour sélectionner uneplante ou une partie de plante

La présente invention porte également sur l'identification et la sélection de plantes, appartenant avantageusement à la famille des *Rosaceae*, présentant un état particulier de stimulation des défenses naturelles, que cet état soit obtenu :
(a) naturellement (par croisement ou l'utilisation des variations naturelles) ou artificiellement (en induisant des variations, par exemple par transgénèse des plantes ou des parties de plantes, ou par mutagénèse en utilisant un ou plusieurs agents mutagènes) ; et/ou
(b) suite à l'application d'un composé ou d'une composition stimulatrice des défenses naturelles ; et/ou
(c) suite à l'application d'un ou de plusieurs stress biotique(s) et/ou abiotique(s).

En effet, le dispositif selon l'invention constitue un outil intéressant pour sélectionner une plante, appartenant de préférence à la famille des *Rosaceae*, présentant un état de stimulation des défenses naturelles susceptible de leur conférer une résistance améliorée à au moins un stress biotique et/ou abiotique d'intérêt.

Une méthode pour sélectionner une plante ou partie de la plante ayant un état de stimulation des défenses naturelles améliorée (et donc avec résistance renforcée aux stress biotiques et/ou abiotiques), peut comprendre les étapes suivantes :
(i) appliquer le ou les stress, et/ou le ou les stimulateurs de défenses naturelles, à une plante ou une partie de plante, appartenant avantageusement à la famille des *Rosaceae,*
(ii) déterminer le profil d'expression d'une combinaison de gènes cibles dans un échantillon prélevé à partir de ladite plante ou de ladite partie de plante, au moyen du dispositif selon l'invention,
(iii) comparer le profil d'expression obtenu à l'étape (ii) avec un profil d'expression de référence, pour déterminer ou évaluer l'état de stimulation des défenses naturelles dans ledit échantillon,
(iv) sélectionner positivement ladite plante si la comparaison de l'étape (iii) montre que ledit plant ou ladite partie de plant possède un état de stimulation des défenses naturelles susceptible de leur conférer une résistance améliorée à au moins un stress biotique et/ou abiotique d'intérêt.

La plante ou partie de la plante sélectionnée à l'étape (iv) comporte avantageusement un état particulier de stimulation des défenses naturelles, en présence d'agents stimulateurs des défenses naturelles et/ou de stress biotiques et/ou de stress abiotiques.

Le profil d'expression obtenu à l'étape (ii), au moyen du dispositif selon l'invention, est comparé selon une méthode décrite précédemment, c'est-à-dire par exemple avec un profil d'expression de référence qui correspondent aux valeurs observées pour des échantillons de plantes ou de parties de plantes choisis avantageusement parmi :
- les échantillons de plantes ou de parties de plantes non soumises à un stress ou un stimulateur des défenses naturelles ;
- les échantillons de plantes ou de parties de plantes soumises à un stimulateur des défenses naturelles ; ou
- les échantillons de plantes ou de parties de plantes soumises à un stress.

La surexpression d'au moins un gène cible, et de préférence encore d'une combinaison de gènes cibles, dans un échantillon étudié, en comparaison d'échantillons de plantes ou de parties de plantes soumises à un stress ou un stimulateur des défenses naturelles, permet d'identifier les plantes ou parties de plantes présentant un état de stimulation des défenses naturelles susceptible de présenter une résistance améliorée à au moins un stress biotique et/ou abiotique d'intérêt.

Le dispositif selon l'invention permet ainsi de cribler les génotypes pour leur réactivité aux produits SDN.

### Séquences

SEQ ID N°1 à 31 : séquences des ADNc issus des gènes cibles et des gènes de références ; SEQ ID N°32 à 93 : séquences d'amorces PCR pour l'amplification des séquences SEQ ID N°1 à 31

### Figures

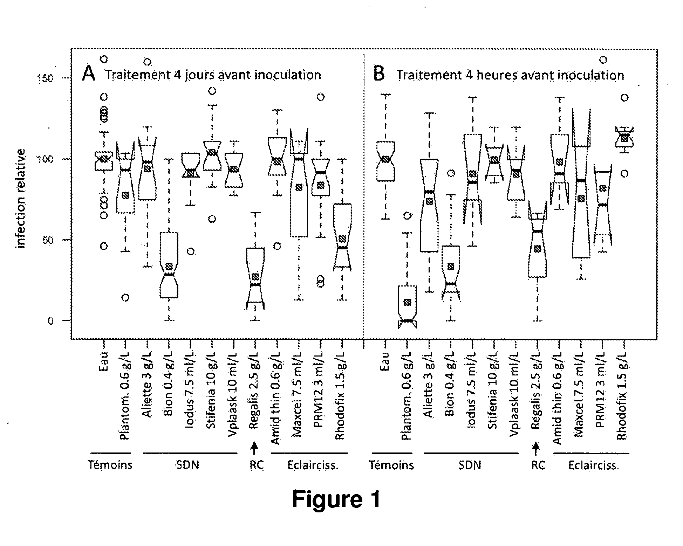

Figure 1 : Efficacité de protection de 10 SDN candidats contre *E*. *amylovora* sur semis de Pommier. L'eau et la Plantomycine (Plantom.) sont utilisées comme témoins négatifs et positifs respectivement.
   RC : régulateur de croissance ; Eclairciss : éclaircisseurs ; Représentation en boîtes à moustaches, ces dernières indiquant les intervalles de confiance des médianes (P=0.05) ; Au moins six répétitions biologiques de 10 semis de 2 expériences indépendantes.

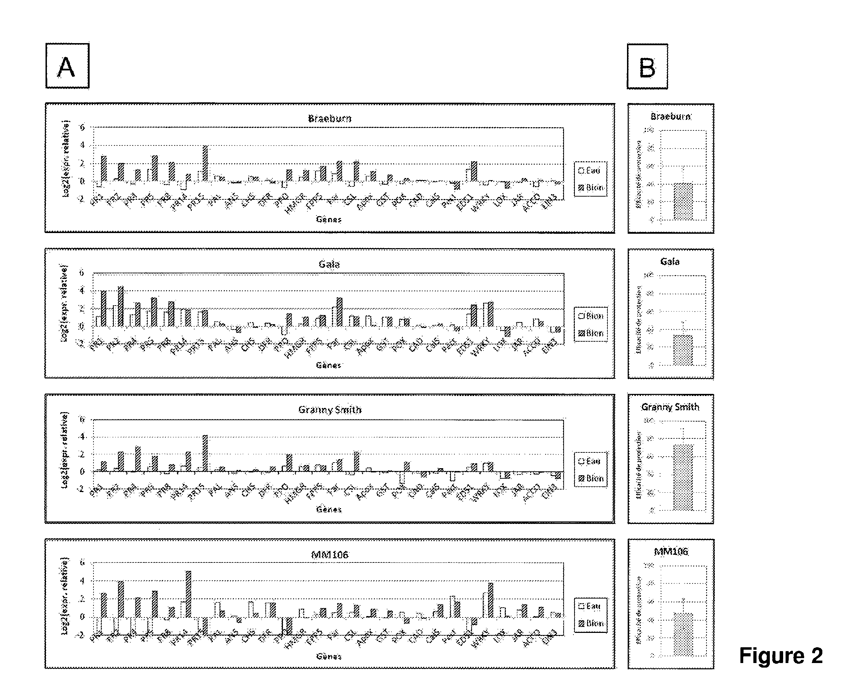

Figure 2 : A) Modulation de l'expression des 28 gènes de l'outil dans des feuilles de 4 variétés de Pommier (scions greffés) 3 jours après traitement au Bion ou à l'eau. Différences d'expression par rapport à un prélèvement effectué à J0 sur des plantes non traités de chaque génotype. Une répétition biologique. B) Efficacité moyenne de protection vis-à-vis du feu bactérien effectuée en parallèle (n=12).

### Exemple

### Matériel :

### Dispositif selon l'invention

Plaque de microtitration 96-puits prête à l'emploi, contenant les couples d'amorces SEQ ID n°32 à 93 pour (i) les 28 gènes cibles SEQ ID n°1 à 28 et (ii) les 3 gènes de référence SEQ ID n°29 à 31, (répartis dans trois puits pour chaque couple) sous forme déshydratées (évaporation une nuit à 60°C) et en quantité suffisante pour atteindre les concentrations finales optimales détaillées dans le tableau 3 ci-dessous.

**Tableau 3 : Amorces et concentrations optimales pour la technique PCR**

| **Nom du gène** | **Amorces** | **SEQ ID** | **Taille amplicon (bp)** | **Concentration optimale (nm)** |
|---|---|---|---|---|
| **PR-1** | PR1-di | 32 | 167 | 200 |
| | PR1-re | 33 | | |
| **PR-2** | PR2-di | 34 | 152 | 400 |
| | PR2-re | 35 | | |
| **PR-4** | PR4-di | 36 | 123 | 200 |
| | PR4-re | 37 | | |
| **PR-5** | PR5-di | 38 | 100 | 600 |
| | PR5-re | 39 | | |
| **PR-8** | PR8-di | 40 | 173 | 200 |
| | PR8-re | 41 | | |
| **PR-14** | PR14-di | 42 | 192 | 200 |
| | PR14-re | 43 | | |
| **PR-15** | PR15-di | 44 | 132 | 200 |
| | PR15-re | 45 | | |
| **PAL** | PAL-di | 46 | 133 | 200 |
| | PAL-re | 47 | | |
| **CHS** | CHS-di | 48 | 234 | 100 |
| | CHS-re | 49 | | |
| **DFR** | DFR-di | 50 | 239 | 200 |
| | DFR-re | 51 | | |
| **ANS** | ANS-di | 52 | 298 | 200 |
| | ANS-re | 53 | | |
| **PPO** | PPO-di | 54 | 124 | 200 |
| | PPO-re | 55 | | |
| **HMGR** | HMGR-di | 56 | 206 | 400 |
| | HMGR-re | 57 | | |
| **FPPS** | FPPS-di | 58 | 255 | 200 |
| | FPPS-re | 59 | | |
| **Far** | Far-di | 60 | 161 | 200 |
| | Far-re | 61 | | |
| **CSL** | CSL-di | 62 | 134 | 200 |
| | CSL-re | 63 | | |
| **APOX** | APX-di | 64 | 185 | 200 |
| | APX-re | 65 | | |
| **GST** | GST-di | 66 | 185 | 200 |
| | GST-re | 67 | | |
| **POX** | POX-di | 68 | 197 | 100 |
| | POX-re | 69 | | |
| **CalS** | CalS-di | 70 | 176 | 200 |
| | CalS-re | 71 | | |
| **Pect** | Pect-di | 72 | 173 | 300 |
| | Pect-re | 73 | | |
| **CAD** | CAD-di | 74 | 85 | 200 |
| | CAD-re | 75 | | |
| **EDS1** | EDS1-di | 76 | 269 | 200 |
| | EDS1-re | 77 | | |
| **WRKY** | WRKY-di | 78 | 171 | 200 |
| | WRKY-re | 79 | | |
| **LOX2** | LOX2-di | 80 | 199 | 100 |
| | LOX2-re | 81 | | |
| **JAR** | JAR-di | 82 | 118 | 200 |
| | JAR-re | 83 | | |
| **ACCO** | ACCO-di | 84 | 193 | 200 |
| | ACCO-re | 85 | | |
| **EIN3** | EIN3-di | 86 | 212 | 400 |
| | EIN3-re | 87 | | |
| **TuA** | TuA-di | 88 | 118 | 300 |
| | TuA-re | 89 | | |
| **Actin** | Actin-di | 90 | 140 | 100 |
| | Actin-re | 91 | | |
| **GAPDH** | GAPDH-di | 92 | 103 | 300 |
| | GAPDH-re | 93 | | |

### Essai 1 - Expression relative des gènes de défense dans des feuilles de pommiers soumis à différents traitements

### Protocole :

L'expression des 28 gènes cibles a été suivie (i) dans des feuilles de semis de Pommier (obtenus par pollinisation libre de la variété Golden Delicious, voir Brisset et al. Eur. J. Plant Pathol 106, 529-536, 2000) soumis à des traitements de type SDN et à divers stress abiotiques, ainsi que (ii) dans des feuilles de scions greffés de deux génotypes de Pommier, Evereste et MM106 (voir Venisse et al. Molecular Plant-Microbe Interactions 15, 1204-1212, 2002), après infection par *Erwinia amylovora,* agent du feu bactérien.

Le protocole mis en oeuvre comprend les étapes 1 à 7 suivantes :
1) Traitement des plants par pulvérisation, arrosage ou infiltration du produit (ou d'eau osmosée pour les témoins négatifs).
   Les divers traitements ont été appliqués de la manière suivante :
   - Baba (a.i. 95% acide DL-ß-amino-n-butyrique, Sigma ref A-2004) appliqué par arrosage d'une solution 10 mM (5 ml par pot de 150 ml),
   - Bion® (a.i. 50% acibenzolar-S-methyl, Syngenta) appliqué par pulvérisation jusqu'à ruissellement d'une solution à 0,4 g/l,
   - Rhodofix® (a.i. 1% acide α-naphtylacétique, Nufarm SAS) appliqué par pulvérisation jusqu'à ruissellement d'une solution à 1,5 g/l,
   - Régalis® (a.i. 10% prohexadione-calcium, BASF Agro, BASF Agro) appliqué par pulvérisation jusqu'à ruissellement d'une solution à 2,5 g/l,
   - PRM® 12 RP (a.i. 11,3% éthéphon, Bayer CropScience) appliqué par pulvérisation jusqu'à ruissellement d'une solution à 3 ml/l,
   - MeJA (a.i. 95% methyl jasmonate, Aldrich ref 39,270-7) appliqué par arrosage d'une solution 10 mM (5 ml par pot de 150 ml),
   - Deshydratation appliquée par arrêt de l'arrosage,
   - PEG (a.i. polyéthylène glycol 6000, Merck ref 807491) appliqué par imbibition jusqu'à saturation du terreau (2 fois 3 mn) à l'aide d'une solution à 36%,
   - Paraquat (a.i. paraquat dichloride x-hydrate PESTANAL®, Fluka ref 36541) appliqué par pulvérisation jusqu'à ruissellement d'une solution 100 µM,
   - H₂O₂ (a.i. 30% peroxyde d'hydrogène) appliqué par pulvérisation jusqu'à ruissellement d'une solution à 10 ml/l,
   - *E. amylovora* appliqué par infiltration d'une suspension de la souche CFBP1430 ajustée à 10⁷ cfu/ml.
2) Pulvérisation optionnelle de peroxyde d'hydrogène 24h après l'étape 1), pour mimer l'attaque par un pathogène (ce qui permet de différencier les stimulateurs directs et les potentialisateurs).
3) Prélèvement de tissus 24h, 48h et 72h après traitement.
4) Congélation immédiate des tissus dans l'azote liquide et conservation à -80°C.
5) Extraction d'ARN, reverse-transcription, vérification d'absence d'ADN génomique selon Venisse et al. (Molecular Plant-Microbe Interactions 15, 1204-1212, 2002).
6) Amplification par PCR quantitative de chaque extrait en préparant le mix suivant : 5 µg d'ADNc, X µl de mix qPCR selon les instructions du fournisseur, et qsp 2500 µl eau ultrapure, en le distribuant à raison de 25 µl par puits de la plaque 96-puits prête à l'emploi, et en effectuant l'amplification selon un programme qPCR de 40 cycles.
7) Exploitation des résultats selon la méthode du ΔΔCt qui fournit les expressions relatives des gènes de défense dans un échantillon donné par rapport à un échantillon non traité appelé « calibrateur », expressions normalisées par la moyenne géométrique des gènes de référence de ces échantillons, (Vandesompele et al., Genome Biol. 3(7) : research0034.1-0034.11, 2002 ; Livak et Schmittgen, Methods 25:402-408, 2001).

Les résultats d'expression des 28 gènes cibles sont détaillés dans les tableaux 4, 5 et 6 ci-après, consistant en une matrice des expressions relatives par rapport à des témoins « eau » (prélevés aux mêmes temps) transformées en log de base 2 (J=jour, h=heure).
Ces résultats sont répartis dans trois tableaux, uniquement dans un souci de présentation.

### Résultats :

a) Les produits Bion, Rhodofix et Régalis montrent une activation assez similaire de la combinaison de gènes suivants: PR-1, PR-2, PR-4, PR-5, PR-8, PR-14, HMGR, Far, CSL, POX, Pect, EDS1 et WRKY, avec des niveaux de surexpression relative (par rapport à l'eau) souvent bien supérieurs à 3 (ou log₂(expression relative)>1,58, tableaux 4 à 6).
   Ces trois produits assurent par ailleurs une protection significative et reproductible vis-à-vis du feu bactérien.
   Il est intéressant de noter que parmi ces 3 produits, seul le Bion (analogue de l'acide salicylique) est homologué comme SDN (sur des espèces autres que le Pommier).
   Les inventeurs ont déjà montré son efficacité de protection vis-à-vis du feu bactérien, mais également envers la tavelure et le puceron cendré, protection corrélée à l'accumulation de quelques protéines de défense (Brisset et al., 2000 et 2005).
   Le Rhodofix (analogue d'auxine) est un éclaircisseur utilisé dans les vergers de Pommier pour alléger la charge en fruits.
   D'après les présents essais, ce produit aurait également des effets de stimulation de défense, ce qui n'a jamais été décrit.
   Quant au Régalis (inhibiteur de gibbérellines par inhibition de dioxygénases), il est également utilisé dans les vergers de Pommier comme réducteur de croissance des pousses. Les inventeurs ont déjà démontré sa capacité de protection vis-à-vis du feu bactérien, qui pouvait être liée à la réduction de croissance (Brisset et al., 2005).
   Les analyses d'expression de gènes montrent qu'il a également la capacité d'induire des défenses, en particulier de type protéines PR, ce qui, là encore, n'a jamais été décrit.
b) Les deux produits MeJA et PRM 12 montrent de leur côté une activation assez similaire d'une combinaison de gènes de défense : PR-14, PAL, CHS, DFR, CSL, Pect, ACCO et EIN3 avec des niveaux de surexpression relative par rapport à l'eau souvent supérieurs à 3 (ou log₂(expression relative)>1,58, tableaux 4 à 6).
   Au niveau protection, ces deux produits montrent une efficacité très variable suivant les essais mais, dans le meilleur des cas, n'atteint jamais l'efficacité des produits Bion, Rhodofix et Régalis.
   Il est à remarquer que PRM 12 (analogue de l'éthylène) est également un éclaircisseur utilisé dans les vergers de Pommier.
   Au vu des présents résultats, le Rhodofix devrait être préféré au PRM 12 pour sa double fonction d'éclaircisseur et de stimulateur de défenses.
c) Le Baba appliqué dans nos conditions expérimentales (arrosage) montre peu d'effet sur les modulations de gènes et aucun effet de protection vis-à-vis du feu bactérien.
   Il représente donc un témoin négatif dans nos expériences. Il valide également l'utilisation de l'H₂O₂ dans le mode opératoire. En effet le Baba est connu comme potentialisateur et non comme stimulateur direct, son action sur les défenses n'étant révélée qu'après le stress provoqué par un agent pathogène.
   Dans notre mode opératoire, l'action de ce dernier est simulée par l'application d'H₂O₂. Les résultats montrent bien que ce traitement supplémentaire déclenche l'expression de quelques gènes (POX, Pect par exemple) qui ne sont pas activés par le seul traitement Baba.
   Ce phénomène en revanche n'est pas clairement observé pour les autres stimulateurs qui semblent donc être plutôt des stimulateurs directs.
d) Vu dans leur ensemble, les résultats obtenus avec les 6 produits type SDN semblent indiquer que la surexpression des gènes PR-1, PR-2, PR-4, PR-5, PR-8, PR-14, HMGR, Far, CSL, POX, Pect, EDS1 et WRKY (avantageusement avec des niveaux d'expression relative, par rapport à l'eau, supérieurs à 3), permet d'assurer une protection significative vis-à-vis du feu bactérien en conditions contrôlées.
   En revanche, la surexpression des gènes PR-14, PAL, CHS, DFR, CSL, Pect, ACCO et EIN3 (avec des niveaux d'expression relative par rapport à l'eau avantageusement supérieurs à 3) ne permet pas d'assurer cette protection.
e) Les stress abiotiques ont été appliqués en vue de répondre à deux questions : i) peut-on avoir des artefacts sur l'expression des gènes de défense si les expérimentations de produits type SDN se déroulent dans des conditions environnementales plus contraignantes pour les plantes (c'est-à-dire observer des activations de gènes dues aux conditions stressantes et non aux SDN) et ii) des conditions environnementales stressantes empêchent-elles la réaction des plantes aux SDN (ce qui aurait alors un impact pour la réussite des traitements en verger).
   Les résultats actuels répondent en partie à la première question. Les stress hydrique (déshydratation), osmotique (PEG) et faiblement oxydant (H₂O₂ - servant également de témoin pour les tests de potentialisation) ne modulent que faiblement les expressions des gènes cibles. Seul un fort stress oxydant (paraquat = herbicide provoquant la production d'ion superoxyde) active fortement quelques gènes mais dans l'ensemble, les activations sont plus faibles que celles obtenues avec le Bion par exemple.
f) Les réponses des gènes à une infection par *E. amylovora* montre que cette bactérie module dans l'ensemble les mêmes gènes cibles que les traitements Bion, Rhodofix ou Régalis, mais souvent avec une amplitude plus faible en particulier pour les gènes de protéines PR.

Comme la bactérie réprime fortement les gènes des voies de l'acide jasmonique et des phenylpropanoïdes on aurait pu s'attendre a ce que des produits provoquant l'effet inverse de type MeJA ou PRM 12 soient efficaces en terme de protection ce qui n est pas le cas dans nos conditions expérimentales.

Les résultats dans leur ensemble montrent au contraire que les produits qui assurent une protection significative activent la même combinaison de gènes que la bacténe elle-même (PR-1, PR-2, PR-4, PR-5, PR-8 PR-14 HMGR Far, CSL, POX, Pect EDS1 et WRKY).

**Tableau 4**

| Expression relative des gènes de défense dans des feuilles de pommiers soumis à différents traitements (SDN potentiels, stress abiotiques, infection par E. amylovora) | | | | | | | | | | | |
|---|---|---|---|---|---|---|---|---|---|---|---|
| **Traitements** | | **Délai de prélèvement** | **PR-1** | **PR-2** | **PR-4** | **PR-5** | **PR-8** | **PR-14** | **PR-15** | **PAL** | **ANS** |
| **Baba** | **-H₂O₂** | **J2** | **1,85** | **3,04** | **3,00** | **2,75** | 0,02 | -0,29 | **1,84** | 0,42 | 1,28 |
| | | **J2** | -0,70 | 0,24 | -0,71 | 0,92 | 0,10 | ***-2,34*** | -0,53 | 0,27 | 1,53 |
| | | **J3** | 0,01 | 0,20 | ***-2,45*** | -0,26 | -0,37 | ***-3,35*** | 0,04 | 0,20 | -1,09 |
| | | **J3** | **2,08** | ***-1,69*** | -0,57 | 0,86 | -0,52 | **3,90** | **1,82** | 0,29 | -0,10 |
| | **+H₂O₂** | **J2** | **5,93** | **8,90** | **6,58** | **2,84** | **3,33** | **6,56** | 084 | 029 | 1,41 |
| | | **J2** | 0,08 | -0,98 | 0,16 | 0,40 | 0,39 | ***-2,63*** | 0,71 | 0,55 | 2,12 |
| | | **J3** | **2,71** | **4,26** | ***-2,18*** | **1,91** | 1,39 | -0,53 | 1,46 | -0,84 | 0,19 |
| | | **J3** | 1,77 | **-2,32** | -0,86 | 1,34 | -1,00 | 1,17 | **2,06** | -0,12 | 1,25 |
| **Bion** | **-H₂O₂** | **J2** | **8,37** | **6,89** | **8,61** | **5,77** | **6,07** | **3,31** | **1,88** | -0,14 | 1,51 |
| | | **J2** | **3,20** | **9,64** | **7,17** | 1,02 | **3,50** | **3,75** | **1,61** | 1,11 | 0,21 |
| | | **J3** | **5.99** | **7,99** | **5,43** | **4,46** | **2,23** | **4,09** | **-1,98** | -1,29 | -1,56 |
| | | **J3** | **2,14** | 1.61 | **8,07** | **3,88** | **1,62** | 049 | **4,96** | -0,15 | 0,14 |
| | **+H₂O₂** | **J2** | **8,53** | **11,96** | **10,25** | **6,52** | **5,57** | **2,22** | **4,09** | -0,25 | -0,15 |
| | | **J2** | **3,87** | **6,36** | **5,10** | **2,43** | **3,25** | **2,95** | **1,72** | 1,91 | 1,50 |
| | | **J3** | **7,66** | **13,12** | **6,37** | **4,11** | **2,74** | **5,01** | 1,33 | **-2,25** | **-4,05** |
| | | **J3** | **5,35** | **7,07** | **8,03** | **5,68** | **3,62** | **4,52** | **3,60** | 0,83 | 0,14 |
| **Rhodofix** | **-H₂O₂** | **J2** | **2,61** | **9,34** | **6,87** | **1,75** | **3,16** | **5,43** | 0,98 | **1,78** | 2,40 |
| | | **J3** | **5,99** | **7,56** | **9,06** | **3,15** | **3,56** | **6,59** | **2,93** | 0,32 | 0,77 |
| | **+H₂O₂** | **J2** | **3,87** | **6,85** | **7,42** | **2,72** | **3,19** | **4,49** | **4,83** | 1,15 | 149 |
| | | **J3** | **6,47** | **6,19** | **8,82** | **3,13** | **3,74** | **4,29** | **3,15** | 0,14 | 1,29 |
| **Régalis** | **-H₂O₂** | **J2** | **4,76** | **3,80** | **3,25** | **2,42** | **4,07** | **7,55** | **2,77** | 0,22 | 1,31 |
| | | **J2** | **2,08** | **5,91** | **3,45** | -0,84 | **2,29** | **3,31** | 1,12 | 1,36 | 0,77 |
| | | **J3** | **3,62** | **4,38** | **3,09** | 0,96 | 1,36 | 079 | -0.08 | 0,64 | -0,05 |
| | | **J3** | **4,11** | **3,84** | **3,80** | 122 | **1,91** | **6,20** | 1,37 | 0,58 | 2,70 |
| | **+H₂O₂** | **J2** | **4,02** | **11,09** | **8,82** | **4,24** | **4,07** | **5,07** | 063 | 0,81 | **2,37** |
| | | **J2** | **1,85** | 1,57 | **6,39** | ***-1,92*** | **2,72** | **4,80** | **6,12** | **2,69** | **1,92** |
| | | **J3** | **4,00** | **8,88** | **2,56** | 1,46 | 1,00 | **4,66** | **3,73** | -0,51 | ***-1,58*** |
| | | **J3** | **1,99** | 0,07 | **2,72** | **1,66** | -0,39 | **2,50** | 0,54 | 0,12 | 0,06 |

| **Traiteme nts** | | **Délai de prélève ment** | **PR-1** | **PR-2** | **PR-4** | **PR-5** | **PR-8** | **PR-14** | **PR-15** | **PAL** | **ANS** |
|---|---|---|---|---|---|---|---|---|---|---|---|
| **PRM12** | **-H₂O₂** | **J2** | 0,34 | **2,00** | 0,96 | 0,61 | 0,37 | 0,16 | 0,30 | 0,41 | 0,19 |
| | | **J3** | 1,13 | -1,11 | **5,13** | -0,73 | **2,56** | **5,92** | **5,40** | **4,10** | **4,49** |
| | **+H₂O₂** | **J2** | **2,41** | **1,94** | 0,14 | -0,17 | 0,91 | **1,90** | **2,46** | 1,38 | **3,45** |
| | | **J3** | **1,89** | ***-2,70*** | 0,21 | **1,64** | -0,30 | **5,05** | **3,13** | -0,33 | **2,86** |
| **MeJA** | **-H₂O₂** | **J2** | 1,56 | **4,03** | **2,68** | -0,59 | **1,85** | **3,45** | -0,19 | **3,17** | **2,31** |
| | | **J3** | **2,35** | **3,69** | **2,63** | 1.49 | 1.00 | **5,09** | 1.21 | **1,66** | **1,58** |
| | **+H₂O₂** | **J2** | 0,40 | **3,89** | 1,25 | ***-2,09*** | 1,41 | -0,64 | **3,20** | **3,36** | **3,29** |
| | | **J3** | **2,84** | 0,66 | **2,84** | -1,22 | 1.51 | **3,66** | **2,77** | **2,01** | 1,44 |
| **Deshydratation** | | **J2** | **-2,01** | ***-1,80*** | ***-4,15*** | 0,57 | 0,39 | ***-2,11*** | 1,82 | ***-2,26*** | 0,60 |
| | | **J4** | -0,91 | ***-1,65*** | ***-4,21*** | 0,00 | 0,00 | -0,56 | 0,00 | -0,12 | 0,00 |
| | | **J7** | 1,12 | -1,28 | **-4,36** | 1,20 | 0,70 | **2,71** | **3,01** | -1,54 | -1,25 |
| | | **J10** | 0,47 | -1,06 | **-3,33** | **2,49** | **2,15** | **3,09** | **2,66** | **-2,12** | -1,24 |
| **PEG** | | **J1** | **2,56** | **4,72** | **3,01** | 0,00 | 0,00 | 1,46 | 0,00 | 0,41 | 0,00 |
| | | **J2** | **4,07** | 0,29 | 1,00 | **-3,97** | -1,15 | **6,49** | -0,87 | 0,20 | 1,02 |
| | | **J2** | **-2,07** | **-2,05** | **-5,05** | 1,44 | 0,15 | **3,82** | 1,10 | **-4,43** | **-1-86** |
| | | **J3** | 1,07 | 0,69 | **3,92** | **-11.1** | **1,75** | **5,87** | **-6,54** | 0,68 | **3,82** |
| | | **J4** | -1,32 | -1,24 | **1,64** | -0,20 | **1,60** | **5,33** | **1,83** | -0,64 | 0,02 |
| | | **J7** | 0,92 | **-2,37** | 0,03 | -1,07 | 1,52 | **8,06** | 1,51 | **-2,10** | 0,21 |
| | | **J10** | 0.16 | -1.62 | **2,30** | -1.38 | **1,63** | **5,71** | **1,61** | **-2,72** | -1.37 |
| **Paraquat** | | **J1** | 0,55 | **4,09** | **7,10** | 0,00 | 0,00 | **2,61** | 0,00 | 1,99 | 0,00 |
| | | **J2** | **3,00** | **5,88** | **6,93** | **-3,05** | **5,12** | **6,04** | **4,77** | **2,56** | **-2,09** |
| | | **J3** | 0,54 | **2,53** | **6,63** | **-8,56** | **5,36** | 1,28 | -0,34 | 0,66 | -0,78 |
| **H₂O₂** | | **J2** | **2,44** | **4,75** | **2,53** | 3,02 | 0,83 | -1,92 | -0,20 | **1,91** | -0,58 |
| | | **J2** | **-2,09** | **3,92** | **4,27** | -0,64 | 0,79 | -1,17 | 1,35 | 0,91 | 0,82 |
| | | **J3** | 0,66 | **2,36** | **-2,25** | 0,41 | 0,38 | 0,70 | -0,82 | -0,87 | **-2,02** |
| | | **J3** | **4,31** | **3,38** | **6,36** | **4,37** | **2,00** | 3,18 | 1,32 | -0,06 | 0,17 |
| ***E. amylovora*** | | **6h** | **4,82** | **7,68** | **6,71** | 1,45 | **5,90** | **4,20** | **6,54** | 1,49 | ***-2,33*** |
| | | **6h** | 1,56 | 1,28 | **4,30** | **5,49** | **6,86** | **3,90** | **4,82** | 0,87 | 0,61 |
| | | **24h** | **5,24** | **3,14** | **5,19** | **1,98** | **5,04** | **3,90** | **9,64** | 0,44 | -0,82 |
| | | **24h** | **4,18** | -0,65 | **2,91** | -0,65 | **3,82** | **2,00** | **9,00** | -0,63 | ***-2,17*** |

**Tableau 5**

| Expression relative des gènes de défense dans des feuilles de pommiers soumis à différents traitements (SDN stress abiotiques, infection par E. amylovora) | | | | | | | | | | | |
|---|---|---|---|---|---|---|---|---|---|---|---|
| **Traitements** | | **Délai de prélèvement** | **CHS** | **DFR** | **PPO** | **HMGR** | **FPPS** | **Far** | **CSL** | **APOX** | **GST** |
| **Baba** | **-H₂O₂** | **J2** | 0,27 | 0,64 | -0,06 | 1,22 | -0,02 | 0,85 | -0,37 | 0,02 | 0,05 |
| | | **J2** | 0,29 | 093 | -1,07 | -0,67 | -0,41 | 0,28 | 0,38 | **2,14** | -0,34 |
| | | **J3** | 0,19 | -002 | 0,54 | **1,95** | 0,46 | 0,81 | 0,55 | -1,18 | 0,51 |
| | | **J3** | 0,31 | 072 | 1,24 | -0,52 | 0,56 | -1,03 | 1,34 | 0,11 | -0,14 |
| | **+H₂O₂** | **J2** | -0,50 | -0,28 | ***-2,03*** | **2,50** | 0,60 | 1,91 | 1,50 | 1,49 | 0,14 |
| | | **J2** | 1,18 | 1,17 | -1,09 | ***-1,67*** | -0,79 | -1,50 | 0,36 | **1,76** | -0,71 |
| | | **J3** | -0,47 | 0,42 | -0,67 | **1,98** | 1,46 | 1,25 | -0,34 | 1,20 | 0,88 |
| | | **J3** | 0,12 | 073 | **1,87** | -0,09 | -0,08 | -0,31 | 0,56 | **2,41** | -0,27 |
| **Bion** | **-H₂O₂** | **J2** | -0,36 | -0,09 | **1,78** | **3,27** | -1,44 | **4,65** | **4,37** | 1,49 | 0,64 |
| | | **J2** | 1,50 | **2,12** | **5,84** | **2,30** | 1,01 | **2,22** | **4,44** | 0,11 | **1,81** |
| | | **J3** | -1,36 | -0,49 | **1,65** | **4,30** | **1,59** | **3,82** | **4,71** | -0,34 | 1,24 |
| | | **J3** | -0,95 | -0,13 | **4,27** | 1,21 | 0.74 | **2,13** | **4,05** | 1,09 | 0,36 |
| | **+H₂O₂** | **J2** | -0,87 | 1,18 | -0,39 | **5,25** | **1,78** | **4,75** | **5,08** | **1,98** | 0,93 |
| | | **J2** | 1,57 | 1,44 | **2,22** | 1,04 | 0,26 | **3,69** | 2,97 | -0,06 | 0,68 |
| | | **J3** | -1,81 | 0,16 | **2,35** | **6,40** | **2,38** | **3,03** | **6,33** | -0,37 | **2,08** |
| | | **J3** | 0,03 | 1.38 | **3,84** | **2,82** | **1,75** | **2,30** | **3,97** | **2,74** | 1,14 |
| **Rhodofix** | **-H₂O₂** | **J2** | 1,79 | **2,28** | **1,63** | 0,93 | -0,45 | **1,98** | 2,99 | -1,09 | **1,76** |
| | | **J3** | 0,60 | 1,89 | **3,86** | 1,57 | 0,54 | **2,53** | 4,13 | -1 04 | **1,59** |
| | **+H₂O₂** | **J2** | 1,71 | 1,74 | **1,84** | 0,13 | -021 | **1,64** | **2,93** | 0,34 | **1,58** |
| | | **J3** | 0,50 | 1.68 | **3,01** | 1,52 | 0,55 | **1,71** | **4,55** | -0,20 | 1,18 |
| **Régalis** | **-H₂O₂** | **J2** | 0,16 | 0,63 | -0,16 | **1,72** | 0,11 | **2,12** | 0,35 | 1,25 | 1,09 |
| | | **J2** | 1,83 | **2,13** | 0,41 | -0,04 | -022 | 0,76 | **1,87** | 0,39 | 1 56 |
| | | **J3** | 022 | 0,67 | 0,57 | **2,90** | 1,42 | **1,79** | **1,90** | -0,73 | **2,28** |
| | | **J3** | 0,83 | **2,32** | 1,15 | 0,31 | 0,14 | -0,20 | **2,15** | -1,34 | 1,52 |
| | **+H₂O₂** | **J2** | 0,14 | 0,67 | ***-1,85*** | **3,50** | 1,10 | **2,43** | **1,63** | **1,63** | 1,48 |
| | | **J2** | **2,88** | **3,35** | **4,53** | -0,92 | 0,22 | 0,59 | **3,11** | 0,79 | **2,14** |
| | | **J3** | 0,85 | **2,57** | -0,43 | **3,49** | **2,97** | **2,62** | **2,12** | -069 | 1,77 |
| | | **J3** | 0,18 | 0,51 | 1,47 | -0,29 | 0,09 | -0,42 | 1,18 | ***-3,31*** | -0,46 |
| **PRM12** | **-H₂O₂** | **J2** | 0,08 | 0,23 | **1,59** | 0,23 | 0,46 | -0,18 | 0,59 | 0,53 | 0,48 |
| | | **J3** | **3,78** | **4,60** | **5,67** | 1,41 | 0,62 | 0,57 | **4,12** | 1,15 | **1,88** |
| | **+H₂O₂** | **J2** | **1,96** | **1,98** | -058 | -1,17 | 1,18 | -1,85 | **1,90** | 1,01 | 0,44 |
| | | **J3** | 0,23 | 1,10 | 0,63 | 0,09 | 0,30 | -1,62 | 1,42 | ***-1,91*** | 0,68 |
| **MeJA** | **-H₂O₂** | **J2** | **2,97** | **2,92** | 1,28 | 1,49 | 0,00 | **2,34** | **2,29** | **-3,00** | 1,00 |
| | | **J3** | 1,22 | 1,55 | 0,78 | 1,16 | 0,13 | 1,51 | **1,81** | 0,95 | -0,15 |
| | **+H₂O₂** | **J2** | **3,73** | **3,43** | 0,80 | -0,38 | -0,07 | 0,74 | **2,26** | **1,87** | 0,47 |
| | | **J3** | 1,47 | 1,36 | 1,23 | 0,60 | 0,43 | 0,39 | 1,46 | **2,11** | 0,87 |
| **Deshydratation** | | **J2** | 0,73 | 1,83 | -1,22 | 0,38 | -1,48 | **2,62** | -0,41 | -1,40 | ***-2,02*** |
| | | **J4** | **2,34** | **2,06** | -0,69 | 1,55 | ***-2,61*** | 0,92 | -0,86 | 0,00 | ***-3,05*** |
| | | **J7** | 1,45 | **2,08** | -0,74 | 1,05 | **-2,70** | **2,51** | -0,10 | 0,54 | -1,37 |
| | | **J10** | 1,08 | 1,87 | -0,14 | 1,28 | **-3,63** | **1,64** | 0,50 | **1,90** | -1,55 |
| **PEG** | | **J1** | 0,93 | 0,87 | 0,37 | -1,18 | -0,12 | **3,26** | 1,24 | 0,00 | 0,20 |
| | | **J2** | 0,05 | 1,12 | 0,35 | -0,87 | 0,29 | 0,96 | 1,18 | **2,38** | -0,01 |
| | | **J2** | -0,67 | 0,78 | **1,76** | **1,66** | ***-2,51*** | 1,06 | -0,15 | -1,37 | -0,73 |
| | | **J3** | 0,64 | **2,42** | -1,57 | 0,20 | -0,79 | 1,45 | **2,23** | 1,31 | -0,39 |
| | | **J4** | **1,96** | **1,80** | 1,17 | 1,33 | ***-3,95*** | -1,20 | 1,20 | -0,35 | -0,71 |
| | | **J7** | **1,74** | **1,83** | ***-2,02*** | -0,07 | ***-4,15*** | 0,18 | 0,75 | 0,68 | -0,79 |
| | | **J10** | 1,09 | 1,00 | 1,22 | 0,45 | ***-4,63*** | ***-2,01*** | **1,65** | -1,06 | -0.82 |
| **Paraquat** | | **J1** | -1,56 | -0,74 | 0,67 | **2,85** | 1,02 | **2,76** | **2,29** | 0,00 | **1,99** |
| | | **J2** | -1,49 | -1,14 | **6,27** | 1,20 | 0,15 | **2,30** | **4,42** | **2,01** | **1,83** |
| | | **J3** | **-2,04** | ***-2,09*** | **4,63** | -0.24 | ***-1,83*** | **1,85** | **5,43** | 1.48 | 0,80 |
| **H₂O₂** | | **J2** | 0,35 | 0,50 | **-1,99** | **2,70** | **1,94** | **2,37** | 0,53 | 0,41 | -0,81 |
| | | **J2** | 1,33 | 0,80 | -0,71 | -0,07 | -1,18 | -0,97 | -0,02 | 0,38 | -0,02 |
| | | **J3** | -0,51 | -0,73 | ***-1,75*** | **3,13** | **1,96** | 0,51 | -0,38 | -1,38 | 0,33 |
| | | **J3** | -0,09 | 0,57 | 1,24 | 1,37 | 0,85 | **2,02** | **1,64** | 0,77 | 0,40 |
| ***E. amylovora*** | | **6h** | ***-2,67*** | -1,16 | **6,27** | **4,71** | **5,12** | **6,13** | **4,94** | ***-2,33*** | **4,10** |
| | | **6h** | ***-4,28*** | ***-4,32*** | **6,18** | **2,86** | **2,64** | **4,46** | **3,90** | 0,61 | **2,53** |
| | | **24h** | -1,32 | -0,16 | **7,60** | **3,71** | **2,52** | **3,58** | **5,20** | -0,82 | **3,24** |
| | | **24h** | **-*2*,*85*** | ***-1,79*** | **8,04** | **2,68** | **1,78** | **5,68** | **3,70** | ***-2,17*** | **3,43** |

**Tableau 6**

| | | | | | | | | | | | | |
|---|---|---|---|---|---|---|---|---|---|---|---|---|
| **Baba** | **-H₂O₂** | **J2** | 0,44 | -0,45 | 0,19 | -0,04 | 0,41 | -1,51 | 0,37 | -0,80 | 0,10 | -0,09 |
| | | **J2** | 1,57 | 0,26 | 0,66 | -1,38 | 0,08 | -0,34 | -0,61 | 0,56 | -0,26 | **3,15** |
| | | **J3** | -1,16 | 0,71 | 0,00 | -0,73 | 0,16 | -1,33 | -0,69 | -0,29 | 0,00 | -0,65 |
| | | **J3** | 0,95 | -0,30 | 0,35 | **1,66** | ***-1,70*** | **2,55** | 0,09 | 0,29 | 0,43 | 0,41 |
| | **+H₂O₂** | **J2** | **2,20** | -0,87 | 0,75 | **2,04** | 1,13 | 0,34 | 0,10 | -0,44 | 1,14 | 0,60 |
| | | **J2** | **2,28** | -0,16 | 0,44 | ***-1,76*** | -0,69 | ***-1,71*** | -0,41 | -0,16 | **2,13** | **1,94** |
| | | **J3** | 1,41 | -0,15 | **2,10** | **2,96** | 0,97 | 0,27 | -0,62 | 1,52 | 0,64 | **1,69** |
| | | **J3** | **3,22** | -0,29 | **1,88** | **2,11** | -0,58 | -0,35 | 0,09 | 1,02 | **2,14** | 1,32 |
| **Bion** | **-H₂O₂** | **J2** | **3,76** | -0,36 | 1,31 | **2,63** | **3,21** | **1,67** | -0,75 | -1,13 | 1,28 | -0,64 |
| | | **J2** | **2,77** | 0,56 | -0,27 | -0,32 | **3,65** | **4,54** | -1,00 | -1,32 | **2,24** | **2,03** |
| | | **J3** | **3,56** | -0,30 | 0,49 | **1,61** | **3,26** | **2,22** | -1,18 | -0,57 | 1,36 | 0,05 |
| | | **J3** | **4,65** | 0,34 | 0,89 | 0,99 | **2,50** | **4,79** | 0,00 | -0,62 | 0,69 | 0,22 |
| | **+H₂O₂** | **J2** | **4,75** | -1,34 | 0,97 | **2,35** | **3,50** | **2,08** | ***-1,78*** | -1,39 | 1,60 | -0,09 |
| | | **J2** | **1,76** | 0,28 | 0,06 | 0,50 | **2,74** | **3,47** | 0,31 | -0,32 | **3,40** | **2,22** |
| | | **J3** | **1,72** | -1,08 | 0,94 | **1,89** | **4,09** | **1,77** | ***-1,71*** | -1,46 | 1,78 | -0,70 |
| | | **J3** | **5,67** | 0,31 | 1,27 | **3,73** | **4,15** | **7,33** | 0,16 | 0.87 | **3,01** | **1,67** |
| **Rhodofix** | **-H₂O₂** | **J2** | **3,68** | 009 | 0,44 | **1,68** | **2,49** | 0,38 | 0,39 | -1,04 | 0,98 | **2,03** |
| | | **J3** | **6,80** | -0,10 | 0,51 | **2,97** | **3,04** | **4,50** | -009 | -1,54 | **2,42** | -0,13 |
| | **+H₂O₂** | **J2** | **4,84** | 0,15 | 090 | -0,18 | **2,16** | **2,19** | 0,19 | -0,60 | **2,68** | 1,42 |
| | | **J3** | **5,52** | -026 | 1,11 | **2,66** | 1,12 | **4,09** | 0,40 | 0,20 | **4,22** | 0,89 |
| **Régalis** | **-H₂O₂** | **J2** | **2,92** | 063 | 0,39 | **3,00** | **2,14** | **1,84** | -059 | -0,14 | 0,93 | 1,52 |
| | | **J2** | **2,94** | 0,63 | -0,46 | 0,30 | 1,43 | 0,52 | -0,15 | -0,10 | **1,81** | 1,56 |
| | | **J3** | 1,50 | 0,82 | 0,19 | **1,63** | **3,43** | -0,99 | 0,20 | -0,66 | 0,66 | -0,44 |
| | | **J3** | **5,21** | 0,78 | -0,07 | **2,72** | 1,49 | 1,08 | 0,25 | -0,38 | 1,52 | -0,17 |
| | **+H₂O₂** | **J2** | **4,60** | -054 | 0,17 | **3,65** | 1,40 | -0,35 | -1,07 | -1,78 | 1,27 | 0,68 |
| | | **J2** | **2,65** | 0,68 | -0,72 | ***-1,86*** | 0,76 | **2,12** | 0,44 | -0,64 | **2,81** | 0,91 |
| | | **J3** | **2,10** | -0,31 | 0,58 | 0,44 | **2,50** | -0,96 | 0,10 | -0,27 | 0,85 | -1,09 |
| | | **J3** | 1,28 | -0,04 | 0,51 | 1,12 | 0,01 | -0,02 | 0,89 | ***-3,59*** | **3,40** | 0,66 |
| **PRM12** | **-H₂O₂** | **J2** | 0,56 | 0,67 | 0,78 | 0,29 | 0,34 | -1,70 | -0,39 | -0,34 | 0,93 | **2,56** |
| | | **J3** | **3,25** | 0,94 | 1,05 | -0,90 | -0,13 | **4,08** | 1,72 | 0,40 | **2,23** | 0,59 |
| | **+H₂O**₂ | **J2** | **3,59** | 0,36 | 0,30 | 1,47 | -0,50 | -0,94 | -0,24 | -0,22 | **3,56** | **2,74** |
| | | **J3** | 2,52 | 0,00 | 1,24 | **4,88** | -0,32 | 1,27 | 0,13 | 1,03 | **4,56** | **2,32** |
| **MeJA** | **-H₂O₂** | **J2** | 0,66 | 1,52 | 0,05 | 0,98 | **1,91** | 1,25 | **1,60** | 1,25 | **1,92** | **3,82** |
| | | **J3** | **3,86** | 0,01 | 1,14 | **3,87** | 1,22 | 0,85 | 1,45 | 0,63 | **1,96** | 1,22 |
| | **+H₂O₂** | **J2** | **2,84** | 0,70 | 0,48 | ***-2,02*** | -0,17 | -0,06 | 1,51 | 0,73 | **3,48** | **2,54** |
| | | **J3** | **4,29** | 0,35 | 1,48 | **2,16** | 0,98 | **3,46** | **1,99** | **1,61** | **2,28** | **1,80** |
| **Deshydratation** | | **J2** | -0,03 | -0,89 | 0,23 | ***-1,95*** | -0,07 | ***-1,80*** | 1,19 | 0,30 | 0,32 | **-1,87** |
| | | **J4** | 0,00 | -0,87 | 0,00 | 0,00 | 0,44 | ***-2,58*** | 1,07 | 0,00 | 0,71 | 0,00 |
| | | **J7** | 1,54 | -048 | -0,53 | **2,56** | 0,13 | 0,63 | **1,85** | -0,46 | 0,03 | -1,23 |
| | | **J10** | **2,44** | -043 | 0,12 | **4,46** | 0,23 | -0,39 | **2,06** | 0,04 | **1,74** | 091 |
| **PEG** | | **J1** | 0,00 | 0,59 | 0,00 | 0,00 | 0,24 | -1,23 | 1,51 | 0,00 | ***-1,96*** | 0,00 |
| | | **J2** | -1,33 | 0,72 | 0,24 | **2,18** | 0,46 | 0,70 | **2,48** | 1,06 | 0,69 | 0,02 |
| | | **J2** | -1,07 | -0,52 | 0,38 | -1,23 | -0,92 | -0,10 | **2,19** | -0,46 | -0,56 | ***-1,82*** |
| | | **J3** | ***-3,29*** | -031 | -089 | -098 | 0,96 | **3,41** | 0,41 | -1,00 | 0,51 | -0,36 |
| | | **J4** | 0,81 | 0,86 | 0,43 | **2,37** | ***-1,62*** | -1,24 | 1,36 | -1,43 | -0,33 | 0,63 |
| | | **J7** | 0,00 | 0,06 | 0,86 | **2,38** | -0,54 | **-*2,30*** | **1,96** | -019 | -0,36 | 0,18 |
| | | **J10** | ***-2,50*** | 0,58 | -0,08 | 0,56 | ***-2,40*** | -1,11 | 1,00 | ***-1,66*** | 1,16 | -0,07 |
| **Paraquat** | | **J1** | 0,00 | 0,04 | 0,00 | 0,00 | 0,83 | **4,01** | -0,58 | 0,00 | -1,33 | 0,00 |
| | | **J2** | **4,26** | 0,27 | 0,42 | **2,17** | **2,89** | **6,37** | -0,54 | -0,81 | 0,02 | 0,21 |
| | | **J3** | -0,53 | -0,51 | 0,01 | **-2,82** | **2,28** | **2,76** | ***-2,37*** | **-2,46** | 0,41 | -0,11 |
| **H₂O₂** | | **J2** | 1,07 | ***-1,64*** | **1,67** | -0,44 | 1,42 | -0,04 | 0,18 | 1,09 | 0,89 | 1,44 |
| | | **J2** | 1,06 | 0,29 | 0,27 | ***-1,62*** | -0,36 | -0,12 | -0,45 | -0,27 | 1,36 | 1,53 |
| | | **J3** | -0,26 | -0,72 | -0,10 | 0,71 | 0,19 | ***-2,26*** | 0,16 | -0,56 | 0,30 | -0,26 |
| | | **J3** | **1,88** | -0,54 | 0,93 | **1,65** | **2,08** | 4,02 | 0,42 | 0,43 | 0,84 | 0,41 |
| ***E. amylovora*** | | **6h** | **2,76** | **1,95** | 1,31 | -1,27 | 1,36 | **6,02** | ***-4,60*** | ***-3,83*** | **1,66** | **5,00** |
| | | **6h** | **3,64** | **1,63** | **2,92** | -0,60 | **2,03** | **8,29** | ***-4,63*** | -0,19 | 1,37 | **6,02** |
| | | **24h** | -0,79 | **1,81** | 0,09 | ***-3,72*** | 1,25 | **2,13** | ***-3,50*** | ***-2,34*** | 1,31 | **1,74** |
| | | **24h** | 1,12 | **2,46** | -0,42 | **-5,98** | 0,91 | **2,51** | **-2,57** | **-5,31** | 1,11 | 0,97 |

### Essai 2 - Validation de la pertinence des gènes sélectionnés à partir de 10 SDN

### Protocole :

Les produits testés sont listés dans le tableau 7. Il s'agit de 10 candidats SDN, ainsi qu'un antibiotique (la Plantomycine).

**Tableau 7**

| Produits testés | Matière active | Concentration testée* | Remarques |
|---|---|---|---|
| Aliette (BayerCropScience) | Fosétyl-Al | 6 g/l | Fongicide, SDN |
| Amid-Thin (Nufarm) | 1-Naphtalèneacétamide | 0,6 g/l | Eclaircisseur analogue auxine |
| Bion (Syngenta) | Acibenzolar-S-methyl | 0,4 g/l | Analogue SA, SDN |
| lodus (Goëmar) | Laminarine | 7,5 ml/l | SDN |
| Maxcel (Valent Biosciences) | 6-benzyl adénine | 7,5 ml/l | Eclaircisseur analogue cytokinine |
| PRM12 (BaverCropScience) | Ethéphon | 3 ml/l | Eclaircisseur précurseur d'éthylène |
| Régalis (BASF) | Prohexadione-Ca | 2,5 g/l | Régulateur de croissance analogue acide 2-oxoglutarique |
| Rhodofix (Nufarm) | ∝-Naphtyl acide acétique | 1,5 g/l | Eclaircisseur analogue auxine |
| Stifénia (S.O.F.T.) | Extrait de graines de fénugrec | 10 g/l | SDN |
| V-Plaask (Plaaskem) | Dérivés SA + fertilisant | 10 ml/l | Fertilisant et SDN |
| Plantomycine | Streptomycine | 0,56 g/l | Antibiotique |

| | | | |
|---|---|---|---|
| * Concentration en produit formulé | | | |

### • Pour l'analyse des défenses

Les tests sont conduits en conditions semi-contrôlées en serre (23°C jour/17°C nuit, éclairage naturel, complément néon), sur semis de Pommier (issus de pépins de la variété Golden Delicious), en croissance active, au stade 3 à 6 feuilles.

Les plantes sont pulvérisées jusqu'à refus avec les produits candidats ou avec de l'eau (témoin négatif) à J0, au pulvérisateur à air comprimé.

La moitié de ces plantes est pulvérisée avec du peroxyde d'hydrogène (dilution au 200ème d'une solution à 30%) au pulvérisateur manuel à J1 (J0 + 1 jour) pour simuler une attaque *d'Erwinia amylovora,* agent du feu bactérien (ceci permet de repérer un effet potentialisateur des défenses *versus* stimulateur direct, pour les produits testés).

Des prélèvements de disques foliaires (10 disques de 0,6 cm de diamètre sur 5 feuilles F1 poolées / prélèvement) sont effectués à J0 (semis non traités = calibrateur), puis à J1 (avant traitement au peroxyde d'hydrogène), et enfin à jour J2 (J0 + 2 jour) et J3 (J0 + 3 jour) sur les lots traités, ou non, au peroxyde d'hydrogène.

Les disques foliaires sont immédiatement congelés dans l'azote liquide et conservés à-80°C jusqu'à extraction des ARN. Ces derniers sont rétrotranscrits en ADNc et les niveaux d'expression de gènes de défense sont suivis par qPCR (SYBR Green) à l'aide de l'outil selon l'invention.

Trois essais biologiques indépendants ont été réalisés pour l'ensemble des produits.

Les niveaux d'expression relative sont calculés avec la méthode du ΔΔCt : il s'agit d'expressions relatives par rapport au calibrateur (J0), ou par rapport aux témoins eau à chaque temps de prélèvement, normalisées par la moyenne géométrique des expressions relatives de 3 gènes de référence (TuA, actin, GAPDH). Ces expressions relatives sont transformées en log₂ pour donner le même poids aux inductions et aux répressions de gènes.

### • Pour l'analyse du pouvoir de protection

Ces mêmes produits ont été testés pour leur pouvoir de protection vis-à-vis du feu bactérien de la manière suivante.

Les essais ont été réalisés sur le même type de matériel végétal et dans des conditions de serre identiques que ci-dessus.

Les produits sont pulvérisés avec le même pulvérisateur que ci-dessus, 4 jours ou 4 heures avant inoculation artificielle par *E. amylovora* (parcelles de 3x10 semis par produit et par délai traitement-inoculation).

Le jour de l'inoculation, la plus jeune feuille développée (F1) de chaque semis (qu'il ait été traité 4 jours avant par les produits, ou qu'il s'apprête à recevoir un traitement dit « -4h ») est blessée à l'aide d'un scalpel (2 coupures parallèles au 1/3 inférieur de la feuille, et perpendiculaires à la nervure principale).

Les traitements « -4h » sont alors réalisés.

Quatre heures après traitement, une suspension bactérienne de la souche CFBP1430 d'*E. amylovora,* préparée dans de l'eau stérile à 10⁸ cfu/mL, est inoculée par pulvérisation (au pulvérisateur à pression) sur l'ensemble des plantes traitées 4 jours ou 4 heures avant, et blessées 4 heures avant.

La notation des symptômes, c'est-à-dire présence d'une nécrose ayant évolué sur tige à partir de la feuille blessée, est réalisée 3 semaines après inoculation. Le pourcentage de plantes infectées est calculé dans chaque parcelle de 10 plantes et rapporté à la moyenne des pourcentages d'infection obtenus pour les 3 parcelles témoins « eau » du même essai (infection relative). Les distributions des pourcentages d'infection relative obtenus (intra et interexpériences) sont représentées par des boîtes à moustaches de Tuckey : la médiane est représentée par le large trait noir au centre de la boîte et la moyenne par le carré ; les encoches représentent les intervalles de confiance de la médiane (P=0.05).

Au moins deux essais indépendants ont été réalisés sur l'ensemble des produits.

### Résultats :

### • Analyse des défenses

Les résultats d'expression moyennée des 28 gènes cibles sont détaillés dans les tableaux 8 et 9 ci-après ; ces résultats sont répartis dans plusieurs tableaux, uniquement dans un souci de présentation.

Les résultats d'expression des gènes issus de l'outil selon l'invention (Tableaux 8 et 9) révèlent 4 produits (Bion, lodus, Régalis, Rhodofix) capables d'activer des défenses de manière répétable sur les 3 répétitions biologiques indépendantes.

Les inductions concernent un jeu assez proche de gènes dans les feuilles de Pommier avec quelques différences cependant.
a) Bion est le produit qui active le plus de gènes, de manière la plus intense (certains gènes sont régulièrement exprimés 2¹⁰ fois plus dans les tissus traités au Bion que dans les tissus témoins) et de façon très reproductible. Les gènes de la voie SA sont particulièrement induits, et cette forte induction est corrélée avec une répression des gènes de la voie JA, ce qui est cohérent avec l'antagonisme voie SA/voie JA décrit dans la littérature.
b) lodus, Régalis et Rhodofix n'activent, de manière significative, qu'une partie des gènes induits par le Bion, ou de manière moins constante. Par exemple, PR1, PR5 ou PPO sont 3 gènes fortement activés par le Bion et, pas ou peu par ces 3 produits.
c) Régalis montre un effet potentialisateur car les inductions de gènes se manifestent surtout après le traitement au peroxyde d'hydrogène (particulièrement visible pour le gène PECT).
d) Les 3 autres produits qui revendiquent une action SDN, à savoir Aliette, Stifenia et VPlaask, ainsi que les 3 autres éclaircisseurs, à savoir Amid Thin, Maxcel et PRM12, ou encore le produit biocide Plantomycine, ne provoquent pas d'induction reproductible et significative.

### • Analyse de la protection

Parmi les 11 produits testés, seuls trois manifestent une efficacité de protection vis-à-vis du feu bactérien lorsqu'ils sont pulvérisés 4 jours avant inoculation (conditions pour révéler un effet stimulateur) : il s'agit du Bion, de Régalis et de Rhodofix qui réduisent significativement les pourcentages d'infection des semis (Figure 1A).

Les produits qui n'induisent aucun des gènes de défense présents sur l'outil, ainsi que lodus qui est capable d'induire quelques défenses, n'ont pas d'efficacité de protection lorsqu'ils sont appliqués 4 jours avant inoculation.

Pulvérisés quelques heures avant inoculation, Bion conserve une efficacité très significative ; et Régalis présente toujours une efficacité qui reste également significative, quoique moins élevée (Figure 1B).

Ces efficacités n'atteignent cependant pas celle obtenue avec la Plantomycine, biocide vis-à-vis d'*E*. *amylovora.*

Aucun autre produit ne diminue, de manière significative et reproductible, les pourcentages d'infection des semis.

### • Conclusion

Au vu de ces résultats, l'outil selon l'invention permet d'éliminer la majorité des produits candidats SDN qui s'avèrent sans potentiel de protection de semis de Pommier vis-à-vis du feu bactérien, c'est-à-dire Aliette, Amid Thin, Maxcel, PRM12, Stifenia, V-Plaask.

Parmi les 4 produits retenus par le crible (Bion, lodus, Régalis, Rhodofix), seul lodus ne montre finalement pas d'efficacité de protection sur semis vis-à-vis du feu bactérien.

L'outil selon l'invention est donc utile pour sélectionner les produits ayant un potentiel de protection (tout au moins vis-à-vis du feu bactérien), et qui méritent d'être retenus pour expérimentation.

**Tableau 8**

| **Traitement** | **Délai** | **PR1** | **PR2** | **PR4** | **PR5** | **PR8** | **PR14** | **PR15** | **PAL** | **ANS** | **CHS** | **DFR** | **PPO** | **HMGR** | **FPPS** |
|---|---|---|---|---|---|---|---|---|---|---|---|---|---|---|---|
| **Aliette** | J1 | -0,97 | 0,16 | 0,31 | 0,50 | -0,28 | 0,10 | 2,13 | 0,37 | -0,09 | -0,01 | -1,01 | 2,64 | 0,18 | -0,40 |
| | J2 | -2,12 | -2,17 | 0,92 | -2,16 | 0,87 | -0,20 | 0,46 | -0,25 | 0,21 | -0,03 | 0,22 | -1,07 | 0,63 | 0,12 |
| | J3 | -0,55 | 0,49 | 0,48 | 0,26 | -0,02 | -1,27 | -0,41 | -1,17 | -1,32 | -1,55 | -0,92 | 1,00 | 0,41 | -0,18 |
| | J2 + H₂O₂ | -0,68 | 1,05 | 3,91 | -0,94 | 3,17 | 0,81 | 0,00 | -0,95 | -0,75 | -0,70 | -0,43 | -0,09 | 0,54 | 0,00 |
| | J3 + H₂O₂ | 0,04 | 3,21 | 0,70 | 1,30 | 0,24 | -0,01 | 0,53 | -1,26 | -1,58 | -1,36 | -1,06 | 1,64 | 0,44 | -0,10 |
| **Amid Thin** | J1 | 0,81 | 0,80 | 0,86 | -0,57 | 0,39 | 1,50 | -0,39 | 0,09 | 0,23 | 0,23 | 0,45 | 1,05 | 0,52 | -0,39 |
| | J2 | -0,13 | -0,73 | 0,65 | -0,81 | 0,40 | -0,32 | 0,52 | 0,06 | 0,74 | 0,54 | 0,53 | -0,45 | -0,05 | 0,02 |
| | J3 | -0,58 | 0,44 | -0,37 | 0,21 | -0,15 | 0,33 | 0,04 | -0,23 | -0,05 | -0,56 | -0,23 | 0,95 | 0,50 | 0,59 |
| | J2 + H₂O₂ | 0,37 | 2,33 | 3,22 | -0,07 | 2,39 | 1,78 | 1,66 | -0,16 | -0,39 | -0,19 | -0,08 | 0,38 | -0,17 | 0,45 |
| | J3 + H₂O₂ | 0,16 | 2,30 | 1,21 | 0,49 | 1,06 | 1,04 | 0,01 | -1,17 | -1,52 | -1,73 | -0,64 | 0,10 | 0,42 | 0,43 |
| **Bion** | J1 | 1,74 | 6,30 | 5,80 | 3,73 | 2,51 | 1,06 | -0,62 | -0,17 | -1,79 | -1,64 | -1,51 | 1,19 | 2,18 | 0,93 |
| | J2 | 3,47 | 8,78 | 9,62 | 4,12 | 4,82 | 3,99 | -0,89 | 2,22 | 0,97 | 0,78 | 1,10 | 3,65 | 3,31 | 1,74 |
| | J3 | 6,18 | 11,20 | 9,96 | 4,70 | 5,29 | 7,61 | 4,24 | 1,11 | 0,09 | 0,26 | 0,30 | 5,55 | 1,64 | 1,02 |
| | J2 + H₂O₂ | 3,58 | 9,23 | 9,75 | 4,07 | 5,72 | 4,19 | 1,06 | 2,06 | -0,17 | -0,16 | 0,04 | 4,74 | 3,84 | 1,42 |
| | J3 + H₂O₂ | 6,07 | 11,05 | 8,99 | 4,46 | 4,97 | 6,87 | 2,40 | 0,78 | 0,53 | 0,04 | 0,20 | 4,32 | 2,23 | 0,75 |
| | | | | | | | | | | | | | | | |
| **Iodus** | J1 | 1,67 | 6,04 | 6,91 | 1,24 | 3,37 | 4,09 | 1,90 | 0,50 | -1,37 | -1,03 | -0,62 | 2,55 | 2,20 | 1,55 |
| | J2 | 4,53 | 4,74 | 6,88 | 3,15 | 5,46 | 6,92 | 1,75 | 1,13 | 1,04 | 0,14 | 1,61 | 1,81 | 2,76 | -0,02 |
| | J3 | 3,30 | 3,22 | 4,64 | 1,56 | 2,43 | 1,26 | 0,08 | -2,39 | 0,07 | -0,42 | -0,03 | 2,48 | 0,48 | -0,25 |
| | J2 + H₂O₂ | 3,93 | 4,28 | 6,98 | 3,35 | 5,28 | 6,86 | 1,51 | 0,11 | 0,86 | -0,63 | 1,10 | 0,89 | 2,71 | 0,05 |
| | J3 + H₂O₂ | 3,54 | 3,31 | 4,74 | 2,02 | 2,65 | 1,76 | 0,44 | 0,69 | 0,53 | -0,20 | 0,54 | 2,39 | 0,75 | -0,19 |
| **Maxcel** | J1 | 0,45 | -0,87 | 0,99 | -0,20 | 0,10 | 0,77 | -0,01 | -0,01 | 0,20 | -0,17 | 0,59 | 0,26 | 0,11 | -0,59 |
| | J2 | -0,20 | -1,11 | 0,18 | -0,21 | 0,34 | 1,03 | 0,26 | -0,31 | 0,65 | 0,07 | 0,55 | -1,06 | 0,17 | -0,06 |
| | J3 | 0,75 | -0,27 | -0,01 | 0,08 | -0,71 | -2,12 | 1,00 | -0,47 | -1,14 | -0,75 | -0,21 | 0,61 | 0,04 | 0,43 |
| | J2 + H₂O₂ | 0,77 | 1,23 | 2,92 | -0,46 | 2,81 | 0,88 | 0,32 | -0,29 | 0,44 | -0,50 | 0,59 | -1,08 | 0,05 | -0,03 |
| | J3 + H₂O₂ | 0,64 | 2,02 | 0,30 | 0,89 | 1,15 | -0,49 | -0,14 | -0,42 | -0,85 | -0,77 | -0,10 | 0,09 | 0,67 | 0,47 |
| **PRM12** | J1 | -0,79 | 0,01 | 2,58 | -0,61 | 0,92 | 1,82 | -0,97 | 1,06 | 0,92 | 0,37 | 1,13 | 0,62 | 0,98 | -0,70 |
| | J2 | 0,00 | -0,31 | 0,66 | -1,59 | 0,74 | -1,04 | -0,64 | -0,44 | 0,32 | -0,04 | 0,00 | -0,22 | 0,36 | -0,10 |
| | J3 | -0,49 | 0,10 | 0,11 | 0,20 | -0,76 | -1,25 | 1,16 | -0,60 | -0,91 | -0,80 | -0,52 | 1,15 | -0,09 | -0,09 |
| | J2 + H₂O₂ | 1,17 | 2,72 | 2,68 | -0,41 | 2,77 | 1,47 | 1,75 | -0,01 | 0,86 | 0,33 | 0,92 | 1,03 | 0,49 | 0,33 |
| | J3 + H₂O₂ | -0,35 | 2,21 | 0,64 | 0,49 | 0,68 | -1,21 | 0,17 | -1,18 | -1,36 | -1,48 | -0,70 | 0,15 | 0,19 | -0,14 |
| **Regalis** | J1 | -0,24 | 0,79 | 3,93 | -0,05 | 1,52 | 1,62 | 1,27 | 0,31 | 1,11 | 0,78 | 0,47 | 3,48 | 0,66 | 0,16 |
| | J2 | 0,28 | 2,27 | 4,29 | 1,65 | 2,45 | 1,75 | 1,43 | 0,01 | 1,77 | 0,82 | 1,02 | 0,32 | 0,20 | 0,41 |
| | J3 | 0,86 | 4,69 | 3,25 | 1,13 | 2,96 | 1,70 | -0,04 | 0,63 | 1,54 | 0,97 | 1,04 | 0,38 | 0,64 | 0,17 |
| | J2 + H₂O₂ | 1,19 | 5,46 | 7,22 | 2,55 | 4,21 | 4,20 | 2,33 | 0,69 | 1,43 | 0,84 | 1,23 | 2,70 | 1,44 | 0,47 |
| | J3 + H₂O₂ | 2,87 | 6,25 | 6,17 | 2,94 | 4,20 | 3,20 | 1,13 | 1,29 | 1,52 | 1,25 | 1,78 | 2,35 | 1,08 | 0,45 |
| **Rhodofix** | J1 | 1,46 | 4,99 | 6,65 | 0,83 | 3,69 | 6,53 | 0,88 | 0,39 | 0,00 | 0,15 | 0,64 | 2,77 | 1,89 | 0,76 |
| | J2 | 1,27 | 6,82 | 7,40 | 3,26 | 4,33 | 4,18 | -0,26 | 1,21 | 1,44 | 1,51 | 1,03 | 0,35 | 1,70 | 0,56 |
| | J3 | 0,34 | 4,00 | 2,56 | 1,51 | 0,57 | 0,42 | 0,68 | 0,09 | -0,17 | -0,11 | 0,05 | -0,12 | -0,23 | -0,08 |
| | J2 + H₂O₂ | 0,37 | 6,46 | 6,92 | 2,61 | 4,04 | 4,83 | -0,44 | -0,01 | -0,10 | 0,06 | 0,01 | 0,48 | 1,25 | 0,36 |
| | J3 + H₂O₂ | 2,19 | 4,95 | 4,82 | 2,02 | 2,71 | 1,34 | 0,06 | -0,01 | -0,24 | -0,45 | 0,82 | 0,45 | 1,45 | 0,62 |
| **Stifenia** | J1 | -0,80 | 0,14 | -0,99 | -0,46 | -0,54 | -0,75 | -1,84 | -1,12 | -1,13 | -1,00 | -1,59 | -0,82 | -0,40 | -0,25 |
| | J2 | 0,72 | 1,28 | 1,56 | 3,09 | 2,33 | 1,12 | 1,19 | 0,31 | 0,88 | 0,68 | 0,63 | -0,08 | 0,05 | 0,12 |
| | J3 | 0,61 | 0,24 | 0,47 | 0,11 | 1,11 | 1,84 | 1,19 | 0,39 | 0,30 | 0,64 | 1,14 | -0,50 | 0,57 | 1,29 |
| | J2 + H₂O₂ | 1,32 | 1,72 | 4,01 | 2,55 | 5,59 | 2,16 | 0,97 | 0,53 | 0,71 | 0,03 | 0,58 | 0,15 | 0,99 | -0,13 |
| | J3 + H₂O₂ | 1,31 | -0,02 | 0,00 | -0,07 | 1,19 | 0,11 | -0,26 | -0,70 | -0,13 | -0,39 | 0,23 | 0,81 | 0,58 | 0,22 |
| **Plantomyc.** | J1 | 1,23 | 1,05 | 1,51 | -0,19 | 0,21 | 0,35 | 0,43 | -0,48 | -1,42 | -0,99 | -1,04 | 1,91 | 0,02 | 0,01 |
| | J2 | -1,62 | -1,16 | -0,33 | -0,45 | 0,03 | 0,18 | -0,94 | -1,14 | -0,79 | -1,28 | -1,50 | -0,61 | 0,16 | -0,50 |
| | J3 | 1,39 | 2,59 | 1,35 | 1,12 | 0,27 | 0,14 | 1,93 | -0,17 | -1,00 | -0,25 | -0,96 | 1,13 | -0,27 | 0,07 |
| | J2 + H₂O₂ | 1,54 | 2,96 | 4,03 | 0,07 | 3,00 | 1,80 | 0,37 | -0,28 | -0,07 | 0,07 | 0,04 | 1,38 | 0,89 | 0,09 |
| | J3 + H₂O₂ | 2,03 | 3,31 | 2,19 | 0,60 | 1,14 | -0,06 | 0,58 | -0,88 | -0,63 | -0,44 | -0,68 | 1,75 | 0,30 | -0,34 |
| **Vplaask** | J1 | -0,66 | -0,20 | 0,25 | 1,60 | 0,17 | -0,05 | 0,83 | 0,24 | 0,52 | 0,35 | -0,20 | 0,88 | -1,60 | -0,43 |
| | J2 | 0,76 | -0,15 | -0,98 | 1,53 | 2,50 | 2,37 | 1,02 | -3,41 | -0,25 | -1,44 | -0,33 | -0,78 | 0,77 | -2,23 |
| | J3 | 0,40 | -0,45 | 0,38 | 0,57 | 0,16 | -1,44 | 0,23 | -0,20 | -0,61 | -1,29 | -0,78 | 0,35 | 0,28 | 0,13 |
| | J2 + H₂O₂ | 0,37 | 1,11 | 1,39 | 1,53 | 2,83 | 2,22 | 2,54 | -1,33 | 0,27 | -1,03 | 0,64 | 0,79 | 0,84 | 0,47 |
| | J3 + H₂O₂ | 1,43 | 0,48 | 0,24 | 1,25 | 0,30 | -1,88 | 1,05 | -0,79 | -1,39 | -1,61 | -1,00 | 1,26 | 0,34 | 0,57 |
| **H₂0₂** | J2 + H₂O₂ | 0,85 | 3,69 | 3,89 | 0,91 | 2,12 | 1,32 | -0,26 | -0,56 | -0,63 | -0,80 | -0,65 | 1,20 | 1,05 | 0,06 |
| | J3 + H₂O₂ | 1,41 | 4,05 | 2,59 | 1,73 | 1,73 | 3,29 | 1,57 | -0,57 | -0,49 | -0,75 | -0,36 | 0,71 | 0,14 | 0,08 |

**Tableau 9**

| **Traitement** | **Délai** | **FAR** | **CSL** | **APOX** | **GST** | **POX** | **CAD** | **CalS** | **PECT** | **EDS1** | **WRKY** | **LOX2** | **JAR** | **ACCO** | **EIN3** |
|---|---|---|---|---|---|---|---|---|---|---|---|---|---|---|---|
| **Aliette** | J1 | -0,90 | -0,16 | -0,29 | -0,23 | 0,99 | 0,02 | -0,22 | -0,32 | 0,09 | -0,04 | 0,39 | -0,40 | -0,23 | -0,35 |
| | J2 | -1,62 | -0,21 | 0,09 | -0,06 | -0,91 | -0,12 | 0,06 | -0,89 | 0,44 | 1,18 | 0,23 | -0,25 | -0,08 | 0,07 |
| | J3 | 0,78 | -0,19 | -0,29 | 0,16 | -0,23 | -0,05 | -0,09 | -0,24 | -0,36 | -1,39 | 0,05 | -0,08 | -0,13 | 0,54 |
| | J2 + H₂O₂ | -1,25 | 0,23 | 0,12 | 0,73 | 2,39 | 0,06 | 0,24 | 0,61 | 1,20 | 0,51 | 0,21 | -0,45 | 0,44 | 0,45 |
| | J3 + H₂O₂ | 1,28 | 0,24 | 0,12 | 0,42 | 2,08 | 0,08 | 0,25 | 0,24 | -0,21 | -0,30 | 0,29 | 0,15 | -0,09 | 0,69 |
| **Amid Thin** | J1 | 0,06 | 0,17 | -0,40 | 0,51 | 0,91 | 0,31 | -0,14 | 1,21 | 0,18 | 0,02 | 0,45 | 0,11 | 0,57 | 0,71 |
| | J2 | -0,54 | 0,16 | -0,42 | 0,46 | 0,01 | 0,37 | 0,09 | 0,11 | 1,03 | -0,33 | 0,38 | -0,09 | 0,44 | 0,59 |
| | J3 | -0,30 | -0,14 | -0,14 | 0,54 | 0,37 | 0,05 | 0,18 | 0,37 | 0,32 | 0,38 | 0,19 | 0,13 | 0,36 | 0,13 |
| | J2 + H₂O₂ | 0,20 | 0,51 | 0,13 | 0,88 | 1,16 | 0,41 | 0,71 | 1,62 | 1,74 | 1,53 | -0,09 | 0,42 | 0,89 | 0,35 |
| | J3 + H₂O₂ | 0,96 | -0,39 | -0,47 | 0,44 | 0,77 | -0,15 | 0,33 | 0,03 | 0,27 | -0,45 | -0,40 | -0,28 | 0,04 | -0,27 |
| **Bion** | J1 | 4,20 | 1,74 | -0,06 | 2,35 | 1,66 | 0,51 | -0,04 | 0,17 | 3,21 | 2,30 | -0,67 | -1,10 | 0,89 | 0,52 |
| | J2 | 3,26 | 5,50 | 0,42 | 2,08 | 3,27 | 0,14 | -0,17 | 1,03 | 4,14 | 5,54 | -0,36 | -0,82 | 1,26 | 1,02 |
| | J3 | 2,54 | 7,15 | 0,62 | 1,86 | 3,47 | 0,38 | 0,49 | 2,26 | 4,09 | 4,99 | -1,25 | -0,84 | 1,11 | 0,30 |
| | J2 + H₂O₂ | 2,36 | 6,70 | 0,49 | 1,99 | 3,67 | 0,25 | -0,18 | 1,10 | 4,65 | 5,61 | -1,52 | -2,01 | 1,12 | 0,64 |
| | J3 + H₂O₂ | 2,02 | 6,54 | 0,60 | 1,46 | 4,19 | -0,02 | 0,16 | 2,07 | 4,22 | 5,04 | -1,34 | -1,06 | 0,98 | -0,06 |
| **Iodus** | J1 | 2,58 | 3,78 | 0,13 | 1,75 | 1,19 | -0,43 | -0,15 | 0,16 | 0,74 | 1,90 | -0,34 | -0,05 | 0,91 | 0,03 |
| | J2 | 2,45 | 4,08 | 0,36 | 2,07 | 0,94 | 0,65 | 0,50 | 4,70 | 3,14 | 2,68 | 0,68 | 0,49 | 2,52 | 1,14 |
| | J3 | 2,23 | 2,33 | 0,66 | 0,98 | 2,57 | 0,84 | 0,78 | 1,97 | 2,63 | 2,16 | 0,33 | 0,24 | 2,94 | 0,82 |
| | J2 + H₂O₂ | 3,05 | 3,40 | -0,08 | 1,90 | 1,46 | -0,19 | 0,71 | 4,55 | 1,33 | 2,11 | 0,34 | 1,30 | 2,10 | 0,87 |
| | J3 + H₂O₂ | 1,32 | 3,15 | -0,32 | 1,91 | 2,73 | 0,12 | 0,42 | 1,21 | 2,05 | 2,33 | -0,25 | 0,37 | 1,23 | 0,11 |
| **Maxcel** | J1 | -0,73 | -0,17 | -0,17 | -0,23 | -0,07 | -0,46 | -0,39 | -0,23 | 0,11 | -0,51 | -0,20 | -0,42 | -0,19 | 0,10 |
| | J2 | -0,83 | 0,66 | -0,60 | 0,56 | -0,39 | 0,45 | 0,45 | 0,14 | 1,12 | -0,11 | 0,29 | 0,00 | 0,52 | 0,93 |
| | J3 | 0,15 | -0,35 | 0,07 | -0,04 | 0,01 | -0,01 | 0,50 | -0,19 | 0,22 | -0,64 | 0,02 | 0,25 | -0,02 | -0,23 |
| | J2 + H₂O₂ | -0,26 | 1,01 | -0,07 | 0,79 | 1,34 | 0,51 | 0,11 | 0,12 | 2,25 | 1,34 | -0,01 | -0,18 | 0,54 | 0,95 |
| | J3 + H₂O₂ | 0,18 | 0,17 | 0,13 | 0,52 | 1,45 | -0,13 | 0,36 | 0,45 | 1,17 | 0,50 | 0,21 | 0,21 | 0,44 | 0,52 |
| **PRM12** | J1 | 0,48 | -0,03' | -0,70 | 0,22 | -0,12 | 0,24 | -0,23 | 0,80 | 0,50 | -0,21 | 0,06 | -0,90 | 0,40 | 1,61 |
| | J2 | -1,41 | 0,27 | -0,66 | 0,47 | -0,19 | 0,39 | 0,59 | 0,78 | 0,94 | -0,36 | 0,02 | -0,50 | 0,63 | 1,41 |
| | J3 | -0,43 | 0,02 | -0,06 | 0,55 | 0,34 | -0,03 | 0,70 | 0,73 | -0,09 | 0,07 | -0,38 | -0,10 | 0,35 | -0,05 |
| | J2 + H₂O₂ | 0,42 | 1,71 | 0,39 | 0,86 | 1,42 | 0,39 | 0,65 | 2,22 | 0,81 | 1,83 | -0,03 | 0,10 | 1,11 | 1,72 |
| | J3 + H₂O₂ | -0,40 | -0,28 | -0,27 | 0,27 | 0,73 | -0,24 | 0,15 | 0,19 | 0,08 | 0,25 | 0,13 | 0,03 | 0,18 | 0,03 |

| **Traitement** | **Délai** | **FAR** | **CSL** | **APO X** | **GST** | **POX** | **CAD** | **CalS** | **PECT** | **EDS1** | **WRK Y** | **LOX2 JAR** | | **ACC O** | **EIN3** |
|---|---|---|---|---|---|---|---|---|---|---|---|---|---|---|---|
| **Regalis** | J1 | 1,83 | 0,89 | -0,07 | 1,26 | 0,86 | 0,40 | -0,01 | -0,44 | 0,77 | 2,10 | -0,07 | -0,40 | 0,31 | 0,31 |
| | J2 | 2,15 | 1,87 | -0,06 | 1,79 | 1,80 | 0,68 | 0,14 | 0,73 | 1,60 | 0,42 | 0,03 | -0,14 | 0,66 | 0,06 |
| | J3 | 2,06 | 1,16 | 0,24 | 1,45 | 2,91 | 0,43 | 0,10 | 0,73 | 1,99 | 1,66 | 0,13 | -0,03 | 0,53 | -0,10 |
| | J2 + H₂O₂ | 3,72 | 3,19 | -0,08 | 2,61 | 3,32 | 0,41 | 0,21 | 2,30 | 2,78 | 2,14 | -0,08 | -0,13 | 1,25 | 0,48 |
| | J3 + H₂O₂ | 2,81 | 2,81 | 0,41 | 2,12 | 4,25 | 0,75 | 0,41 | 2,58 | 2,46 | 3,33 | 0,24 | 0,02 | 1,21 | 0,62 |
| **Rhodofix** | J1 | 3,09 | 0,90 | -0,27 | 2,34 | -0,11 | -0,01 | 0,81 | 2,72 | 2,76 | 1,47 | 0,08 | -0,69 | 2,01 | 0,91 |
| | J2 | 3,18 | 2,97 | -0,57 | 2,02 | 0,86 | 0,73 | 1,24 | 3,50 | 4,17 | 0,84 | 0,86 | -0,31 | 1,79 | 1,59 |
| | J3 | 1,51 | 1,21 | 0,08 | 1,01 | 1,74 | 0,50 | 1,07 | 3,01 | 0,91 | -0,58 | 0,62 | 0,39 | 0,79 | 0,81 |
| | J2 + H₂O₂ | 3,16 | 2,41 | -0,55 | 1,77 | 1,14 | 0,21 | 0,73 | 2,89 | 3,61 | 0,45 | 0,09 | -0,87 | 1,32 | 0,83 |
| | J3 + H₂O₂ | 1,32 | 2,97 | -0,08 | 1,11 | 0,80 | 0,18 | 0,67 | 2,21 | 2,30 | 0,87 | 0,08 | -0,57 | 0,98 | -0,17 |
| **Stifenia** | J1 | 0,15 | -0,85 | -0,64 | -0,32 | -0,44 | -0,42 | -0,71 | -1,08 | -0,32 | 0,33 | -0,48 | -0,07 | -0,12 | -0,42 |
| | J2 | 0,62 | 0,95 | 0,20 | 0,78 | 0,18 | 0,15 | 0,48 | 1,82 | 1,20 | 0,69 | 0,66 | 0,54 | 1,91 | 0,69 |
| | J3 | 0,01 | 0,71 | 0,49 | 7,33 | 0,76 | 0,05 | 0,68 | 2,06 | 2,83 | 0,43 | -0,16 | 1,47 | 5,54 | 0,88 |
| | J2 + H₂O₂ | 1,45 | 1,07 | -0,71 | 0,84 | 0,65 | 0,48 | 0,31 | 3,30 | 1,94 | 1,14 | -0,03 | 0,51 | -0,33 | 1,06 |
| | J3 + H₂O₂ | 0,48 | -0,29 | 0,08 | 7,24 | 0,89 | 0,05 | 0,40 | 1,93 | 2,78 | 0,65 | -0,58 | 1,31 | 4,23 | 0,84 |
| **Plantomyc.** | J1 | -0,03 | -0,59 | -0,02 | -0,03 | 1,37 | -0,09 | -0,08 | 0,21 | 0,14 | 1,36 | 0,12 | 0,27 | 0,29 | 0,00 |
| | J2 | -1,10 | -0,96 | -0,68 | -0,06 | -0,72 | -0,15 | -0,07 | -0,57 | -0,02 | -1,10 | -0,24 | -0,62 | -0,15 | 0,20 |
| | J3 | 0,53 | 0,65 | 0,39 | -0,01 | 0,80 | 0,10 | 0,02 | 0,18 | -0,14 | 1,00 | -0,16 | 0,24 | 0,14 | 0,05 |
| | J2 + H₂O₂ | 0,28 | 1,46 | -0,37 | 0,65 | 3,03 | -0,04 | -0,01 | 1,06 | 0,99 | 1,47 | -0,01 | -0,27 | 0,47 | 0,55 |
| | J3 + H₂O₂ | 0,49 | 0,61 | -0,17 | 0,25 | 2,60 | 0,05 | -0,05 | 0,69 | 0,29 | 0,11 | 0,00 | -0,12 | 0,21 | 0,36 |
| **Vplaask** | J1 | -0,49^{.} | 0,62 | -0,06 | -0,40 | 0,11 | -0,55 | -0,31 | -0,40 | -0,39 | -0,53 | -0,43 | -0,12 | 0,42 | -0,31 |
| | J2 | -0,42 | -0,54 | -0,18 | 1,32 | 0,32 | 0,58 | 0,54 | 3,05 | 0,81 | 1,01 | 0,03 | 0,38 | 1,60 | 1,12 |
| | J3 | 0,26 | 0,49 | -0,47 | 3,32 | 0,75 | -0,02 | 0,74 | -0,16 | 0,95 | 0,56 | -0,21 | 0,46 | 1,95 | 0,36 |
| | J2 + H₂O₂ | -0,44 | 0,66 | -0,68 | 0,92 | 0,96 | 0,37 | 0,56 | 2,96 | 0,95 | 0,43 | 0,02 | 0,29 | 1,88 | 1,04 |
| | J3 + H₂O₂ | 0,23 | 0,25 | -0,21 | 3,31 | 2,25 | 0,07 | 0,78 | 0,74 | 0,05 | 1,54 | -0,04 | 0,85 | 2,89 | 0,75 |
| **H202** | J2+ H₂O₂ | 1,07 | 2,28 | -0,37 | 0,21 | 2,12 | -0,32 | -0,31 | 0,01 | 1,12 | 1,42 | -0,39 | -0,62 | 0,60 | -0,20 |
| | J3 + H₂O₂ | 0,12 | 1,32 | 0,36 | 0,30 | 1,61 | 0,30 | 0,04 | 1,53 | 1,44 | 1,27 | -0,03 | 0,09 | 0,37 | 0,33 |

### Essai 3 : Vérification de la fiabilité de l'outil sur des plantes soumises à 3 conditions de stress : lumineux, thermique, hydrique

### Protocole :

### • Stress lumineux

Des semis de Golden Delicious (similaires à ceux de l'essai 2) sont déplacés (J0) de la serre d'élevage vers diverses serres, chambres climatiques ou armoires climatiques, possédant des éclairages qualitativement et quantitativement différents (Tableau 10) avec une photopériode de 16h jour/8h nuit en cas d'éclairage artificiel et une thermopériode de 23°C jour/17°C nuit

**Tableau 10 - Conditions d'éclairage testées**

| N° condition | Lieu | Type d'éclairage | Contrôle température | Humidité |
|---|---|---|---|---|
| Condition 1 | Serre | Naturel + néon | Ouvrants | Ambiante |
| Condition 2 | Chambre | HPS + MH | Climatiseur | Contrôlée |
| Condition 3 | Chambre | MH | Climatiseur | Ambiante |
| Condition 4 | Chambre | HPIT | Climatiseur | Contrôlée |
| Condition 5 | Chambre | Néon | Climatiseur | Contrôlée |
| Condition 6 | Serre | Naturel + HPS | Ventilateur extracteur | Ambiante |
| Condition 7 | Serre | Naturel + HPS | Ventilateur extracteur | Ambiante |
| Condition 8 | Serre | Naturel + néon | Ventilateur extracteur + coolinq | Ambiante |
| Condition 9 | Armoire | Néon | Climatiseur | Ambiante |
| Condition 10 | Armoire | Néon | Climatiseur | Contrôlée |

Ils y subissent une acclimatation de 24h puis sont pulvérisés jusqu'à refus au pulvérisateur manuel avec du Bion (0,4 g/l) ou de l'eau (J1).

Des prélèvements (méthodologie identique à celle de l'essai 2) sont effectués à J0 avant déplacement (3 répétitions de 10 disques), puis à J1 avant traitement (3 répétitions de 10 disques) puis à J3 et J4 sur les lots traités (1 répétition de 10 disques/traitement/condition).

Les échantillons sont extraits et analysés à l'aide de l'outil selon l'invention de manière identique à celle de l'essai 2. Cette expérience a été réalisée une fois.

### • Stress thermique

Des semis de Golden Delicious (similaires à ceux de l'essai 2) sont déplacés (J0) de la serre d'élevage vers 2 armoires climatiques réglées sur le même régime de photopériode (16h jour/8h nuit) mais sur deux régimes différents de thermopériode (23°C jour/17°C nuit ou 35°C jour/17°C nuit).

Ils y subissent une acclimatation de 24h puis sont pulvérisés jusqu'à refus au pulvérisateur manuel avec du Bion (0,4 g/l) ou de l'eau (J1).

Des prélèvements (méthodologie identique à celle de l'essai 2) sont effectués à J1 avant traitement puis à J4 sur les lots traités (1 répétition de 10 disques/traitement/condition de température/date de prélèvement).

Les échantillons sont extraits et analysés à l'aide de l'outil selon l'invention de manière identique à celle de l'essai 2. Deux répétitions biologiques indépendantes ont été réalisées.

### • Stress hydrique

Des semis de Golden Delicious (similaires à ceux de l'essai 2) sont déplacés en serre d'expérimentation (J-1) et disposés en 3 lots.

Un lot subit immédiatement deux arrosages successifs au PEG6000 à 36% (submersion, attente du ressuyage, puis nouvelle submersion) (PEG à J-1).

Après une acclimatation de 24h de l'ensemble des semis, un 2ème lot est également traité au PEG6000 de façon identique que précédemment (PEG à J0).

L'ensemble des lots est ensuite pulvérisé jusqu'à refus au pulvérisateur manuel pour moitié au Bion (0,4 g/l) et pour moitié à l'eau.

Des prélèvements (méthodologie identique à celle de l'essai 2) sont effectués à J0 sur le lot non traité au PEG et avant traitement Bion ou eau, puis à J2 et J3 sur l'ensemble des lots (1 répétition de 10 disques/traitement/condition hydrique/date de prélèvement).

Les échantillons sont extraits et analysés à l'aide de l'outil selon l'invention de manière identique à celle de l'essai 2. Cette expérience a été réalisée une fois.

### Résultats :

### • Stress lumineux

Les résultats d'expression des 28 gènes cibles sont détaillés ci-après dans les tableaux 11 et 12 ; ces résultats sont répartis dans plusieurs tableaux, uniquement dans un souci de présentation.

Dans ces tableaux, les lignes correspondent respectivement :
- « Non traité » = modulation observées 24h (J1) après déplacement de la serre d'élevage ;
- « Eau» et « Bion » = modulation observées 48 et 72 heures après traitement, soit 3 et 4 jours après déplacement de la serre d'élevage (J3 et J4), moyennée, dans les feuilles de semis pulvérisés au Bion ou à l'eau, respectivement.

L'observation des profils d'induction des semis après 24h d'acclimatation dans les différentes conditions d'éclairage (tableaux 11 et 12) montre une corrélation entre puissance (et spectre) de l'éclairage d'appoint et induction des gènes de la voie des phénylpropanoïdes.

L'analyse des profils après traitement des semis au Bion, ou à l'eau, révèle que les conditions d'éclairage les plus stressantes ne permettent pas de mettre en évidence un fort pouvoir inducteur du Bion notamment au niveau des protéines PR (Tableau 11), ce qui n'est pas le cas des conditions moins stressantes.

Quelques gènes sont cependant moins sensibles aux conditions d'éclairage que ceux codant certaines protéines PR ou des enzymes de la voie des phénylpropanoïdes et permettent de mettre en évidence l'effet stimulateur du Bion dans la majorité des conditions : il s'agit de PR5, FAR et EDS1.

### • Stress thermique

Les résultats moyennés d'expression des 28 gènes cibles sont détaillés dans les tableaux 13 et 14 ; ces résultats sont répartis dans plusieurs tableaux, uniquement dans un souci de présentation.

Pour ces tableaux, la légende est la suivante : T° normales = 23°C jour / 17°C nuit ; T° normales puis stressantes après traitement = 23°C jour / 17°C nuit pendant 24h, puis 35°C jour / 17°C nuit ; T° stressantes = 35°C jour / 17°C nuit ; T° stressantes puis normales après traitement = 35°C jour / 17°C nuit pendant 24h puis 23°C jour / 17°C nuit.

Des conditions de stress thermiques (35°C) sont capables d'activer fortement certains gènes de l'outil selon l'invention, en particulier plusieurs protéines PR, dans les semis témoins (traités à l'eau) lorsque le stress est maintenu tout au long de l'expérimentation (4 jours), ou appliqué juste après pulvérisation de l'eau (Tableaux 13 et 14).

Cette induction est corrélée à une forte répression des gènes de la voie des phénylpropanoïdes (excepté PPO).

Ces effets d'induction/répression sont transitoires dès lors que le stress est interrompu, puisqu'ils ne sont plus observés dans les semis témoins stressés 24h puis remis en conditions « normales ».

L'induction de certaines protéines PR provoquée par le stress thermique appliqué jusqu'à la date de prélèvement masque les capacités du Bion à induire les protéines PR (Tableau 13).

Néanmoins d'autres gènes de l'outil permettent de révéler l'effet du Bion, même dans ces conditions stressantes, car faiblement induits par le stress thermique à lui seul (CSL, POX, WRKY).

Enfin un stress thermique transitoire appliqué quelques heures avant traitement au Bion ne gêne pas la mise en évidence de l'effet inducteur de ce produit.

### • Stress hydrique

Les résultats d'expression des 28 gènes cibles sont détaillés dans les tableaux 15 et 16 ; les résultats sont répartis dans plusieurs tableaux, uniquement dans un souci de présentation.

Dans les semis de Pommier, le stress hydrique tel qu'il a été appliqué (PEG) n'induit pas à lui seul les gènes de l'outil (Tableaux 15 et 16) : on n'observe pas de nettes différences entre les profils d'expression obtenus dans les 3 lots de plantes, stressées ou non, et pulvérisées à l'eau. De plus, il ne semble pas perturber l'effet inducteur du Bion, qui est révélé dans les 3 conditions.

### • Conclusion

Ces expériences démontrent l'intérêt d'étudier plusieurs groupes de gènes de défenses, voire plusieurs gènes à l'intérieur de chaque groupe, pour révéler l'effet stimulateur d'un SDN candidat.

En effet, des conditions environnementales un peu stressantes (fort éclairage et température élevée) peuvent induire par elles-mêmes certains gènes de défense présents sur l'outil selon l'invention et masquer les effets des SDN.

Pour une utilisation optimale de l'outil dans un objectif de criblage de produits candidats, il est cependant conseillé de réaliser les expériences dans des conditions d'éclairage artificiel modéré et dans des conditions thermiques les plus contrôlées possibles. Un stress hydrique, dans la limite du raisonnable bien-sûr, ne devrait pas perturber l'utilisation de l'outil.

**Tableau 11**

| | | **PR1** | **PR2** | **PR4** | **PR5** | **PR8** | **PR14** | **PR15** | **PAL** | **ANS** | **CHS** | **DFR** | **PPO** | **HMGR** | **FPPS** |
|---|---|---|---|---|---|---|---|---|---|---|---|---|---|---|---|
| **C1=Serre 1 d'élevage** | **Non traité** | 0,17 | -0,11 | -0,53 | -0,59 | 0,28 | -0,13 | -0,49 | 0,04 | 0,07 | -0,11 | -0,17 | -0,58 | 0,27 | -0,12 |
| **C2=Chambre climatique 1 (éclairage HPS + MH)** | **Non traité** | -0,62 | -0,18 | -1,14 | -1,98 | 1,73 | -1,02 | -0,96 | 6,72 | 6,96 | 6,58 | 6,30 | -1,08 | -1,22 | -2,25 |
| | **Eau** | -1,00 | -0,23 | -2,57 | 1,46 | 2,22 | -1,56 | 0,03 | 5,84 | 5,52 | 5,44 | 4,28 | 0,53 | 0,02 | -1,91 |
| | **Bion** | 0,37 | 0,99 | -0,53 | 5,11 | 3,70 | -0,26 | -0,13 | 5,90 | 5,78 | 5,42 | 4,33 | 0,18 | 0,92 | -2,06 |
| **C3=Chambre climatique 2 (éclairage MH)** | **Non traité** | -0,20 | 0,40 | -0,90 | -0,32 | 2,30 | -0,19 | -0,24 | 5,48 | 5,27 | 5,18 | 4,75 | 0,11 | -0,66 | -0,96 |
| | **Eau** | -0,44 | 3,52 | -0,39 | 3,06 | 2,68 | -1,02 | -0,11 | 4,83 | 3,94 | 4,34 | 3,39 | -0,59 | 0,19 | 0,06 |
| | **Bion** | 0,38 | 1,95 | -0,44 | 5,30 | 4,83 | -1,07 | -1,09 | 4,80 | 4,33 | 4,25 | 3,56 | 0,43 | 1,06 | -0,99 |
| **C4=Chambre climatique 3 (éclairage naturel + HPIT)** | **Non traité** | -0,30 | 0,10 | -1,32 | -0,80 | 0,16 | -0,71 | -0,40 | 5,67 | 5,18 | 5,04 | 4,13 | -0,65 | -0,74 | -1,87 |
| | **Eau** | -0,21 | -0,95 | -1,15 | 2,06 | 1,72 | -0,92 | 0,34 | 3,05 | 3,73 | 3,56 | 2,31 | 0,14 | -0,25 | -0,05 |
| | **Bion** | 2,98 | 5,12 | 4,90 | 6,7 | 6,05 | 0,30 | 2,61 | 3,73 | 3,16 | 3,57 | 2,36 | 3,02 | 1,91 | 0,37 |
| **C5=Chambre climatique 4 (éclairage néon ACTIVA172)** | **Non traité** | -0,47 | 0,19 | -3,56 | -0,47 | 0,96 | -0,97 | -0,20 | 3,99 | 4,55 | 4,10 | 4,04 | -0,88 | -1,14 | -1,39 |
| | **Eau** | -0,66 | -1,56 | -1,39 | 2,19 | 1,12 | -1,85 | -0,02 | 3,88 | 4,87 | 4,37 | 3,25 | 0,22 | -0,67 | -1,23 |
| | **Bion** | -0,38 | 3,73 | 0,97 | 6,49 | 4,67 | -0,62 | 1,83 | 4,54 | 5,28 | 4,91 | 3,81 | -0,05 | 0,33 | -1,27 |
| **C6=Serre 2 ' (éclairage naturel + HPS)** | **Non traité** | 0,46 | 0,161 | -1,65 | 0,05 | 2,03 | -0,50 | 0,31 | 5,20 | 5,25 | 5,13 | 4,63 | -0,42 | -0,66 | -1,59 |
| | **Eau** | 0,33 | -0,35 | -0,87 | 0,60 | 2,93 | -0,72 | 0,24 | 6,13 | 5,93 | 5,62 | 4,65 | 1,30 | -0,15 | -1,21 |
| | **Bion** | 3,23 | 7,90 | 6,36 | 6,13 | 6,24 | 1,70 | -0,89 | 4,86 | 5,11 | 4,78 | 4,11 | 2,22 | 1,40 | -1,16 |
| **C7=Serre 3 (éclairage naturel + HPS)** | **Non traité** | -0,05 | 0,23 | -1,10 | -0,62 | 1,63 | -0,13 | 0,33 | 3,44 | 1,65 | 2,50 | 0,15 | -0,65 | -0,16 | -0,59 |
| | **Eau** | -0,42 | -1,23 | -2,61 | 1,36 | 1,25 | -0,57 | 0,48 | 3,42 | 3,43 | 3,51 | 2,34 | 0,21 | -0,58 | -0,85 |
| | **Bion** | 1,62 | 6,48 | 5,54 | 7,45 | 6,08 | 1,66 | 1,60 | 4,55 | 4,48 | 3,98 | 3,72 | 2,72 | 1,68 | -0,05 |
| **C8=Serre 4 (éclairage naturel + néon)** | **Non traité** | 0,23 | 0,50 | -0,94 | 0,19 | 1,26 | -0,30 | 0,55 | 0,89 | 0,03 | 0,22 | -0,68 | -0,01 | -0,03 | 0,44 |
| | **Eau** | 0,23 | 0,38 | -0,49 | 2,31 | 2,28 | 0,04 | 0,29 | 1,37 | 1,11 | 1,28 | -0,22 | 0,08 | 0,58 | 0,75 |
| | **Bion** | 2,58 | 8,28 | 8,08 | 7,30 | 7,92 | 5,60 | 1,82 | 3,44 | 1,61 | 1,84 | 0,88 | 3,93 | 3,00 | 1,60 |
| **C9= Armoire 1 (éclairage néon)** | **Non traité** | -1,55 | -0,64 | 0,11 | -0,55 | 1,34 | 0,24 | 0,14 | 2,66 | 3,14 | 3,04 | 3,40 | -0,39 | -0,04 | -1,15 |
| | **Eau** | 0,64 | 1,16 | 1,38 | 1,73 | 3,19 | 1,24' | 1,75 | 2,79 | 3,26 | 2,88 | 3,56 | 0,81 | 1,10 | -0,59 |
| | **Bion** | 2,32 | 7,42 | 8,21 | 4,16 | 5,30 | 5,81 | 2,07 | 1,83 | 1,91 | 1,82 | 2,29 | 3,27 | 2,41 | 0,05 |
| **C10= Armoire 2 (éclairage néon)** | **Non traité** | 0,20 | -0,41 | -1,62 | -0,72 | 0,72 | 2,85 | 0,03 | 2,38 | 3,27 | 2,78 | 3,62 | -1,76 | -0,50 | -0,67 |
| | **Eau** | 2,25 | 5,52 | 1,79 | 3,62 | 3,70 | 6,49 | -0,26 | 0,22 | 1,07 | 0,60 | 2,03 | -1,49 | 0,75 | -0,17 |
| | **Bion** | 2,84 | 8,57 | 6,78 | 4,63 | 5,43 | 5,37 | -0,16 | 1,87 | 2,48 | 2,19 | 3,17 | 1,84 | 2,24 | -0,25 |

**Tableau 12**

| | | **FAR** | **CSL** | **APOX** | **GST** | **POX** | **CAD** | **CalS** | **PECT** | **EDS1** | **WRKY** | **LOX2** | **JAR** | **ACCO** | **EIN3** |
|---|---|---|---|---|---|---|---|---|---|---|---|---|---|---|---|
| **C1=Serre 1 d'élevage** | **Non traité** | 0,76 | -0,27 | 0,18 | -0,17 | 0,77 | 0,19 | 0,01 | -0,18 | -0,23 | -0,22 | 0,07 | -0,03 | -0,05 | 0,03 |
| **C2= Chambre climatique 1 (eclairage HPS + MH)** | **Non traité** | -6,15 | 4,58 | 1,08 | -2,55 | 0,80 | 0,39 | -1,10 | 0,40 | 1,20 | 0,55 | 0,36 | -0,02 | -0,84 | -0,58 |
| | **Eau** | -3,36 | 2,96 | 0,54 | -2,89 | 1,08 | 0,03 | -1,19 | -1,05 | 0,47 | -0,06 | -0,86 | -1,28 | -1,90 | -0,81 |
| | **Bion** | -0,35 | 2,94 | 0,26 | -2,84 | 2,09 | 0,17 | -1,05 | -0,12 | 3,08 | -0,50 | -1,44 | -1,40 | -1,38 | -0,24 |
| **C3= Chambre climatique 2 (éclairage MH)** | **Non traité** | -0,24 | 2,93 | 1,37 | -1,31 | 1,30 | 0,58 | -0,62 | 0,66 | -0,35 | -0,261 | 0,79 | 0,551 | -0,17 | 0,65 |
| | **Eau** | 1,75 | 2,11 | 0,66 | -2,02 | 1,30 | 0,20 | -1,13 | -0,64 | 1,32 | 0,76 | -0,45 | 0,49 | -0,67 | 0,30 |
| | **Bion** | 3,62 | 2,31 | 0,78 | -1,77 | 1,92 | 0,22 | -0,73 | 0,72 | 4,31 | -0,12 | 0,06 | 0,46 | -0,90 | 0,69 |
| **C4= Chambre climatique 3 (éclairage naturel + HPIT)** | **Non traité** | -1,46 | 2,30 | 1,59 | -2,20 | 0,58 | 0,03 | -0,80 | -0,51 | -1,04 | -0,12 | 0,39 | -0,14 | -1,19 | 0,20 |
| | **Eau** | 0,41 | 2,21 | 0,85 | -0,62 | 0,02 | 0,42 | -0,35 | -0,81 | 0,03 | -0,59 | -0,50 | -0,14 | 0,18 | 0,08 |
| | **Bion** | 2,77 | 3,62 | 0,88 | -0,37 | 2,03 | 0,70 | -0,38 | 1,36 | 4,31 | 3,88 | -0,65 | -0,18 | 0,78 | 1,00 |
| **C5= Chambre climatique 4 (éclairage néon ACTIVA172)** | **Non traité** | -1,06 | 2,44 | 1,11 | -2,03 | 1,83 | 0,29 | -0,91 | -0,60 | -1,63 | -0,53 | 0,17 | -0,05 | -0,69 | -0,72 |
| | **Eau** | 0,93 | 1,88 | 0,85 | 2,64 | 1,31 | -0,07 | -1,16 | -1,74 | 0,25 | -1,08 | -0,70 | -0,39 | -0,54 | 0,59 |
| | **Bion** | 3,38 | 2,84 | 1,14 | -2,67 | 2,93 | 0,21 | -0,98 | -0,38 | 3,73 | 1,17 | -0,64 | -0,10 | -0,70 | -0,06 |
| **C6=Serre 2 (éclairage naturel + HPS)** | **Non traité** | 0,34 | 2,96 | 1,01 | -2,08 | 1,99 | 0,54 | -0,87 | -0,43 | 0,19 | 1,471 | 0,65 | 0,271 | -0,70 | -0,51 |
| | **Eau** | 2,57 | 3,59 | 1,05 | -2,46 | 2,45 | 0,33 | 0,82 | -0,24 | 0,48 | -0,21 | 0,68 | 0,14 | -0,48 | 0,55 |
| | **Bion** | 5,30 | 4,54 | 0,91 | -1,58 | 4,03 | 0,39 | -1,12 | 1,60 | 4,35 | 4,24 | 0,37 | -0,22 | 0,52 | 1,14 |
| **C7=Serre 3 (éclairage naturel + HPS)** | **Non traité** | -0,44 | -0,06 | 0,75 | -1,02 | 1,26 | 0,07 | -0,38 | -0,23 | -0,10 | 0,91 | 0,86 | 0,49 | -0,57 | 0,21 |
| | **Eau** | 1,78 | 1,04 | 0,88 | 2,07 | 1,54 | 0,04 | -1,08 | -1,02 | 0,36 | 0,29 | -0,45 | -0,31 | -0,33 | 0,01 |
| | **Bion** | 5,67 | 4,22 | 1,42 | -0,93 | 3,37 | 0,48 | -0,54 | 1,25 | 4,44 | 1,78 | 0,11 | 0,55 | 0,31 | 0,96 |
| **C8=Serre 4 (éclairage naturel + néon)** | **Non traité** | 1,20 | -0,37 | 0,95 | 0,04 | 0,70 | 0,23 | 0,27 | 0,33 | -0,54 | 1,27 | 0,65 | 0,80 | 0,38 | -0,32 |
| | **Eau** | 0,38 | 1,52 | 0,45 | -0,40 | 0,97 | 0,30 | 0,07 | 0,12 | 0,40 | 1,45 | 0,15 | 0,34 | 0,55 | 0,28 |
| | **Bion** | 4,83 | 5,99 | 0,90 | 1,52 | 5,50 | 0,63 | 0,33 | 2,42 | 5,40 | 5,02 | -0,17 | -0,13 | 2,06 | 2,01 |
| **C9= Armoire 1 (éclairage néon)** | **Non traité** | -0,27 | 0,97 | 0,25 | -1,86 | -0,78 | 0,00 | -0,72 | -0,05 | 0,85 | 1,20 | 0,07 | -0,50 | -0,79 | 0,15 |
| | **Eau** | 2,86 | 2,06 | 0,21 | -1,14 | 2,36 | 0,22 | -0,12 | 2,61 | 3,05 | 2,13 | 0,19 | -0,66 | -0,82 | 1,05 |
| | **Bion** | 5,22 | 3,67 | 0,77 | 0,07 | 3,36 | 0,03 | -0,15 | 2,90 | 5,30 | 5,37 | -0,08 | -0,58 | 1,02 | 0,91 |
| **C10= Armoire 2 (éclairage néon)** | **Non traité** | 0,14 | 1,45 | 0,12 | -0,57 | -0,55 | 0,00 | -0,96 | 1,36 | 0,32 | -0,20 | 0,10 | 0,11 | -0,05 | 0,73 |
| | **Eau** | 3,59 | 0,52 | 0,13 | -0,09 | 1,75 | -0,02 | -0,31 | 3,18 | 3,27 | -1,10 | -0,24 | -0,26 | -0,17 | 0,67 |
| | **Bion** | 5,00 | 2,53 | 0,32 | 0,43 | 4,22 | 0,36 | -0,64 | 4,23 | 4,88 | 3,94 | -0,16 | -0,54 | 0,73 | 1,24 |

**Tableau 13**

| | | **PR1** | **PR2** | **PR4** | **PR5** | **PR8** | **PR14** | **PR15** | **PAL** | **ANS** | **CHS** | **DFR** | **PPO** | **HMGR** | **FPPS** |
|---|---|---|---|---|---|---|---|---|---|---|---|---|---|---|---|
| **T° normales** | **Eau** | 0,99 | -1,03 | -0,48 | 2,02 | 0,63 | -1,46 | 0,77 | -1,05 | 1,05 | -1,03 | -1,68 | -0,4 | 0,91 | -1,39 |
| | **Bion** | 3,79 | 6,22 | 7,77 | 4,21 | 3,67 | 1,89 | -0,57 | -2,16 | -1,35 | 1,64 | -1,77 | -0,76 | 1,63 | -1,12 |
| **T° normales puis stressantes** | **Eau** | 5,59 | 7,91 | 9,28 | 1,34 | 3,53 | 7,13 | -0,01 | -4,77 | -4,96 | -5,65 | -4,77 | 0,13 | 1,36 | 0,77 |
| | **Bion** | 9,82 | 11,2 | 12,9 | 4,34 | 6,38 | 7,86 | 0,66 | -3,71 | -4,7 | 5,86 | -5,39 | 2,82 | 4,36 | 1,18 |
| **T° stressantes** | **Eau** | 7,02 | 7,46 | 9,57 | 2,04 | 4,78 | 4,79 | 4,62 | -3,96 | 4,61 | -5,04 | -5,27 | 1,61 | 2,43 | 0,92 |
| | **Bion** | 9,55 | 11,3 | 14 | 3,71 | 7,96 | 9,23 | 4,65 | -3,93 | 5,29 | -6,17 | -5,36 | 5,42 | 4,77 | 1,36 |
| **T° stressantes puis normales** | **Eau** | 2,95 | 1,1 | 0,87 | 2,64 | 2,19 | -0,27 | -0,07 | 1,19 | 0,5 | -1,01 | 1,08 | 1,54 | 1,48 | 1,14 |
| | **Bion** | 6,46 | 8,67 | 9,55 | 4,9 | 5,36 | 4,57 | 0,12 | 1,95 | -1,94 | -2,48 | -1,63 | 1,35 | 3,17 | 0,77 |

**Tableau 14**

| | | **FAR** | **CSL** | **APOX** | **GST** | **POX** | **CAD** | **Cals** | **PECT** | **EDS1** | **WRKY** | **LOX2** | **JAR** | **ACCO** | **EIN3** |
|---|---|---|---|---|---|---|---|---|---|---|---|---|---|---|---|
| **T° normales** | **Eau** | 2,44 | -0,24 | -1,29 | -1,05 | -1,03 | -0,25 | -0,45 | 0,48 | 2,02 | 1,15 | 0,19 | -1,04 | -0,8 | 0,32 |
| | **Bion** | 3,34 | 0,91 | -0,97 | -0,33 | 1,96 | 0,11 | -0,54 | 1,22 | 2,96 | 0,93 | 0,16 | -0,82 | -0,24 | 0,14 |
| **T° normales puis stressantes** | **Eau** | 1,16 | 0,82 | -1,45 | 2,4 | 0,97 | 0,43 | 1,12 | 3,49 | 2,56 | 1,03 | 1,35 | 1,03 | 1,69 | 1,94 |
| | **Bion** | 0,45 | 4,65 | -1,18 | 1,89 | 5,54 | 0,4 | 1,05 | 2,04 | 4,79 | 5,48 | 0,66 | 0,63 | 1,29 | 0,98 |
| **T° stressantes** | **Eau** | 1,16 | 1,26 | -1,23 | 2,28 | 1,96 | 0,66 | 0,65 | 2,77 | 2,46 | 1,51 | 0,49 | 0,45 | 1,16 | 1,29 |
| | **Bion** | -0,47 | 6,17 | -0,56 | 2,83 | 6 | 0,93 | 1,3 | 2,56 | 4,44 | 4,66 | -0,64 | 0,83 | 1,52 | 1,23 |
| **T° stressantes puis normales** | **Eau** | 1,88 | -0,13 | -0,86 | -0,4 | 0,79 | 0,03 | -0,29 | 1,16 | 2,45 | -0,58 | 0,39 | 0,72 | 0,61 | 0,82 |
| | **Bion** | 4 | 2,71 | 0,57 | 1,68 | 3,72 | 0,19 | 0,09 | 1,9 | 3,73 | 4,93 | 0,15 | -0,59 | 1,27 | 0,89 |

**Tableau 15**

| | | **PR1** | **PR2** | **PR4** | **PR5** | **PR8** | **PR14** | **PR15** | **PAL** | **ANS** | **CHS** | **DFR** | **PPO** | **HMGR** | **FPPS** |
|---|---|---|---|---|---|---|---|---|---|---|---|---|---|---|---|
| Arrosage normal | Eau | -1,0 | 4,7 | 5,1 | 1,8 | 1,3 | 1,3 | -0,3 | 1,3 | 1,8 | 1,8 | 0,7 | -0,1 | -0,1 | 0,2 |
| | Bion | 2,8 | 10,3 | 12,0 | 5,2 | 5,0 | 5,0 | -0,4 | 1,3 | 1,1 | 1,1 | 0,9 | 5,1 | 2,4 | 1,0 |
| PEG à J-1 | Eau | 0,1 | 1,5 | 5,7 | 1,8 | 1,6 | 3,5 | 0,5 | 0,9 | 1,5 | 1,7 | 1,1 | 0,2 | 0,2 | -0,3 |
| | Bion | 7,2 | 10,8 | 11,0 | 6,2 | 4,6 | 7,3 | 0,0 | 1,3 | 1,4 | 1,6 | 1,4 | 2,7 | 1,9 | 0,5 |
| PEG à J0 | Eau | 1,8 | 4,0 | 7,9 | 0,8 | 2,5 | 1,0 | 0,3 | 1,0 | 2,1 | 1,6 | 0,7 | 1,9 | 0,2 | 0,0 |
| | Bion | 7,1 | 10,6 | 11,0 | 6,1 | 4,8 | 4,5 | -1,0 | 0,9 | 1,5 | 1,3 | 1,3 | 2,6 | 1,9 | 0,1 |

**Tableau 16**

| | **FAR** | **FAR** | **CSL** | **APOX** | **GST** | **POX** | **CAD** | **CalS** | **PECT** | **EDS1** | **WRKY** | **LOX2** | **JAR** | **ACCO** | **ElN3** |
|---|---|---|---|---|---|---|---|---|---|---|---|---|---|---|---|
| Arrosage normal | Eau | 1,2 | 1,1 | 0,3 | 0,1 | -1,2 | -0,5 | 0,1 | 0,3 | 1,6 | -0,3 | -0,3 | -0,2 | 0,2 | -2,1 |
| | Bion | 4,0 | 5,5 | 0,1 | 1,6 | 2,0 | -0,3 | -0,2 | 1,5 | 4,7 | 5,9 | -0,5 | -0,3 | 1,8 | -0,7 |
| PEG à J-1 | Eau | 3,0 | 1,8 | 0,4 | 0,4 | -0,9 | 0,0 | 0,0 | 1,6 | 1,3 | 0,2 | 0,7 | 0,2 | 0,9 | -1,4 |
| | Bion | 5,9 | 4,7 | 0,9 | 1,1 | 1,0 | 0,1 | 0,3 | 2,7 | 4,4 | 5,1 | 1,1 | 0,5 | 2,1 | -0,4 |
| PEG à J0 | Eau | 2,8 | 1,7 | 0,6 | 0,4 | -2,0 | 0,1 | 0,5 | 0,7 | 1,1 | 0,2 | 1,1 | 0,4 | 0,8 | -1,6 |
| | Bion | 5,0 | 4,7 | 0,4 | 10 | 2,0 | 0,2 | 0,1 | 1,5 | 4,4 | 3,7 | 0,5 | 0,0 | 1,2 | -0,9 |

### Essai 4 : Vérification de la capacité de l'outil à étudier diverses variétés de Pommier pour leur réponse aux SDN

### Méthode:

Cette expérimentation est effectuée une fois sur jeunes scions greffés, de 4 variétés en croissance active: Braeburn, Gala, Granny Smith, MM106 élevés, directement en serre d'expérimentation.

### • Pour l'analyse des défenses

Les scions sont pulvérisés jusqu'à refus au pulvérisateur à air comprimé avec du Bion (0,4 g/l) ou de l'eau (J0).

Des prélèvements des feuilles F3 (3ème étage foliaire, feuilles jeunes non encore complètement développées) sont effectués à J0 (avant traitement) et à J3 sur l'ensemble des lots traités (trois ½ limbes prélevés sur trois feuilles F3 (jeunes feuilles non entièrement développées) poolées / prélèvement).

Les échantillons sont extraits et analysés à l'aide de l'outil de manière identique à celle de l'essai 2.

### • Pour l'analyse du pouvoir de protection

Les scions sont pulvérisés de la même manière jusqu'à refus au pulvérisateur à air comprimé avec du Bion (0,4 g/l) ou de l'eau (J0).

A J4, les 2 plus jeunes feuilles développées (F1 et F2) de chaque scion sont coupées, au 2/3 et perpendiculairement à la nervure principale, à l'aide de ciseaux préalablement trempés dans une suspension bactérienne d'*Erwinia amylovora* préparée dans de l'eau stérile à 10⁸ cfu/ml. Une douzaine de pousses en croissance active ont été inoculées par variété et par traitement.

La notation des symptômes, c'est-à-dire présence d'une nécrose sur la nervure des feuilles inoculées (noté 0,5), ou ayant atteint le pétiole (noté 1), ou ayant évolué sur tige (noté 1+longueur de nécrose sur tige en cm), est réalisée 3 semaines après inoculation. La longueur moyenne de nécrose sur scions pulvérisés (µₑₐᵤ) est calculée et l'efficacité relative de protection du Bion est estimée selon la formule : 100 - ((Lg_{Bion} x 100)/µₑₐᵤ).

### Résultats :

Le Bion provoque des inductions dans les 4 variétés selon un profil qualitatif assez similaire (Figure 2A).

Au niveau quantitatif, on ne repère pas une variété qui « réponde » globalement plus fortement qu'une autre. Les différences de niveaux d'induction varient en amplitude selon les gènes.

Concernant les efficacités de protection de ces 4 variétés par le Bion, vis-à-vis du feu bactérien, on observe des différences marquées, Granny Smith étant la variété « répondant » le mieux au Bion, et Gala, celle « répondant » le moins (Figure 2B).

### Conclusion :

L'outil mis au point sur des semis de Golden Delicious peut donc être utilisé sur d'autres variétés de Pommier. Il permet de révéler la réactivité des 4 autres variétés testées ici, Braebum, Gala, Granny Smith et MM106, à un traitement au Bion, réactivité confirmée par les protections observées après inoculation artificielle par *E. amylovora.*

## Revendications

1. Dispositif pour déterminer ou étudier l'état de stimulation des défenses naturelles de plantes ou parties de plantes, lequel dispositif comprend des moyens de détermination du niveau d'expression en ARNm exprimé par une combinaison de gènes cibles dans un échantillon de plantes ou parties de plantes, lesdits moyens de détermination comprenant :
(a) un moyen de détermination du niveau d'expression en ARNm d'au moins un gène cible choisi parmi les gènes cibles suivants : PR-1, PR-2, PR-4, PR-5, PR-8, PR-14, PR-15 ;
(b) un moyen de détermination du niveau d'expression en ARNm d'au moins un gène cible choisi parmi les gènes cibles suivants : PAL, CHS, DFR, ANS, PPO ;
(c) un moyen de détermination du niveau d'expression en ARNm d'au moins un gène cible choisi parmi les gènes cibles suivants : HMGR, FPPS, Far ;
(d) un moyen de détermination du niveau d'expression en ARNm du gène cible CSL ;
(e) un moyen de détermination du niveau d'expression en ARNm d'au moins un gène cible choisi parmi les gènes suivants : APOX, GST, POX ;
(f) un moyen de détermination du niveau d'expression en ARNm d'au moins un gène cible choisi parmi les gènes cibles suivants : CalS, Pect, CAD ;
(g) un moyen de détermination du niveau d'expression en ARNm d'au moins un gène cible choisi parmi les gènes cibles suivants : EDS1, WRKY ;
(h) un moyen de détermination du niveau d'expression en ARNm d'au moins un gène cible choisi parmi les gènes cibles suivants : LOX2, JAR ;
(i) un moyen de détermination du niveau d'expression en ARNm d'au moins un gène cible choisi parmi les gènes cibles suivants : ACCO, EIN3.

2. Dispositif selon la revendication 1, **caractérisé en ce qu'**il comprend les moyens de détermination du niveau d'expression en ARNm des gènes suivants :
(i) pour le groupe (a), un moyen de détermination du niveau d'expression en ARNm de l'un au moins des gènes suivants : PR-1, PR-2, PR-4 et/ou PR-8, un moyen de détermination du niveau d'expression en ARNm du gène PR-5, un moyen de détermination du niveau d'expression en ARNm du gène PR-14 et un moyen de détermination du niveau d'expression en ARNm du gène PR-15,
(ii) pour le groupe (b), un moyen de détermination du niveau d'expression en ARNm du gène PAL, un moyen de détermination du niveau d'expression en ARNm de l'un au moins des gènes suivants : CHS, DFR ou ANS, et un moyen de détermination du niveau d'expression en ARNm du gène PPO,
(iii) pour le groupe (c), un moyen de détermination du niveau d'expression en ARNm de l'un au moins des gènes suivants : HMGR et Far, et un moyen de détermination du niveau d'expression en ARNm du gène FPPS,
(iv) pour le groupe (e), un moyen de détermination du niveau d'expression en ARNm du gène APOX et un moyen de détermination du niveau d'expression en ARNm de l'un au moins des gènes suivants : GST et POX.

3. Dispositif selon l'une quelconque des revendications 1 ou 2, **caractérisé en ce qu'**il comprend les moyens de détermination du niveau d'expression en ARNm de la combinaison des gènes cibles suivants : PR-1, PR-2, PR-4, PR-5, PR-8, PR-14, PR-15, PAL, CHS, DFR, ANS, PPO, HMGR, FPPS, Far, CSL, APOX, GST, POX, CalS, Pect, CAD, EDS1, WRKY, LOX2, JAR, ACCO, EIN3.

4. Dispositif selon l'une quelconque des revendications 1 à 3, **caractérisé en ce que** lesdits moyens de détermination du niveau d'expression en ARNm d'un gène cible sont choisis parmi les fragments d'acide nucléique capables de s'hybrider de manière spécifique aux ARNm exprimés par ledit gène cible ou aux ADNc correspondants, ou à des fragments desdits ARNm ou desdits ADNc.

5. Dispositif selon la revendication 4, **caractérisé en ce que** les fragments d'acide nucléique consistent en des amorces nucléotidiques s'hybridant spécifiquement avec les ARNm, les ADNc, ou des fragments de ceux-ci.

6. Dispositif selon la revendication 5, **caractérisé en ce que** lesdites amorces nucléotidiques sont adaptées à la détermination du niveau d'expression en ARNm des gènes cibles par une méthode de PCR quantitative.

7. Dispositif selon la revendication 6, **caractérisé en ce que** les amorces sont choisies parmi les séquences suivantes SEQ ID n°32 à 87.

8. Dispositif selon la revendication 4, **caractérisé en ce que** lesdits fragments d'acide nucléique sont immobilisés sur un support.

9. Dispositif selon la revendication 8, **caractérisé en ce qu'**il consiste en une puce à ADN.

10. Procédé pour identifier un profil d'expression en ARNm d'une combinaison de gènes cibles permettant de déterminer, ou au moins d'évaluer, un état de stimulation des défenses naturelles de plantes ou de parties de plantes, lequel procédé comprend les étapes suivantes :
(i) déterminer le profil d'expression en ARNm d'une combinaison de gènes cibles au moyen du dispositif selon l'une des revendications 1 à 9, sur un ensemble de plantes ou de parties de plantes, dont l'état de stimulation de leurs défenses naturelles est connu, puis
(ii) déterminer un profil d'expression en ARNm de ladite combinaison de gènes cibles correspondant à un état déterminé de stimulation des défenses naturelles desdites plantes ou parties de plantes, partant des données issues de l'étape (i).

11. Procédé pour déterminer ou évaluer l'état de stimulation des défenses naturelles d'une plante ou d'une partie de plante, comprenant les étapes suivantes :
(i) prélever un échantillon à partir de ladite plante ou de ladite partie de plante,
(ii) déterminer le profil d'expression en ARNm d'une combinaison de gènes cibles dans ledit échantillon prélevé à l'étape (i), au moyen du dispositif selon l'une des revendications 1 à 9,
(iii) comparer le profil d'expression en ARNm obtenu à l'étape (ii) avec un profil d'expression en ARNm de référence,
(iv) déterminer ou évaluer l'état de stimulation des défenses naturelles de ladite plante ou de ladite partie de plante, à partir dudit profil d'expression en ARNm obtenu lors de l'étape (ii).

12. Procédé pour sélectionner une substance ayant la propriété de moduler l'état de stimulation des défenses naturelles d'une plante ou d'une partie de plante, comprenant les étapes suivantes :
(i) mettre en contact ladite plante ou ladite partie de plante avec la substance à tester,
(ii) déterminer le profil d'expression en ARNm d'une combinaison de gènes cibles dans un échantillon prélevé à partir de ladite plante ou de ladite partie de plante suite à l'étape (i), au moyen du dispositif selon l'une des revendications 1 à 9,
(iii) comparer le profil d'expression en ARNm obtenu à l'étape (ii) avec un profil d'expression en ARNm de référence, pour déterminer ou évaluer l'état de stimulation des défenses naturelles dans ledit échantillon,
(iv) sélectionner positivement ladite substance si la comparaison à l'étape (iii) montre que ladite substance testée à l'étape (i) module l'état de stimulation des défenses naturelles de ladite plante ou de ladite partie de plante.

13. Procédé selon la revendication 12, pour sélectionner une substance ayant la propriété de générer un état de stimulation des défenses naturelles d'une plante ou d'une partie de plante appartenant à la famille des Rosaceae, assurant une protection vis à vis d'un stress biotique, **caractérisé en ce que** le profil d'expression en ARNm de référence à l'étape (iii) correspond aux valeurs observées pour des échantillons de plantes ou de parties de plantes non soumises à un stress ou un stimulateur des défenses naturelles, et **en ce que** l'étape (iv) consiste à sélectionner positivement ladite substance testée si le profil d'expression en ARNm obtenu à l'étape (ii), et comparé à l'étape (iii), montre une surexpression en ARNm de la combinaison de gènes cibles suivantes : PR-1, PR-2, PR-4, PR-5, PR-8, PR-14, HMGR, Far, CSL, POX, Pect, EDS1 et WRKY.

14. Procédé pour sélectionner une plante présentant un état de stimulation des défenses naturelles susceptible de leur conférer une résistance améliorée à au moins un stress biotique et/ou abiotique d'intérêt, comprenant les étapes suivantes :
(i) appliquer ledit ou lesdits stress à une plante ou une partie de plante,
(ii) déterminer le profil d'expression en ARNm d'une combinaison de gènes cibles dans un échantillon prélevé à partir de la plante ou de ladite partie de plante, au moyen du dispositif selon l'une des revendications 1 à 9,
(iii) comparer le profil d'expression en ARNm obtenu à l'étape (ii) avec un profil d'expression en ARNm de référence, pour déterminer ou évaluer l'état de stimulation des défenses naturelles dans ledit échantillon,
(iv) sélectionner positivement ladite plante ou ladite partie de plante si la comparaison de l'étape (iii) montre que ladite plante ou ladite partie de plante possède un état de stimulation des défenses naturelles susceptible de leur conférer une résistance améliorée à au moins un stress biotique et/ou abiotique d'intérêt.

15. Procédé selon l'une quelconque des revendications 10 à 14, **caractérisé en ce que** la ou les plantes appartiennent à la famille des *Rosaceae.*

## Patentansprüche

1. Vorrichtung zum Bestimmen oder Untersuchen des Stimulationszustands der natürlichen Abwehr von Pflanzen oder Pflanzenteilen, wobei die Vorrichtung Einrichtungen zur Bestimmung des mRNA-Expressionsniveaus umfasst, das durch eine Kombination von Target-Genen in einer Probe von Pflanzen oder Pflanzenteilen ausgedrückt wird, wobei die Bestimmungseinrichtungen umfassen:
(a) eine Einrichtung zur Bestimmung des mRNA-Expressionsniveaus wenigstens eines Target-Gens, das aus den folgenden Target-Genen ausgewählt ist: PR-1, PR-2, PR-4, PR-5, PR-8, PR-14, PR-15;
(b) eine Einrichtung zur Bestimmung des mRNA-Expressionsniveaus wenigstens eines Target-Gens, das aus den folgenden Target-Genen ausgewählt ist: PAL, CHS, DFR, ANS, PPO;
(c) eine Einrichtung zur Bestimmung des mRNA-Expressionsniveaus wenigstens eines Target-Gens, das aus den folgenden Target-Genen ausgewählt ist: HMGR, FPPS, Far;
(d) eine Einrichtung zur Bestimmung des mRNA-Expressionsniveaus des Target-Gens CSL;
(e) eine Einrichtung zur Bestimmung des mRNA-Expressionsniveaus wenigstens eines Target-Gens, das aus den folgenden Genen ausgewählt ist: APOX, GST, POX;
(f) eine Einrichtung zur Bestimmung des mRNA-Expressionsniveaus wenigstens eines Target-Gens, das aus den folgenden Target-Genen ausgewählt ist: CalS, Pect, CAD;
(g) eine Einrichtung zur Bestimmung des mRNA-Expressionsniveaus wenigstens eines Target-Gens, das aus den folgenden Target-Genen ausgewählt ist: EDS1, WRKY;
(h) eine Einrichtung zur Bestimmung des mRNA-Expressionsniveaus wenigstens eines Target-Gens, das aus den folgenden Target-Genen ausgewählt ist: LOX2, JAR;
(i) eine Einrichtung zur Bestimmung des mRNA-Expressionsniveaus wenigstens eines Target-Gens, das aus den folgenden Target-Genen ausgewählt ist: ACCO, EIN3.

2. Vorrichtung gemäß Anspruch 1, **dadurch gekennzeichnet, dass** sie die Einrichtungen zur Bestimmung des mRNA-Expressionsniveaus der folgenden Gene umfasst:
(i) für Gruppe (a): eine Einrichtung zur Bestimmung des mRNA-Expressionsniveaus wenigstens eines der folgenden Gene: PR-1, PR-2, PR-4 und/oder PR-8, eine Einrichtung zur Bestimmung des mRNA-Expressionsniveaus des Gens PR-5, eine Einrichtung zur Bestimmung des mRNA-Expressionsniveaus des Gens PR-14 und eine Einrichtung zur Bestimmung des mRNA-Expressionsniveaus des Gens PR-15;
(ii) für Gruppe (b): eine Einrichtung zur Bestimmung des mRNA-Expressionsniveaus des Gens PAL, eine Einrichtung zur Bestimmung des mRNA-Expressionsniveaus wenigstens eines der folgenden Gene: CHS, DFR oder ANS, und eine Einrichtung zur Bestimmung des mRNA-Expressionsniveaus des Gens PPO;
(iii) für Gruppe (c): eine Einrichtung zur Bestimmung des mRNA-Expressionsniveaus wenigstens eines der folgenden Gene: HMGR und Far, und eine Einrichtung zur Bestimmung des mRNA-Expressionsniveaus des Gens FPPS;
(iv) für Gruppe (e): eine Einrichtung zur Bestimmung des mRNA-Expressionsniveaus des Gens APOX und eine Einrichtung zur Bestimmung des mRNA-Expressionsniveaus wenigstens eines der folgenden Gene: GST und POX.

3. Vorrichtung gemäß einem der Ansprüche 1 oder 2, **dadurch gekennzeichnet, dass** sie die Einrichtungen zur Bestimmung des mRNA-Expressionsniveaus der Kombination der folgenden Target-Gene umfasst: PR-1, PR-2, PR-4, PR-5, PR-8, PR-14, PR-15, PAL, CHS, DFR, ANS, PPO, HMGR, FPPS, Far, CSL, APOX, GST, POX, CalS, Pect, CAD, EDS1, WRKY, LOX2, JAR, ACCO, EIN3.

4. Vorrichtung gemäß einem der Ansprüche 1 bis 3, **dadurch gekennzeichnet, dass** die Einrichtungen zur Bestimmung des mRNA-Expressionsniveaus eines Target-Gens aus den Nucleinsäurefragmenten ausgewählt sind, die spezifisch mit den von dem Target-Gen exprimierten mRNAs oder mit den entsprechenden cDNAs oder mit Fragmenten der mRNAs oder der cDNAs hybridisieren können.

5. Vorrichtung gemäß Anspruch 4, **dadurch gekennzeichnet, dass** die Nucleinsäurefragmente aus Nucleotidprimern bestehen, die spezifisch mit den mRNAs, den cDNAs oder deren Fragmenten hybridisieren.

6. Vorrichtung gemäß Anspruch 5, **dadurch gekennzeichnet, dass** die Nucleotidprimer für die Bestimmung des mRNA-Expressionsniveaus der Target-Gene durch ein quantitatives PCR-Verfahren geeignet sind.

7. Vorrichtung gemäß Anspruch 6, **dadurch gekennzeichnet, dass** die Primer aus den folgenden Sequenzen SEQ ID Nr. 32 bis 87 ausgewählt sind.

8. Vorrichtung gemäß Anspruch 4, **dadurch gekennzeichnet, dass** die Nucleinsäurefragmente auf einem Träger immobilisiert sind.

9. Vorrichtung gemäß Anspruch 8, **dadurch gekennzeichnet, dass** sie aus einem DNA-Chip besteht.

10. Verfahren zum Identifizieren eines mRNA-Expressionsprofils einer Kombination von Target-Genen, das es ermöglicht, einen Stimulationszustand der natürlichen Abwehr von Pflanzen oder Pflanzenteilen zu bestimmen oder wenigstens zu bewerten, wobei das Verfahren die folgenden Schritte umfasst:
(i) Bestimmen des mRNA-Expressionsprofils einer Kombination von Target-Genen mittels der Vorrichtung gemäß einem der Ansprüche 1 bis 9 an einer Menge von Pflanzen oder Pflanzenteilen, deren Stimulationszustand ihrer natürlichen Abwehr bekannt ist, dann
(ii) Bestimmen eines mRNA-Expressionsprofils der Kombination von Target-Genen, das einem bestimmten Stimulationszustand der natürlichen Abwehr der Pflanzen oder Pflanzenteile entspricht, ausgehend von den Daten, die aus Schritt (i) hervorgegangen sind.

11. Verfahren zum Bestimmen oder Bewerten des Stimulationszustands der natürlichen Abwehr einer Pflanze oder eines Pflanzenteils, umfassend die folgenden Schritte:
(i) Entnehmen einer Probe von der Pflanze oder dem Pflanzenteil;
(ii) Bestimmen des mRNA-Expressionsprofils einer Kombination von Target-Genen in der in Schritt (i) entnommenen Probe mittels der Vorrichtung gemäß einem der Ansprüche 1 bis 9;
(iii) Vergleichen des in Schritt (ii) erhaltenen mRNA-Expressionsprofils mit einem Referenz-mRNA-Expressionsprofil;
(iv) Bestimmen oder Bewerten des Stimulationszustands der natürlichen Abwehr der Pflanze oder des Pflanzenteils ausgehend von dem in Schritt (ii) erhaltenen mRNA-Expressionsprofil.

12. Verfahren zum Auswählen einer Substanz mit der Eigenschaft, den Stimulationszustand der natürlichen Abwehr einer Pflanze oder eines Pflanzenteils zu modulieren, umfassend die folgenden Schritte:
(i) In-Kontakt-Bringen der Pflanze oder des Pflanzenteils mit der zu testenden Substanz;
(ii) Bestimmen des mRNA-Expressionsprofils einer Kombination von Target-Genen in einer von der Pflanze oder dem Pflanzenteil entnommenen Probe anschließend an Schritt (i) mittels der Vorrichtung gemäß einem der Ansprüche 1 bis 9;
(iii) Vergleichen des in Schritt (ii) erhaltenen mRNA-Expressionsprofils mit einem Referenz-mRNA-Expressionsprofil, um den Stimulationszustand der natürlichen Abwehr in der Probe zu bestimmen oder zu bewerten;
(iv) positives Auswählen der Substanz, wenn der Vergleich in Schritt (iii) zeigt, dass die in Schritt (i) getestete Substanz den Stimulationszustand der natürlichen Abwehr der Pflanze oder des Pflanzenteils moduliert.

13. Verfahren gemäß Anspruch 12 zum Auswählen einer Substanz mit der Eigenschaft, einen Stimulationszustand der natürlichen Abwehr einer Pflanze oder eines Pflanzenteils, die zur Familie der Rosaceae gehören, zu erzeugen, der einen Schutz gegenüber einem biotischen Stress gewährleistet, **dadurch gekennzeichnet, dass** das Referenz-mRNA-Expressionsprofil in Schritt (iii) den Werten entspricht, die bei Proben von Pflanzen oder Pflanzenteilen, die keinem Stress oder Stimulator der natürlichen Abwehr ausgesetzt wurden, beobachtet werden, und dadurch, dass Schritt (iv) darin besteht, die getestete Substanz positiv auszuwählen, wenn das in Schritt (ii) erhaltene und in Schritt (iii) getestete mRNA-Expressionsprofil eine mRNA-Überexpression der Kombination der folgenden Target-Gene zeigt: PR-1, PR-2, PR-4, PR-5, PR-8, PR-14, HMGR, Far, CSL, POX, Pect, EDS1 und WRKY.

14. Verfahren zum Auswählen einer Pflanze, die einen Stimulationszustand der natürlichen Abwehr aufweist, der ihr eine verbesserte Resistenz gegen wenigstens einen interessierenden biotischen und/oder abiotischen Stress verleihen kann, umfassend die folgenden Schritte:
(i) Einwirkenlassen des oder der Stressfaktoren auf eine Pflanze oder einen Pflanzenteil;
(ii) Bestimmen des mRNA-Expressionsprofils einer Kombination von Target-Genen in einer von der Pflanze oder dem Pflanzenteil entnommenen Probe mittels der Vorrichtung gemäß einem der Ansprüche 1 bis 9;
(iii) Vergleichen des in Schritt (ii) erhaltenen mRNA-Expressionsprofils mit einem Referenz-mRNA-Expressionsprofil, um den Stimulationszustand der natürlichen Abwehr in der Probe zu bestimmen oder zu bewerten;
(iv) positives Auswählen der Pflanze oder des Pflanzenteils, wenn der Vergleich in Schritt (iii) zeigt, dass die Pflanze oder der Pflanzenteil einen Stimulationszustand der natürlichen Abwehr besitzt, der ihr eine verbesserte Resistenz gegen wenigstens einen interessierenden biotischen und/oder abiotischen Stress verleihen kann.

15. Verfahren gemäß einem der Ansprüche 10 bis 14, **dadurch gekennzeichnet, dass** die Pflanze oder die Pflanzen zur Familie Rosaceae gehören.

## Claims

1. A device for determining or studying the natural defense stimulation state of plants or plant portions, which device includes means for determining the mRNAs expression level expressed by a combination of target genes in a sample of plants or plant portions, said determination means including:
(a) a means of determining the mRNAs expression level of at least one target gene chosen from among the following target genes: PR-1, PR-2, PR-4 PR-5, PR-8, PR-14, PR-15;
(b) a means of determining the mRNAs expression level of at least one target gene chosen from among the following target genes: PAL, CHS, DFR, ANS, PPO;
(c) a means of determining the mRNAs expression level of at least one target gene chosen from among the following target genes: HMGR, FPPS, Far;
(d) a means of determining the mRNAs expression level of the target gene CSL;
(e) a means of determining the mRNAs expression level of at least one target gene chosen from among the following genes: APOX, GST, POX;
(f) a means of determining the mRNAs expression level of at least one target gene chosen from among the following target genes: CalS, Pect, CAD;
(g) a means of determining the mRNAs expression level of at least one target gene chosen from among the following target genes: EDS1, WRKY;
(h) a means of determining the mRNAs expression level of at least one target gene chosen from among the following target genes: LOX2, JAR;
(i) a means of determining the mRNAs expression level of at least one target gene chosen from among the following target genes: ACCO, EIN3.

2. The device according to claim 1, **characterized in that** it includes the means for determining the mRNAs expression level of the following genes:
(i) for group (a), a means of determining the mRNAs expression level of at least one of the following genes: PR-1, PR-2, PR-4 and/or PR-8, a means of determining the mRNAs expression level of the gene PR-5, a means of determining the mRNAs expression level of the gene PR-14 and a means of determining the mRNAs expression level of the gene PR-15,
(ii) for group (b), a means of determining the mRNAs expression level of the gene PAL, a means of determining the mRNAs expression level of at least one of the following genes: CHS, DFR or ANS, and a means of determining the mRNAs expression level of the gene PPO,
(iii) for group (c), a means of determining the mRNAs expression level of at least one of the following genes: HMGR and Far, and a means of determining the mRNAs expression level of the gene FPPS, and
(iv) for group (e), a means of determining the mRNAs expression level of the gene APOX and a means of determining the mRNAs expression level of at least one of the following genes: GST and POX.

3. The device according claims 1 or 2, **characterized in that** it includes the means for determining the mRNAs expression level of the following combination of target genes: PR-1, PR-2, PR-4, PR-5, PR-8, PR-14, PR-15, PAL, CHS, DFR, ANS, PPO, HMGR, FPPS, Far, CSL, APOX, GST, POX, CalS, Pect, CAD, EDS1, WRKY, LOX2, JAR, ACCO, and EIN3.

4. The device according to any of the claims 1 to 3, **characterized in that** said means for determining the mRNAs expression level of a target gene are chosen from among nucleic acid fragments capable of hybridizing specifically with mRNAs expressed by said target gene or with corresponding cDNAs, or with fragments of said mRNAs or said cDNAs.

5. The device according to claim 4, **characterized in that** the nucleic acid fragments consist of nucleotide primers that hybridize specifically with the mRNAs, cDNAs or with fragments thereof.

6. The device according to claim 5, **characterized in that** said nucleotide primers are suitable for determining the mRNAs expression level of the target genes by means of a quantitative PCR method.

7. The device according to claim 6, **characterized in that** the primers are chosen from among the following sequences: SEQ ID Nos. 32 to 87.

8. The device according to claim 4, **characterized in that** said nucleic acid fragments or said antibodies are immobilized on a substrate.

9. The device according to claim 8, **characterized in that** it consists of a DNA chip.

10. A method for identifying an mRNAs expression profile for a combination of target genes making it possible to determine, or at least evaluate, a stimulation state of the natural defenses of plants or portions of plans, which method includes the following steps:
(i) determining the mRNAs expression profile of a combination of target genes by means of the device of any of the claims 1 to 9, for a set of plants or portions of plants, of which the stimulation state of the natural defenses thereof is known, and then
(ii) determining an mRNAs expression profile for said combination of target genes corresponding to a specific stimulation state of the natural defenses of said plants or portions of plants, using the data derived from step (i).

11. A method for determining or evaluating the stimulation state of the natural defenses of a plant or a plant portion, which includes the following steps:
(i) taking a sample from said plant or said plant portion,
(ii) determining the mRNAs expression profile of a combination of target genes in said sample taken in step (i), by means of the device according to any of the claims 1 to 9,
(iii) comparing the mRNAs expression profile obtained in step (ii) with a reference mRNAs expression profile, and
(iv) determining or evaluating the stimulation state of the natural defenses of said plant or said plant portion from said mRNAs expression profile obtained during step (ii).

12. A method for selecting a substance having the property of modulating the stimulation state of the natural defenses of a plant or plant portion, which includes the following steps:
(i) placing said plant or said plant portion in contact with the substance being tested,
(ii) determining the mRNAs expression profile of a combination of target genes in a sample taken from said plant or said plant portion subsequent to step (i), by means of the device according to any of the claims 1 to 9,
(iii) comparing the mRNAs expression profile obtained in step (ii) with a reference mRNAs expression profile, in order to determine or evaluate the stimulation state of the natural defenses in said sample, and
(iv) positively selecting said substance if the comparison in step (iii) shows that said substance tested in step (i) modulates the stimulation state of the natural defenses of said plant or said plant portion.

13. The method according to claim 12, for selecting a substance having the property of generating a natural defense stimulation state of a plant or plant portion belonging to the *Rosaceae* family, ensuring protection against a biotic stress, **characterized in that** the reference mRNAs expression profile in step (iii) corresponds to the values observed for samples of plants or plant portions not subjected to a stress or natural defense stimulator, and **in that** step (iv) consists in positively selecting said tested substance if the mRNAs expression profile obtained in step (ii), and compared in step (iii) shows an mRNAs overexpression of the following combination of target genes: PR-1, PR-2, PR-4, PR-5, PR-8, PR-14, HMGR, Far, CSL, POX, Pect, EDS1 and WRKY.

14. A method for selecting a plant having a natural defense stimulation state capable of giving same improved resistance to at least one biotic and/or abiotic stress of interest, which includes the following steps:
(i) applying said stress or stresses to a plant or plant portion,
(ii) determining the mRNAs expression profile of a combination of target genes in a sample taken from the plant or said plant portion, by means of the device according to any of the claims 1 to 9,
(iii) comparing the mRNAs expression profile obtained in step (ii) with a reference mRNAs expression profile, in order to determine or evaluate the stimulation state of the natural defenses in said sample, and
(iv) positively selecting said plant or said plant portion if the comparison of step (iii) shows that said plant or said plant portion possesses a natural defense stimulation state capable of giving same improved resistance to at least one biotic and/or abiotic stress of interest.

15. The method according to any of the claims 10 to 14, **characterized in that** the plant or plants belong to the Rosaceae family.


### SEQUENCE LISTING

<110> INSTITUT NATIONAL DE LA RECHERCHE AGRONOMIQUE 6 INRA
<120> Dispositif pour déterminer ou étudier l'état de stimulation des défenses naturelles de plantes ou parties de plantes
<130> W637FR
<160> 93
<170> PatentIn version 3.3
<210> 1
   <211> 304
   <212> DNA
   <213> Malus domestica
<400> 1
   tacctcaatt cccacaacgc cgctcgagca gcagtaggcg ttggtccctt gacgtgggat      60
   gacaatgtag caggctatgc acaaaactac gccaaccaac atgttggcga ctgcaatctc     120
   gtgcactccg gtgggccata cggtgaaaac cttgccatga gcactggtga catgtcggga     180
   gcagcggctg tggacctgtg ggtggcggag aaagccgact acagttatga gtcgaactcg     240
   tgtgctgctg gaaaggtgtg tgggcattat acacaggtgg tttggcgtaa ctcggctcgt     300
   gtag                                                                  304
<210> 2
   <211> 320
   <212> DNA
   <213> Malus domestica
<400> 2
   tatgctcttt tcacagctcc gtcagtttta gtacaagatg gccaacgtgg ttatcgtaat      60
   cttttcgatg ccattttgga tgctgtttac gctgcgcttg acaaggtcgg tggaggatct     120
   ttggaaattg ttatatcgga gagtggttgg ccgacagctg gtgggacggc aacaacagtt     180
   gataatgcga ggacatataa ctcgaatttg gttcaacatg tgaagggagg gactccaagg     240
   aagcctggaa ggcccattga aacttacatc tttgccatgt ttgatgagaa tagaaagacc     300
   ccagagcttg agaaacattg                                                 320
<210> 3
   <211> 609
   <212> DNA
   <213> Malus domestica
<400> 3
   aagccttcca agaacaacag agatattgta gattattgcc tgcttgattc ggaaaattac      60
   atatcacaac atggccggga agatcacagc aagctctgtg ttgtttgttt cgatcatgat     120
   ctgtggtttg gtcggcagtg cattgggaca aagtgcgacg aatgtgagag ccacatacca     180
   cctctacaat ccacagcaaa acaattacga cttgcgggca gtaagtgcct actgcgcaac     240
   atgggatgcc gataaatccc tcgaatggcg cagcaaatat ggatggactg ccttttgcgg 300
   acctgctggc cctactgggc aagccgcatg cggaaggtgc ctcttggtga caaacactcg 360
   gactggagct caagcaacgg tgagaattgt tgatcagtgc agcaacggag gattggactt 420
   ggacgtgaat gtttttaacc aaattgacac cgacggcaat ggttaccaac aaggccacct 480
   tatcgtcaac tacgactttg tcgactgcgg cgactaaagc ctgtaaacct taattttttc 540
   aatagctata tagttctcta cttccagtat tggtaaggaa cttaactatg tattactaat 600
   aaataagct 609
<210> 4
   <211> 583
   <212> DNA
   <213> Malus domestica
<400> 4
   actacaaatt ttggtaaagc ttagctccca caatgagcct ccttaaaagc ctcttagttt 60
   tctccatcct atgtgccatt ctcttctttg catcaaccaa tgcagctcga tttgacatcc 120
   aaaacaactg tgcctacact gtatgggcag ctgctgtgcc aggcggtggc cgccagctcg 180
   gcaaaggcca atcctgggcc ttagatgtga gtgctggtac caaaggggct cgcatttggg 240
   gccggacggg atgcaacttc gatggagcag gacgtggcaa atgccaaacg ggtgattgtg 300
   ggggtgtcct ccaatgccaa ggctatggtc aaccaccaaa caccctagct gaatatgccc 360
   ttaatcaatt taacaacttg gatttcattg acatctctct tgttgatggg tttaatgtac 420
   ctatggaatt tagtcccacg tctggtgggt gcactaggag gattaggtgc acggctgata 480
   ttaacgggca gtgccctaac caactgaggg ctccaggagg ctgcaacaac ccttgcactg 540
   tgttcaagac tgatcaatat tgttgcaatt ctggaaattg tgg 583
<210> 5
   <211> 1011
   <212> DNA
   <213> Malus domestica
<400> 5
   atggcatcca aaacacaaac cctagcccta actctgtccc tcttgatcct catttcttca     60
   tgcaagtcct cccaagccgc cggaatcgcg acgtattggg gccaaaacgg caacgaagga    120
   accctagcag aagcttgcaa ctcgggcaac taccagtttg ttaacatagc tttcctctca    180
   acttttggaa acaaccaagc ccctgtccta aacctcgccg gccactgcaa ccccgctagt    240
   ggtacttgca cggggcagag tgccgacatc agaacctgcc aatcaaaaaa cataaaagtc    300
   ctcctctcga ttggaggggc caccgaaact tactctctca cttcagctga tgaggcaagg    360
   caagttgctg attacatctg gaacaacttc ctaggtggtc agtcagattc gcgcccgctt    420
   ggggacgcgg ttttggacgg cgtcgatttc gacattgagt tgggtggtac gcagttctac    480
   gacgagctcg ccaggtcact caaaggacac aacggacagg gaaaaacggt ctatttagcc    540
   gcagctccac aatgcccgtt cccggatact cacctagacg gcgctatcca aaccggttta    600
   tttgactacg tttgggttca gttctacaac aaccccctag cccaatgcca gtatgcagac    660
   ggtaatgccg acgctctttt gagcagttgg aaccgatggg cctcggtttc ggccacccag    720
   gtgttcatgg ggttaccggc agctcctgag gccgctccga gcggcggatt tattcctgct    780
   gacgctctca agtcacaagt tcttccaacg attaagaatt cgcccaagta cggaggagtt    840
   atgctttgga gcaggttcta tgacaacggt tatagtgcat ccattaagga cagcatctaa    900
   ttaacttctc tgcctcaatg gatgctacta cttgtaataa agtgtgttag ggatgatggc    960
   gcctcattcc tttccttatg aataaaacat gcatatgtat tttttggagg c            1011
<210> 6
   <211> 588
   <212> DNA
   <213> Malus domestica
<400> 6
   aacaaaggca gctaaccatg gccaggtttt cggtcatcgt ggcaatagtg ttcttatttg 60
   tgatagctcc ttttgtcagt aatgccacca taacttgtgg tgaagtggta gcttggctca 120
   ctccatgcat accctttgga gtgtttggag gcacagtgcc tccagattgc tgtaaaggca 180
   taaaagagct gaatgctgca caaaacacca cgatggaccg gagaattgct tgtagttgca 240
   ttcaggaagg agctgcagca atccccggga ttaattatga ccggattaac actcttggtg 300
   atgtctgtgg ctctccttgt ccttacaaag tttacccctc tacaaattgc tctgcggtaa 360
   gctgaatcca taggaggatt ggagaatgga gaataaagct tccgagaaga ctattcctcg 420
   aagccatata gctagacaga cctcttagtt gtttaaatca caatgctttt gtggaactta 480
   ataatttgga agggtcgaat cgttctgtat aaggattgta cgtaatccat taccatctat 540
   taataattaa atgatcccct tttatacaaa aaaaaaaaaa aaaaatgc 588
<210> 7
   <211> 717
   <212> DNA
   <213> Malus domestica
<400> 7
   cgaattccgt tgctgtcgca cattaataca agttttgagg catcttaagt taagtttaaa 60
   ccctaattag ccatgaaagg tgctcagttc ctcgtatgca ttgttgccat cttggcattc 120
   gcaacctccc ttgtctctgc ctctgacccc agtcccctcc aggacttctg cgttgcactt 180
   aatgacacca aattaggggt gtttgtgaac gggaaatttt gcaaggaccc aaagcttgca 240
   tcagcaaatg atttcttctt ttctgggctt caaattccaa gaagcacaca aaatccgttg 300
   ggttcaacgg taacacctgc taatgtggat caaatagcag gactaaacac tctcggcata 360
   tccctagctc gcatagactt tgcaccaaat ggcctaaacc ctccccacac tcaccctcgc 420
   ggcacggaaa tcctttttct ccaggaaggt acactctacg ttgggttcgt aacgtccaac 480
   ccagataatc gtctattcac caaggtgttg aacaagggag atgtgtttgt gttcccagtt 540
   ggtcttattc acttccaact gaatgtggga cataccagtg ctgtcgccat tgctggtctt 600
   agcagccaga acccaggagt catcaccata gcaaatgcag tgttcggcgc caaccctccc 660
   atcaatcccg atgttctagc caaggccttc caagtcgatg acaaactggt ttcatat 717
<210> 8
   <211> 301
   <212> DNA
   <213> Malus domestica
<400> 8
   gaggcatttg gaggagaatt gaggaacacc gtaaagaaca cggtgagcca agtcgcaaag 60
   aggactttaa caactggggt aaatggggag cttcacccct caagattctg cgagaaggat 120
   ctcctcaaag ttgtcgatag ggagtatgtt tttgcctata ttgatgagcc ctgcagcgcc 180
   acttatccat tgatgcaaaa actgaggcaa gtgcttgttg agcatgcttt gacaaatggc 240
   gagagtgaga agaatgcaag cacttcaatc ttccaaaaaa ttggagcttt cgaggaagag 300
   c 301
<210> 9
   <211> 442
   <212> DNA
   <213> Malus domestica
<400> 9
   atcacggcgg tgaccttccg tgggcctagt gacacccacc ttgacagtct tgtgggtcaa 60
   gccttgtttg gcgacggtgc agcggccgtc atcattggtg ccgacccagt gcccgaagtc 120
   gagaagccct tgcttgaatt ggtctcggcg gcacaaacca ttctcgctga cagtgatggg 180
   gctatcgacg gacatctccg tgaagtaggg cttacgtttc accttttgaa ggacgttccc 240
   gggcttattt caaagaacat cgaaaagagc cttaacgagg ccttcaagcc tataggcatt 300
   tcggactgga actcactctt ctggattgca cacccaggtg gccctgctat tctggaccaa 360
   gtagaggcca agttggcgtt gaagccggag aaattagaag cgacaaggca agtgttgtca 420
   gattacggca acatgtcaag tg 442
<210> 10
   <211> 290
   <212> DNA
   <213> Malus domestica
<400> 10
   accggttgga tgtacttcgt ctcctttact ctagctgttc aagctgcatg gaaatatgcc 60
   aaagaaaaca acattgattt catcaccatt atcccaactc ttgtaattgg gccatttctc 120
   atgccatcca tgccaccaag cctcatcact ggactttcgc cgattttaag aaatgaatca 180
   cattatggca tcatcaagca gggccagtat gttcacttgg acgacctctg cctttctcac 240
   attcatctgt acaagcatcc aaaagccgag ggccgttaca tttgctcctc 290
<210> 11
   <211> 1074
   <212> DNA
   <213> Malus domestica
<400> 11
   atggtgagct ctgattcagt gaattcaagg gttgagacct tggccggcag tggaatctca      60
   accatcccaa aagagtacat cagacctaaa gatgagcttg taaacattgg tgacatcttc     120
   gaacaagaga agaacaacga agggcctcaa gttcccacca tcgatttgaa ggagatagag     180
   tctgataacg aaaaagtgag agcaaaatgc agggaggagt tgaagaaggc agctgtggac     240
   tggggtgtta tgcaccttgt gaaccatggc atctctgacg agctcatgga caaggtcagg     300
   aaggccggta aggccttctt tgaccttccc attgagctga aggagaagta tgccaatgac     360
   caggcctctg gtaagattca aggctatgga agcaagcttg caaacaatgc atctgggcag     420
   cttgagtggg aggactactt cttccactgt gtatacccag aggacaagcg tgacttgtct     480
   atttggcctc aaacacctgc tgattacatt gaggcaaccg ccgagtatgc taagcaactg     540
   agggagctag caaccaaggt actgaaagtt ctgtcacttg gcttgggatt ggatgaaggg     600
   aggctggaga aagaagttgg tggacttgaa gagctcctct tgcaaatgaa aatcaactac     660
   tacccaaaat gccctcagcc ggagcttgca cttggtgttg aagctcacac tgacgtgagt     720
   gcactcacct tcatcctcca caacatggtt cctggcctgc agcttttcta tgaaggaaag     780
   tgggtcactg ccaagtgcgt tccaaattcc atcgtcatgc acattgggga cacacttgag     840
   attttgagca atgggaagta caaaagtata ctccacaggg gcatggtgaa caaggaaaag     900
   gtgaggattt catgggcagt tttctgtgag ccaccaaagg agaagatcat ccttaagcca     960
   ctgccggaga ccgtgtccga ggaggagccg gcaatgttcc caccacgaac ttttgctgag    1020
   cacattcaac acaagttgtt caggaagagt caaggggctt tgctccccaa gtga          1074
<210> 12
   <211> 1993
   <212> DNA
   <213> Malus domestica
<400> 12
   aaaccagaga gctaagccat gacgtctctt tcacctccgg tagtcaccac ccccaccgtt      60
   cccaaccccg ccacaaaacc tctctccccc ttctctcaaa acaactccca agtttcccta     120
   ctcacaaagc ccaagcgttc ctttgcacgt aaggtctcat gcaaagccac aaacaatgac     180
   caaaatgatc aagcacagtc caaactagac aggagaaatg tgcttcttgg tcttggaggt     240
   ctatacggcg tggcgggtat gggcacagac ccgttcgctt ttgccaagcc tatagcccca     300
   ccagacgtat ctaaatgtgg tcctgcagac ttgccacagg gtgcagtgcc caccaactgc     360
   tgcccgccgc cttccacaaa aatcattgac tttaagctgc ctgcccccgc caaactccgc 420
   atcaggccac cggctcacgc cgttgaccaa gcctacaggg acaaatacta caaagcgatg 480
   gagctcatga aggccctacc cgacgacgac ccacgtagct tcaagcaaca ggcagccgtg 540
   cattgcgctt attgcgacgg cgcctatgac caagtcgggt tcccagaact cgagctccaa 600
   atccacaact catggctctt cttcccgttc caccgttact acttgtactt tttcgagaag 660
   atcctaggca aactcattaa cgacccgaca ttcgctttgc cgttctggaa ctgggactcg 720
   ccagccggca tgccactgcc cgcaatttac gctgatccaa agtcccctct ctacgacaag 780
   ctccgatctg ccaatcatca gcccccgact ctggtcgatc tcgattacaa cgggaccgag 840
   gacaatgtgt caaaggaaac cacaatcaac gccaatctca aaatcatgta caggcaaatg 900
   gtgtccaatt ccaagaatgc taagttgttc tttgggaacc cgtacagggc aggggacgag 960
   cctgaccctg gtggcggctc catcgagggg acaccacacg cgccggttca tttatggacc 1020
   ggtgacaaca cccagcccaa ctttgaggac atggggaatt tttactccgc tggtcgggac 1080
   cccatatttt ttgcacacca ttcgaatgtc gatcgaatgt ggagtatttg gaaaactctt 1140
   ggaggtaaga gaactgatct tactgactcg gactggttgg actccggatt cttgttttac 1200
   aacgagaacg cagagttagt ccgagtcaag gtcagggact gcttggagac caaaaatctt 1260
   gggtatgtat accaagatgt ggacattcct tggctcagct ccaagccaac accgcgaagg 1320
   gcgaaagttg cattgagcaa agtagcgaag aagctgggag ttgcacacgc agctgttgcg 1380
   tcgtccagca aggtggtggc aggcactgag ttcccgataa gtctggggtc gaagataagc 1440
   acggtggtga agagaccgaa gcagaagaag aggagcaaga aggccaagga ggatgaggag 1500
   gagatattgg tgattgaggg aatcgagttt gacagggacg tggctgtgaa gtttgatgtg 1560
   tatgtgaatg acgtcgatga cttgccgagt gggcctgaca agacggagtt tgccggaagc 1620
   tttgtaagtg tgccgcacag ccacaagcac aagaagaaga tgaacactat tttgaggtta 1680
   gggttgacag atttgttgga ggaaattgag gcggaggacg atgacagcgt ggtggtgact 1740
   ttggtgccca agttcggcgc tgtcaagatt ggtggtatca agattgaatt tgcttcttag 1800
   ttagttatgt caacccaggc atcaagaatc gtatgatcat catcatgctt ggatcgatca 1860
   tgtgttgtaa ttcttcaatt tggtggtttt gttccttttg aatagtcaat gttcgtgtac 1920
   aaaataaaca tgatgatgat gtggtatttt catcaccatg aaatatgaat taggaaatca 1980
   cattttgttc gtt 1993
<210> 13
   <211> 1827
   <212> DNA
   <213> Malus domestica
<400> 13
   atggacgtcc gaaggcgatc gacgatggat acacctgcca ccaaggccag aagtgggccg 60
   atgaaggtga aagtggtgga ccacgagaac gacgttggtg tcgtcggggc caaggcctcc 120
   gacgccctgc cgctgccgtt gtacctgact aacgccgtct gcttcactct cttcttctcc 180
   gtcgtctact tcctccttac tcgttggcgc gagaagatca agacctcgac gccgctccac 240
   gtcgtgaacc tctccgagat cgtcgcgata ctcgcgttcg tcgcctcctt catctacttg 300
   ctcggattct tcgggatcga tttcgtgcag tcgctcattc tccgccccag caatgacgtc 360
   tgggccgccg acgatgacga ggaggagcac gagcgcttga tattgaaaga cgacgcgcgg 420
   aaagtgccgt gtggggccgg actcgactgc agcccaattc cccaaattgc ctctgttgct 480
   gctgccgccc ccaaagctgt tgcacagaag gtgtttgata aagaggtagt cctctccact 540
   tcctgcgatt tcaccgccca gccgttgacg gaggaagatg aggaggtggt caagtccgtg 600
   gtggcgggaa ccatcccttc ctactctctg gagtcaaagc tcggagattg caggagggcg 660
   gcggctatca ggcgcgaggc gcttcagagg atcacaggaa agtctctggg tggtctgcca 720
   ttggaggggt tcgattacga gtcaattctg ggtcagtgct gcgagatgcc agttgggtat 780
   gttcagattc cagttgggat tgctgggcct cttatgctcc atggcagaga gttttccgta 840
   ccaatggcca ccaccgaagg ttgcttggtt gccagcacca accgtggctt caaagctatc 900
   aacttgtccg gcggagccac cagtgtgttg ctgagagatg ggatgaccag agcaccttgt 960
   gtgaggttca actctgctaa gagagctgcc gagttgaagt tctacttgga agaacccaac 1020
   aattatgaca ccttgtccac ggttttcaac aggtcaagca gattcggtag gcttcagaca 1080
   attaagtgtg ccattgctgg gaagaacttg tacatgagat tcacctgcag caccggtgat 1140
   gctatgggga tgaacatggt ctccaaaggt gtgcaaaacg tcttggattt cctccagaac 1200
   gacttccctg acatggatgt gattggaatt tccggcaact actgctctga caagaagccc 1260
   gctgcggtga actggattga aggccgcgga aaatcggtgg tctgtgaggc tgtgatcaag 1320
   ggtgatgtgg tgcagaaggt gttgaaaacc aatgtggcgt ccctgtgcga gcttaacatg 1380
   cttaagaacc ttactgggtc tgcaatggct ggagccctcg gtggattcaa cgcacatgcc 1440
   agcaacatcg tctccgccat ctacatcgct accggccaag acccagctca gaatgtggag 1500
   agttctcact gcattaccat gatggaaccc atcaatgatg gacaggacct tcacgtgtct 1560
   gtcaccatgc cttcaattga ggttggtact gttggaggtg ggacccaact tgcatctcaa 1620
   tcagcttgtc tgaaccttct tggagtgaag ggtgctaaca gggaggcacc aggatcaaat 1680
   gcaagattgt tggccactgt tgtggctggt tctgttcttg ctggagagct ttctctcatg 1740
   tctgctatct cagctggaca gcttatgaat agtcacatga aatactacag atcaagcaaa 1800
   gatgtctcag ctgttgcatc cgcttaa 1827
<210> 14
   <211> 1401
   <212> DNA
   <213> Malus domestica
<400> 14
   atttttttcc ctctgtacca ttaagtcgtg tcgtctatac aaagcacatc tctctccctc      60
   tctgtgagtc tgtgagtatc tggggagggc tctgagatta ccagtgtctg tgtggtggcg     120
   gattagattg gcaaaactga caatggcgga tctcaagtca aagtttctga aggtgtactc     180
   cgttctgaaa tcggagctgt tggaagaccc tgctttcgac ttcaccaatg actctcgcca     240
   atgggttgag cggatgctgg actacaatgt gcctggaggg aagcttaatc ggggattgtc     300
   tgttattgac agctatcagt tgttgcaaca aggaagggaa ttaactgaag atgaaatctt     360
   cctggccagc gctctcggtt ggtgcatcga atggcttcaa gcattttttc tggttcttga     420
   tgacatcatg gatggctctc acacacgtcg tggtcagcct tgctggttta gattgcccaa     480
   ggttggtatg attgcagtaa atgatggtgt tgtgcttcga aaccatatcc caaggattct     540
   cagaaagtac ttcagagaaa agccatatta cgtggatctt cttgatttgt tcaatgaggt     600
   ggaatttcaa actgcctcag gtcagatgat agatttgatc actactatcg aaggagaaaa     660
   agatctatcc aaatactcat tgtcaattca ccgccgtatt gttcagtaca agactgccta     720
   ttactcattt tacctttccg ttgcatgtgc attgcttatg tcaggtgagg aactggaaaa     780
   acatattgat gtaaaaaaca ttcttgttga gatggggatc tactttcaag tacaggatga     840
   ttatttggat tgctttggtg atccggaaac gattggtaag ataggaacag atattgaaga     900
   tttcaagtgc tcttggttgg tggtgaaagc tttggaactc tgcaatgagg aacaaaagaa     960
   agtactacat gagaattatg ggaaaccaga cccagaaaat gtggcaacag taaaggccct    1020
   ctacaaagaa ctcgatattg agggtgtatt tgcggattat gagagcaaaa gctacaagaa    1080
   actgacgagt tggattgaag ggcacccaag caaagcggtg caatcagtgt tgaagtcctt    1140
   cttgggcaag atttacaaga ggcagaaata gaaatctcgg atctggattc agttcagccc    1200
   ctcctatttg tatttggtgc atttgtaata aaacatgcag ttgcatttgg tttgcgtcag    1260
   tcagagtcag gtttgcttca cttagttata agttgagtca tcagtgttga atgcagacag    1320
   acaataagca tccacgttaa ttaatacaaa gtttctcagt ttccagttga gaatattaaa    1380
   aaaaaaaaaa aaaaaaaaaa a                                               1401
<210> 15
   <211> 562
   <212 > DNA
   <213> Malus domestica
<400> 15
   cagcattgca gctatcgaaa gcgacaatct cggcacaaga gacgatctct atggtactgc 60
   attacacttc aagatcctca ggcagcatgg ctataaagtt tcacaagata tatttggtag 120
   attcatggat gaaaagggca cattagagaa ccaccatttc gcgcatttaa aaggaatgct 180
   ggaacttttc gaggcctcaa acctgggttt cgaaggtgaa gatattttag atgaggcgaa 240
   agcttccttg acgctagctc tcagagatag tggtcatatt tgttatccag acagtaacct 300
   ttccagggac gtagttcatt ccctggagct tccatcacac cggagagtgc agtggtttga 360
   tgtcaaatgg caaatcaacg cctatgaaaa agacatttgt cgcgtgaacg ccacgttact 420
   cgaattagca aagcttaatt tgaacgtaag tgaggcccaa ctgcaaaaaa acttaaggga 480
   agcatgcagg tggtgggcaa atctgggctt gggagacaac ttgaaatttg caagagatag 540
   actgggtgaa tgttgctgat gt 562
<210> 16
   <211> 198
   <212> DNA
   <213> Malus domestica
<400> 16
   ggatacgaca ctatgaagaa tcgctgggaa aaattgacca acaccctgtc tgtttccaat 60
   cgcttttctc tacagaaaat tgaaccccaa tattgcactt actttcagaa aattagagaa 120
   ccctcaccag cttatgcgtg ggtgaagtgc gagagggaag aagataaaga ttgctacaaa 180
   atactggaag aagcaaaa 198
<210> 17
   <211> 625
   <212> DNA
   <213> Malus domestica
<400> 17
   ggatgccccc gtgccaccac cagaggccgt cttcctgatg ctaccaaggg taatgaccat 60
   ttgagggatg tcttttgcaa gaccatgggc ctcagcgaca aggatattgt tactctctcc 120
   ggtggtcaca ccctgggaag gtgccacaag gagcgatctg gatttgaagg accttggact 180
   cccaaccccc ttatctttga caactcctac ttcaaggtgc ttcttggtgg agaccaggaa 240
   ggtcttctaa tgcttccaac tgacaaggct cttctggatg accctgtctt ccgccctctt 300
   gtggaaaaat atgctgcgga tgaagatgct ttctttgctg actatgctga atctcacatg 360
   aagctctccg agcttgggtt tgctgaggcc taagcagagc tggagaacta caagggatga 420
   agccgatgcc tgtgcgcctt gcttttgtat ttttggatgc ctcttggttg tgaggttgta 480
   ggcagttggt gcttttcttt tttatctatc agattttagg aaagtgggac ttctagttcg 540
   atttgaagaa cgaatgtttt taaactggat ggaatgctgg ttatcatcct cgttgattaa 600
   ataatactgt aattttcaaa aaaaa 625
<210> 18
   <211> 650
   <212> DNA
   <213> Malus domestica
<400> 18
   ggctgccatc aaggtccacg gaaacgttat ttcgaccgct gcaatgcgag tttttgctac 60
   tctctacgag aaagacatcg aattcgagct tgttccaatc gacatgagag ctggtgaaca 120
   caaaaaggag cccttcatat ccctcaatcc atttggtcaa gttccagcat tcgaagacgg 180
   agatcttaag ctctttgaat caagggcgat tacacaatac attgcccacg agtatgctga 240
   caagggaacc cctctagtga tccgagactc aaagaagatg gcaattatat cattgtggtc 300
   ggaggtggag gcccaaaagt tcgacccggc ggccacaaaa ctgacctatg agctagctat 360
   aaagcctatg tttaaaatga ccacagacgc ggcagttgtg gaggaaaatg aagccaagtt 420
   ggctgtggtt cttgatgtat atgagactcg tctggctcag tcgaaatact tggcaggtga 480
   aagcttcact ttggctgatc ttcaccacct ccccaccata cattacttga tgggaacaca 540
   atcgaagaag ctgttcgaat cccgcccccc atgttctcgc atgggtggcg gatatcacag 600
   caaggccagc ttggaacaaa agtcgttgcc cagcaaaagt gaaaacaagt 650
<210> 19
   <211> 503
   <212> DNA
   <213> Malus domestica
<400> 19
   cttcttcttc tctctttgct tcttagttat attggttatc agaaaccttg ttagatatta 60
   gtcatgggtt catatattac tggttcatgc actcccacaa acactaaaat ctcattgtca 120
   ttattattcc ttcttgttgg agtagcttct gctcaattgt cctctacttt ctatggaaca 180
   tcgtgtccta atgccctgtc caccataaaa tcggcagtgg actcagcggt gtcgaaggaa 240
   gctcgcatgg gagcttcctt gcttcgtctt catttccatg attgctttgt taatggatgt 300
   gatgcttcgg ttctgctgga tgacacagcc aatttcacag gagagaaaac agcaggtgct 360
   aatgctaatt ctttgagggg atttgatgta atcgatacaa ttaaatctca attggagagt 420
   ctctgcccca aagtagtctc ttgtgctgac atcttaactg ttgctgctag ggactctatt 480
   gttgcattgg gtggacttac cta 503
<210> 20
   <211> 562
   <212> DNA
   <213> Malus domestica
<220>
   <221> misc_feature
   <222> (472)..(472)
   <223> n is a, c, g, or t
<400> 20
   gatattatta gctttgcgct ttttcattta ccagtatggg ctagtctatc accttaacat 60
   cacaaacaac aaaagtttcc tggtatatgg tgtttcgtgg ctcgtgatca ttctagtttt 120
   gtgtctgatg aaggctgtgt ctgctggaag gcgacgatta agtgcagact atcagctttt 180
   gtttcgactg gttaagggat ttatatttat tacattttta gccatcttca ttaccttaat 240
   agtggtccct catatgacac ttcgggatgt tgtagtgtgc attcttgcct tcatgccaac 300
   gggatgggga ttgcttttga ttgcgcaagc ttgtaagcca ctaattaaac gagcagggtt 360
   ttgggaatca gttcaaacac ttgctcgcgg ttatgaaata atcattggat tgcttctttt 420
   taccccggtt gcattcttgg cttggtttcc gtttgtttcc gaattccaaa cncgaatgct 480
   cttcaaccaa gcattcagca gaggtttaca gatctcacgt attcttggag gacaaaagaa 540
   gggctctcac tcctccaaca aa 562
<210> 21
   <211> 580
   <212> DNA
   <213> Malus domestica
<400> 21
   gcttactaat caacagacat gccaaaatgg tttcaaaaac ctcaacattc ctagttctca 60
   tttgcaatct ttcccaaact acgtcacgct ctcaaccaac ttgtccaaga tgcttagcaa 120
   ctcactatca atacacaagg tctcaacttc ttcatcagct ctacatttta gcaaacaagt 180
   tcgtggaaga cgacggcgtt tgctttcgga tgatgggttc ccagaatggg tttcagccgc 240
   tgatcgaaag ctgctacaat cgataggtag cggaccaaac gctgatatgg tagtagcaca 300
   agacgggtcc gggaattaca agtcaatttc tgaagctgtg gatgctgcgt acaagctaca 360
   gggtggggcg actaaaaggt ttgtcataca cgtgaaggca ggagtttaca gagaaaatat 420
   cgagattaag aggacaatga agaacatcat gttcattgga gatggtatcg atgcaacgat 480
   tgtcacaggg aataaaaatg cccaagatgg ttcaactaca tatcgctctg ccacatttgg 540
   ggctaccgga gatggcttta ttgctcaaaa cttgaaattc 580
<210> 22
   <211> 1262
   <212> DNA
   <213> Malus domestica
<400> 22
   ggcacgagga gagagatgag cagcggagca gggaaggtcg tgtgcgtcac cggtgcgtct 60
   ggatacattg cttcatggct cgttaagctt cttctccagc gcggctacac cgtcaaggcc 120
   tctattcgcg acccaaatga tccaactaag acagagcacc tgcatgcgct tgatggagcc 180
   caagacagac ttcaactttt caaagcaaat ctgttggaag aaggttcttt tgactctgct 240
   gttgagggtt gtgaaggcgt attccatact gcatctccct tttatcatga tgtcacagac 300
   ccaaaggcag aactacttga acctgcggta aaggggaccc ttaatgttct taattcgtgt 360
   gcgaaatcac catcaatcaa acgggtggtt ttgacatctt ctatagccgc tgtagcatat 420
   aatggaaagc ctcgaactcc tgatgtagta gttgatgaga cttggtttac agatccagat 480
   gtttgtaagg aatcaaagct ttggtacgtg ctttcaaaga ctttggctga agatgctgcg 540
   tggaaatttg taaaggagaa gggtattgac ttggttacaa ttaaccccgc aatggtgata 600
   ggtcctctgt tacagccaac tcttaataca agtgctgcag cagttttaaa tgttattaag 660
   ggagctcgaa cttttccaaa tgcaagtttt ggatggatta acgttaaaga tgttgccaat 720
   gcacatattc aagcatttga gaggcctacc gctagtggca gatattgttt ggttgagaga 780
   gttgcccact tctcagaagt tgtgagaatt ttacgtgagc tgtaccctac tttgcaactt 840
   ccagagaagt gtgcagacga caagcctttt gtgccaacat atcaggtgtc caaggagaag 900
   gcgaagagct tgggtgtcga atttattcca ctagatgtta gcctcaagga aacagttgaa 960
   agtctgaagg aaaagggttt tgtcaatttc tgagtcattt cagtacggtg aagatgtttg 1020
   aagtaaattc agtcgcggta aaacatatta tctgcttcca gtttgtgttt ccggttggtg 1080
   acttaaactc cggtttccct ttttcggttt aagacttgct gtccagcttg ttgattccta 1140
   tagatcacca ttgtgtgtgc agaataaaga agaaatgaac cttttgaata aaatcttgga 1200
   atcgtctgag taaagtgcgc tcatgcaact ttatccccat tgataaaaaa aaaaaaaaaa 1260
   aa 1262
<210> 23
   <211> 637
   <212> DNA
   <213> Malus domestica
<220>
   <221> misc_feature
   <222> (585)..(585)
   <223> n is a, c, g, or t
<400> 23
   aaaaagggaa tgaaagcttg gagagatgct ggatataggg gaggagacta tcaagaaaag 60
   ctgctctctt gccttggagg cacacaaatt cccaggtgaa ccgtttcttg tggagaactc 120
   tgctgcagga tcatcagatg ttgttatcag ctttcctgca ggaacttggt ctgtggagag 180
   tttgttttct ggagaaagtg attttggaga agcaaaagtc gatctcgaac tctttccttc 240
   cttgaaaagt gttgggaact atgatccagc aaagccagaa gagatagact ttgcaattgc 300
   aactatcaac caagcattcg ttgacaaagt taaacaaata ttggacaatt cccagctgaa 360
   aatcaaggtg caagaggcca tcactgcaaa gaagagaata atatttaccg gccactctac 420
   cggcggtgca attgctgcgt ttgtcacaat ctggttctta gaaaattgct cgaaagaaat 480
   cagtggatct ttcttctgtg tgactttcgg atctcccctt attggtgacc acattatttc 540
   tcatgctctt aggagagaaa attggtctga atacttcata cattntgtca tgagatatga 600
   cattgtccct cggattttgc ttgctcctct gtcatcc 637
<210> 24
   <211> 342
   <212> DNA
   <213> Malus domestica
<400> 24
   caggtaactt cgttcctata tctcatgtgc tgctactgac aaatggagaa ctgcaatata 60
   cattgggagc aaaagagtct tgtaaatgag ctagcacaag ggagggatct ggctaggcag 120
   ctacagatcc atctcaacgt tccatcttcc tcttatggaa cccgggaatt gctggttcaa 180
   aagatcatac tttcgtacga aaaagcgctt tccatgctga actctagcag ctcagcctca 240
   gaaggtgagc aacaacagcc cacaggtcat gttgcaattc gaatggttga gtctccgccg 300
   cattcgctaa atgaaagtcc ccggagtgaa gactccgacc gc 342
<210> 25
   <211> 648
   <212> DNA
   <213> Malus domestica
<400> 25
   ggaggcttgc taaagctcat gtccttgccc acgattctgg ttatcatcag cttgttagtc 60
   actggctacg aacacattgt gccacagaac cctacataat cgccacaaat cggcaactga 120
   gtgtcatgca cccaatctat agattgttac atccccattt cagatacacc atggagatta 180
   attctcttgc tcgtgaatca cttatcaatg ccggtggtat catcgaaacc tcattttcgc 240
   ccaaaaagta ctcccttgag ctctgctctg ttgcgtatgg gaaggaatgg cggtttgacc 300
   aagaggcact cccagctgac cttattagaa ggggcatggc tgttgaggac ccaactgctc 360
   cacatgggct aaggctaaca attgaagatt acccttttgc caatgatgga ctcctcttat 420
   gggatgctat taaacaatgg gtcactgact atgtaaacca ctactaccca gactccagca 480
   ttgtccagac tgatcaagag ctccaagcat ggtggacaga aatcaaaaca gttggccatg 540
   ctgacaaaaa agatgagcca tggtggccag aactcaacac tccggaagac ctaatgggca 600
   tcatcacaac aatggtttgg gtagcatctg gtcatcatgc agctgtca 648
<210> 26
   <211> 630
   <212> DNA
   <213> Malus domestica
<400> 26
   tttttttttt ttagataaag ttaaacttta tttctgatcc aaactcatga ttttgacatg      60
   taacttatct gatccggaag aatttaaccc tccatatccg tttgccaaat acaataatgt    120
   tgctaattag catatgcagt gctgtaaacc cttttagtgg tgcataaatt gagaatgctc    180
   aagagcacct tgttgctagt gcacctaggg gtcttgaatt ggctcaatgc cgatccattt    240
   cctatgaaat attccaaaat tttcttgaac gttcccttct ccacaatccg aagctctaga    300
   ggtccgatcg aattacttcg cctcgagaca acgtaaccat gatccacgaa agatgcatcc    360
   atttctctgc aacattcacc aagaaccctc tcttcaactt ctcctttgat ctcccagtat    420
   attatgtagt ggcctggttg ggtcagcaca tcagcatggc ttgtgaagtc aatcagttct    480
   gctttggact tgctcaatag ttgagaccct ctctccacca ccatttgaag gtccttttca    540
   gtgttttcac acaactctct gcatttctgc tctgcccacc cactaaatca cttgacacac    600
   ccacaccggc aatggacctc ctcctcctgg                                     630
<210> 27
   <211> 1214
   <212> DNA
   <213> Malus domestica
<220>
   <221> misc_feature
   <222> (999)..(999)
   <223> n is a, c, g, or t
<400> 27
   ccaaacactt gtagagcgag agagagagag agagagagag agagagagaa aatggagaac      60
   ttcccagtta tcaacttgga gagcctcaat ggtgagggaa gaaaagctac aatggaaaag     120
   atcaaagatg cctgtgagaa ctggggtttt tttgagctgg tgagtcatgg gattccaact     180
   gagtttctgg acacagtgga gaggctgaca aaagagcact acaagcagtg tttggagcaa     240
   aggttcaagg agctggtggc cagcaaaggc cttgagggtg ttcagacaga agtcaaagat     300
   atggattggg aaagcacttt ccacttgcgc catcttcctc aatcgaacat ctctgaagta     360
   ccagatctca aggatgagta caggaatgtg atgaaggagt ttgcattgaa attggaaaaa     420
   ttagcagagc agctgctgga cttgttgtgt gagaatcttg gactggaaca agggtacctt     480
   aagaaggcat tttatggaac aaagggacca acttttggca ccaaggtgag caactaccct    540
   ccatgtccca acccagacct gatcaagggt ctccgggccc acaccgatgc cggcggcctc     600
   atcttgctct tccaggatga caaggtcagt ggcctccagc tcctcaagga cggagagtgg     660
   gttgatgtgc ctcccatgcg ccactccatt gttatcaatc ttggtgacca acttgaggtg     720
   atcaccaacg gaaagtacaa gagtgtggaa cacagggtga ttgcccaaac agatggcacc     780
   agaatgtcaa tagcttcatt ctacaaccca ggcagtgatg cggtgatcta cccagcacca     840
   accctagtgg agaaagaagc agaggagaag aatcaagtgt acccgaaatc cgtgttcgaa     900
   gactacatga agctctatgc tggggtcaag ttcgaggcca aggaaccaag atttgaagcc     960
   atgaaagcag tggaaattaa ggccagtttt ggtttgggnc cagttataag tactgcttga    1020
   gagtgattaa atacttttac tagaagctgt tggaaaaaag ggttgtttgc ttaaaagtaa    1080
   ttatgggtgt gtgaacgaaa tttttccttg aactagattt caaggtttta tttactatta    1140
   atgtggaaac cgttgtagaa agtaaccaat gtgatcacta ctttattata tataatatga    1200
   cttcaagatt tgtt                                                      1214
<210> 28
   <211> 616
   <212> DNA
   <213> Malus domestica
<400> 28
   cacttcttga gagcttggag gaagggcttg tcaaagtgaa tcttaacgga tgcttgaatt     60
   tgacagatga agtcgttatg gcgttggtta ggctacatgg ggaaacttta gaagtactga    120
   atcttgatgg ttgcagaaag attactgatg caagcttggc gacaatcgca aacaactgct    180
   tgtttcttcg tgagctagat gtgtcgaagt gtgcgattac agattctggc cttgctgccc    240
   tttcttccgc agatcagatc aacttgcaag tcctctccat ttctggctgt tctgaaatct    300
   cacacaaaag cctcccttcc ctgaaaaaat tgggccagac cctggtgggg ttgaatctcc    360
   aacattgtac tgcactcagc tacagatcaa ttgagctact tgtggagagc ttgtggagat    420
   gcgatatctt ggcctaataa ggaatagaac aatacgcgta tatagcgaag gagagaagca    480
   gatatgaagt aggggtaaga tcaagaaatc aaaagaattc gggttagatt cgtcagttcg    540
   tagatgttaa gcactcacac gggtatccac gttcagcata caacattttt ggcccgttgg    600
   tagtggcctt gctggt                                                    616
<210> 29
   <211> 596
   <212> DNA
   <213> Malus domestica
<400> 29
   tgttcaatgc tgttggtggt ggtactggtt ctggtttggg gtccttgctc ttagaacgct     60
   tgtcggtgga ttatggaaag aagtcaaagc ttgggttcac catctatcct tctccgcagg    120
   tttcaacagc agttgttgag ccttataata gtgttctctc tactcatgcc ctccttgaac    180
   acacagatgt atctgtgctc ttggacaatg aagctatcta tgatatttgc aggagatccc    240
   tagacatcga gagaccaact tacaccaatt tgaatagact gatatcacaa gtcatatcct    300
   ccttgacaac atcattgaga tttgatggag ccattaatgt tgatgtgaca gagttccaga    360
   caaaccttgt tccatatccc cgcatccatt tcatgctttc ctcatatgcc cctgttatct    420
   cagctgctaa ggcataccat gagcagatct caatccctga gatcacaaat gcagtttttg    480
   aaccctcaag catgatggcc aagtgtgatc cccggcatgg aaaatacatg gcctgctgcc    540
   taatgtacag gggtgatgta gttcccaaag atgtcaatgc tgctgttgca actatc        596
<210> 30
   <211> 703
   <212> DNA
   <213> Malus domestica
<400> 30
   accataacaa atggcagata cagaagacat tcaacctctc gtctgtgata atggcactgg     60
   aatggttaag gctggatttg ctggagatga tgctccgagg gctgttttcc ccagtattgt    120
   tggtcgtcct agacacacag gtgttatggt ggggatggga cagaaggatg cctatgttgg 180
   ggatgaggcc cagtcgaaaa ggggtattct cacattaaaa taccccattg aacacgggat 240
   tgttagcaat tgggatgaca tggaaaagat ctggcatcat actttctaca atgagcttcg 300
   tgtcgctcct gaagaacatc ctgtgcttct tactgaagca cctctgaatc ctaaggccaa 360
   cagggaaaag atgactcaga tcatgtttga gaccttcaat gtccctgcta tgtatgttgc 420
   cattcaagca gttctctccc tctatgctag tgggcgtact acaggtattg tgcttgactc 480
   tggtgatgga gtcagtcaca ctgttcccat ctacgagggt tatgcacttc cacatgccat 540
   cctccggctt gaccttgctg gacgagactt aacagattcc ttaatgaaga tcctgactga 600
   gagagggtat acttttacta ctactgctga acggggaatt gtgcgtgaca ttaaagagaa 660
   gcttgcctat gtggcccttg cttatgaaag ggaatcggaa tcg 703
<210> 31
   <211> 545
   <212> DNA
   <213> Malus domestica
<400> 31
   acaggttcgg aattgttgag ggtctcatga ccacggtgca ctccatcact gccacccaaa 60
   agactgttga cggtccatca atgaaggact ggagaggtgg acgtgcagct tccttcaaca 120
   tcattcctag tagcactgga gctgccaagg ctgttggaaa ggtgctccca tctcttaatg 180
   gaaaattgac cggaatgtcc ttccgtgtgc ccactgttga tgtttccgtt gttgacctga 240
   ctgtcaagct tgagaaggct gcaacctatg aacagatcaa ggccgctatc aaggaggagt 300
   ctgaaggcaa gttgaagggt atcttgggtt acaccgaaga tgatgtcgtg tccaccgact 360
   tcattggtga cagcaggtca agcatctttg atgccaaggc tggaattgca ttgaatgaca 420
   actttgtcaa gcttgtctca tggtacgaca acgagtgggg ttacagttcc cgtgtggtag 480
   acttgatcgt gcacgtagca tcgagtctct agggttttta ggttatggtt tgggagttca 540
   caata 545
<210> 32
   <211> 22
   <212> DNA
   <213> artificial sequence
<220>
   <223> amorce
<400> 32
   agcacacgag ttcgactcat aa 22
<210> 33
   <211> 20
   <212> DNA
   <213> artificial sequence
<220>
   <223> amorce
<400> 33
   cacaaaacta cgccaaccaa 20
<210> 34
   <211> 20
   <212> DNA
   <213> artificial sequence
<220>
   <223> amorce
<400> 34
   aattgttata tcggagagtg 20
<210> 35
   <211> 19
   <212> DNA
   <213> artificial sequence
<220>
   <223> amorce
<400> 35
   tggcaaagat gtaagtttc 19
<210> 36
   <211> 17
   <212> DNA
   <213> artificial sequence
<220>
   <223> amorce
<400> 36
   gaaggtgcct cttggtg 17
<210> 37
   <211> 17
   <212> DNA
   <213> artificial sequence
<220>
   <223> amorce
<400> 37
   cgtcggtgtc aatttgg 17
<210> 38
   <211> 18
   <212> DNA
   <213> artificial sequence
<220>
   <223> amorce
<400> 38
   aaaggggctc gcatttgg 18
<210> 39
   <211> 19
   <212> DNA
   <213> artificial sequence
<220>
   <223> amorce
<400> 39
   cttggcattg gaggacacc 19
<210> 40
   <211> 22
   <212> DNA
   <213> artificial sequence
<220>
   <223> amorce
<400> 40
   ccaagcccct gtcctaaacc tc 22
<210> 41
   <211> 22
   <212> DNA
   <213> artificial sequence
<220>
   <223> amorce
<400> 41
   caacttgcct tgcctcatca gc 22
<210> 42
   <211> 21
   <212> DNA
   <213> artificial sequence
<220>
   <223> amorce
<400> 42
   agtgttctta tttgtgatag c 21
<210> 43
   <211> 19
   <212> DNA
   <213> artificial sequence
<220>
   <223> amorce
<400> 43
   caactacaag caattctcc 19
<210> 44
   <211> 21
   <212> DNA
   <213> artificial sequence
<220>
   <223> amorce
<400> 44
   gcctaaaccc tccccacact c 21
<210> 45
   <211> 24
   <212> DNA
   <213> artificial sequence
<220>
   <223> amorce
<400> 45
   catctccctt gttcaacacc ttgg 24
<210> 46
   <211> 20
   <212> DNA
   <213> artificial sequence
<220>
   <223> amorce
<400> 46
   ggatctcctc aaagttgtcg 20
<210> 47
   <211> 19
   <212> DNA
   <213> artificial sequence
<220>
   <223> amorce
<400> 47
   ctcactctcg ccatttgtc 19
<210> 48
   <211> 19
   <212> DNA
   <213> artificial sequence
<220>
   <223> amorce
<400> 48
   attggtgccg acccagtgc 19
<210> 49
   <211> 25
   <212> DNA
   <213> artificial sequence
<220>
   <223> amorce
<400> 49
   aatccagaag agtgagttcc agtcc 25
<210> 50
   <211> 17
   <212> DNA
   <213> artificial sequence
<220>
   <223> amorce
<400> 50
   cggttggatg tacttcg 17
<210> 51
   <211> 17
   <212> DNA
   <213> artificial sequence
<220>
   <223> amorce
<400> 51
   tgtgagaaag gcagagg 17
<210> 52
   <211> 20
   <212> DNA
   <213> artificial sequence
<220>
   <223> amorce
<400> 52
   gcctcaagtt cccaccatcg 20
<210> 53
   <211> 22
   <212> DNA
   <213> artificial sequence
<220>
   <223> amorce
<400> 53
   gaagtagtcc tcccactcaa gc 22
<210> 54
   <211> 17
   <212> DNA
   <213> artificial sequence
<220>
   <223> amorce
<400> 54
   tgcccgccgc cttccac 17
<210> 55
   <211> 25
   <212> DNA
   <213> artificial sequence
<220>
   <223> amorce
<400> 55
   gctccatcgc tttgtagtat ttgtc 25
<210> 56
   <211> 18
   <212> DNA
   <213> artificial sequence
<220>
   <223> amorce
<400> 56
   aacaggtcaa gcagattc 18
<210> 57
   <211> 18
   <212> DNA
   <213> artificial sequence
<220>
   <223> amorce
<400> 57
   ttgtcagagc agtagttg 18
<210> 58
   <211> 19
   <212> DNA
   <213> artificial sequence
<220>
   <223> amorce
<400> 58
   tcttggttgg tggtgaaag 19
<210> 59
   <211> 20
   <212> DNA
   <213> artificial sequence
<220>
   <223> amorce
<400> 59
   ctgcctcttg taaatcttgc 20
<210> 60
   <211> 23
   <212> DNA
   <213> artificial sequence
<220>
   <223> amorce
<400> 60
   gatattttag atgaggcgaa agc 23
<210> 61
   <211> 20
   <212> DNA
   <213> artificial sequence
<220>
   <223> amorce
<400> 61
   gcgttgattt gccatttgac 20
<210> 62
   <211> 21
   <212> DNA
   <213> artificial sequence
<220>
   <223> amorce
<400> 62
   caccctgtct gtttccaatc g 21
<210> 63
   <211> 25
   <212> DNA
   <213> artificial sequence
<220>
   <223> amorce
<400> 63
   agcaatcttt atcttcttcc ctctc 25
<210> 64
   <211> 23
   <212> DNA
   <213> artificial sequence
<220>
   <223> amorce
<400> 64
   gcttccaact gacaaggctc ttc 23
<210> 65
   <211> 18
   <212> DNA
   <213> artificial sequence
<220>
   <223> amorce
<400> 65
   cgcacaggca tcggcttc 18
<210> 66
   <211> 23
   <212> DNA
   <213> artificial sequence
<220>
   <223> amorce
<400> 66
   gagcccttca tatccctcaa tcc 23
<210> 67
   <211> 18
   <212> DNA
   <213> artificial sequence
<220>
   <223> amorce
<400> 67
   gcctccacct ccgaccac 18
<210> 68
   <211> 24
   <212> DNA
   <213> artificial sequence
<220>
   <223> amorce
<400> 68
   tgtcattatt attccttctt gttg 24
<210> 69
   <211> 18
   <212> DNA
   <213> artificial sequence
<220>
   <223> amorce
<400> 69
   aaccgaagca tcacatcc 18
<210> 70
   <211> 21
   <212> DNA
   <213> artificial sequence
<220>
   <223> amorce
<400> 70
   tgccttcatg ccaacgggat g 21
<210> 71
   <211> 23
   <212> DNA
   <213> artificial sequence
<220>
   <223> amorce
<400> 71
   cggaaacaaa cggaaaccaa gcc 23
<210> 72
   <211> 18
   <212> DNA
   <213> artificial sequence
<220>
   <223> amorce
<400> 72
   ggagggaggg tcaactgg 18
<210> 73
   <211> 22
   <212> DNA
   <213> artificial sequence
<220>
   <223> amorce
<400> 73
   caaacaactt ctctccacaa cc 22
<210> 74
   <211> 22
   <212> DNA
   <213> artificial sequence
<220>
   <223> amorce
<400> 74
   ggagcccaag acagacttca ac 22
<210> 75
   <211> 21
   <212> DNA
   <213> artificial sequence
<220>
   <223> amorce
<400> 75
   cgccttcaca accctcaaca g 21
<210> 76
   <211> 24
   <212> DNA
   <213> artificial sequence
<220>
   <223> amorce
<400> 76
   tggagaaagt gattttggag aagc 24
<210> 77
   <211> 22
   <212> DNA
   <213> artificial sequence
<220>
   <223> amorce
<400> 77
   agaaccagat tgtgacaaac gc 22
<210> 78
   <211> 25
   <212> DNA
   <213> artificial sequence
<220>
   <223> amorce
<400> 78
   aatatacatt gggagcaaaa gagtc 25
<210> 79
   <211> 20
   <212> DNA
   <213> artificial sequence
<220>
   <223> amorce
<400> 79
   agagttcagc atggaaagcg 20
<210> 80
   <211> 21
   <212> DNA
   <213> artificial sequence
<220>
   <223> amorce
<400> 80
   gttgcgtatg ggaaggaatg g 21
<210> 81
   <211> 26
   <212> DNA
   <213> artificial sequence
<220>
   <223> amorce
<400> 81
   ggtagtagtg gtttacatag tcagtg 26
<210> 82
   <211> 20
   <212> DNA
   <213> artificial sequence
<220>
   <223> amorce
<400> 82
   cgttcccttc tccacaatcc 20
<210> 83
   <211> 20
   <212> DNA
   <213> artificial sequence
<220>
   <223> amorce
<400> 83
   gggttcttgg tgaatgttgc 20
<210> 84
   <211> 20
   <212> DNA
   <213> artificial sequence
<220>
   <223> amorce
<400> 84
   gttggtgctg ggtagatcac 20
<210> 85
   <211> 20
   <212> DNA
   <213> artificial sequence
<220>
   <223> amorce
<400> 85
   acggagagtg ggttgatgtg 20
<210> 86
   <211> 19
   <212> DNA
   <213> artificial sequence
<220>
   <223> amorce
<400> 86
   cgttatggcg ttggttagg 19
<210> 87
   <211> 20
   <212> DNA
   <213> artificial sequence
<220>
   <223> amorce
<400> 87
   cagaaatgga gaggacttgc 20
<210> 88
   <211> 20
   <212> DNA
   <213> artificial sequence
<220>
   <223> amorce
<400> 88
   gttcaatgct gttggtggtg 20
<210> 89
   <211> 20
   <212> DNA
   <213> artificial sequence
<220>
   <223> amorce
<400> 89
   ctgcggagaa ggatagatgg 20
<210> 90
   <211> 22
   <212> DNA
   <213> artificial sequence
<220>
   <223> amorce
<400> 90
   caacctctcg tctgtgataa tg 22
<210> 91
   <211> 19
   <212> DNA
   <213> artificial sequence
<220>
   <223> amorce
<400> 91
   gcatccttct gtaccatcc 19
<210> 92
   <211> 19
   <212> DNA
   <213> artificial sequence
<220>
   <223> amorce
<400> 92
   gctgccaagg ctgttggaa 19
<210> 93
   <211> 22
   <212> DNA
   <213> artificial sequence
<220>
   <223> amorce
<400> 93
   cagtcaggtc aacaacggaa ac 22